(19) Europäisches Patentamt
European Patent Office
Office européen des brevets

(11) Publication number: 0 081 955
B1

(12) EUROPEAN PATENT SPECIFICATION

(45) Date of publication of patent specification: 21.01.87

(21) Application number: 82306432.4

(22) Date of filing: 03.12.82

(51) Int. Cl.$^4$: C 07 D 275/06, C 07 D 209/50, C 07 D 417/12, A 61 K 31/425, A 61 K 31/40

# (54) Benzo-fused heterocyclic compounds.

(30) Priority: 14.12.81 US 330403
11.06.82 GB 8217002
30.09.82 US 431879

(43) Date of publication of application:
22.06.83 Bulletin 83/25

(45) Publication of the grant of the patent:
21.01.87 Bulletin 87/04

(84) Designated Contracting States:
AT BE CH DE FR IT LI LU NL SE

(56) References cited:
EP-A-0 003 640
EP-A-0 024 510
EP-A-0 067 436
DE-A-3 033 169
US-A-4 104 387
US-A-4 128 658
US-A-4 165 378

(73) Proprietor: AMERICAN HOME PRODUCTS CORPORATION
685, Third Avenue
New York, New York 10017 (US)

(72) Inventor: Schiehser, Guy Alan
914 Charleston Greene
Malvern, PA 19355 (US)
Inventor: Strike, Donald Peter
445 Ivan Avenue
St. Davids, PA 19087 (US)

(74) Representative: Wileman, David Francis et al
c/o John Wyeth and Brother Limited
Huntercombe Lane South
Taplow Maidenhead Berkshire SL6 OPH (GB)

EP 0 081 955 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new benzo-fused heterocyclic compounds, more particularly to benzo-fused heterocyclic compounds having a selective action on $H_2$ histamine receptors and which inhibit gastric acid secretion, to processes for their preparation and to pharmaceutical compositions containing them.

It has been postulated that the physiologically active compound histamine, which occurs naturally within the animal body, is able to combine, in the course of exerting its activity, with certain specific receptors of which there are at least two distinct and separate types. The first has been named the $H_1$ receptor (Ash and Schild, Brit. J. Pharmac., 1966, 27, 427) and the action of histamine at this receptor is blocked (antagonized) by classical "antihistamine" drugs such as mepyramine (pyrilamine). The second histamine receptor has been named the $H_2$ receptor (Black et al., *Nature,* 1972, 236, 385) and the action of histamine at this receptor is blocked by drugs such as cimetidine. It is known that one of the results of the blockage of the action of histamine at the $H_2$ receptor is the inhibition of the secretion of gastric acid and a compound which possesses this ability is therefore useful in the treatment of peptic ulcers and other conditions caused by exacerbated by gastric acidity, including stress ulcers and gastrointestinal bleeding due to trauma.

The commercialization of cimetidine and subsequent follow-up pharmacological research in patients has demonstrated that cimetidine is a drug with limitations, such as short duration of action, anti-androgenic activity, and a tendency to cause confusional states in elderly patients. Obviously, much intensive research has been carried out to find improved $H_2$ antagonists. Indeed, selective $H_2$ antagonists having greater activity than cimetidine have been discovered. Among the better known new $H_2$ antagonists are ranitidine (disclosed in U.S. Patent No. 4,128,658) having the structure:

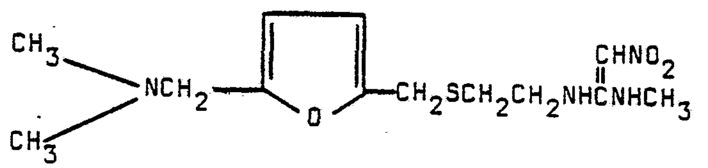

tiotidine (U.S. Patent No. 4,165,378) having the structure:

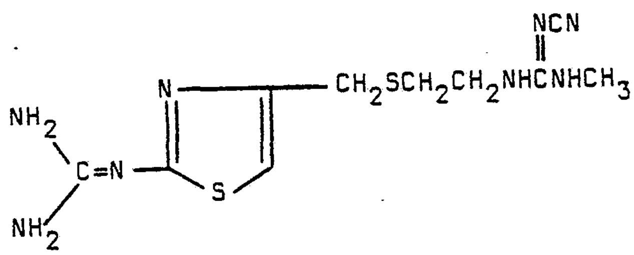

and compounds such as those disclosed in European Patent Application 24,510 having the structure:

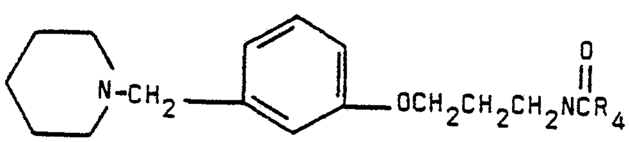

wherein $R_4$ is among others, hydrogen, methyl or methylol and those disclosed in U.K. Patent No. 2,067,987 having the structure:

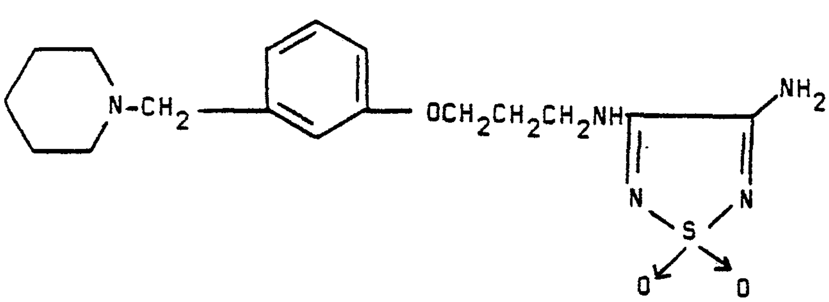

The present invention provides a novel group of compounds, with potent $H_2$ receptor antagonist activity, having the following formula:

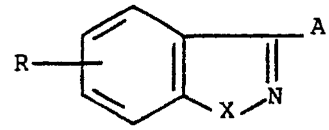

(I)

wherein R is hydrogen, mono- or di-halo, amino, nitro, cyano, hydroxy, trifluoromethyl, mercapto, lower alkyl, lower alkoxy, alkanoyl, cycloalkyl of 5 to 7 carbon atoms, carboxy, alkoxycarbonyl of 2 to 7 carbon atoms, mono- or di-lower alkyl substituted amino, alkanoylamino, lower alkyl thio, lower alkylsulfonyl, sulfamoyl, lower alkyl substituted sulfamoyl, phenyl or phenyl substituted with halo, lower alkyl, lower alkoxy, trifluoromethyl, hydroxy, amino, cyano or nitor; X is $SO_2$, SO, S or C=O and A is an amine of formula:-

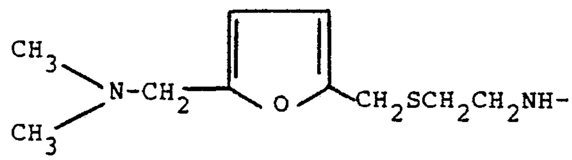

(Ia)

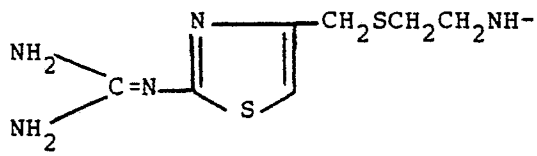

(Ib)

or

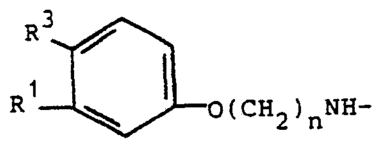

(Ic)

wherein n is 1 to 4; $R^1$ is hydrogen or $R^2CH_2$ wherein $R_2$ is mono- or diloweralkylamino, mono- or di-N-loweralkyl-amino lower alkyl, (2-furyl)methylamino, benzylamino, cycloalkylamino of 5 to 7 carbon atoms, 1-pyrrolidinyl, 1-piperidinyl, 1-hexahydroazepinyl, 1-octahydroazocinyl, 3-thiazolidinyl, 4-morpholinyl or 4-thiomorpholinyl; $R^3$ is hydrogen or (1-piperidinyl)methyl with the proviso that when $R^3$ is (1-piperidinyl)methyl, $R^1$ is hydrogen, and the pharmacologically acceptable salts thereof.

The term "halo" refers to fluoro, chloro and bromo. The terms "lower alkyl" asnd "lower alkoxy" refer to moieties having 1-6 carbon atoms in the carbon chain. The term "alkanoyl" refers to the moiety RCO- wherein R is an alkyl group having 1—4 carbon atoms.

Preferred values are:

(i) A has formula Ic,
(ii) $R^3$ is hydrogen,
(iii) n is 3,
(iv) $R^1$ is $R^2CH_2$ where $R^2$ is diloweralkylamino, 1-pyrrolidinyl, 1-piperidinyl or 1-hexa-hydroazepinyl, and/or
(v) X is $SO_2$.

In general the compounds of formula I are prepared by building up the molecule from appropriate reactive precursors. For example the compounds of the invention can be readily prepared by reacting the halide of an appropriate benzisothiazole or a derivative thereof or isoindolinone halide with the desired amine according to the following reaction sequence;

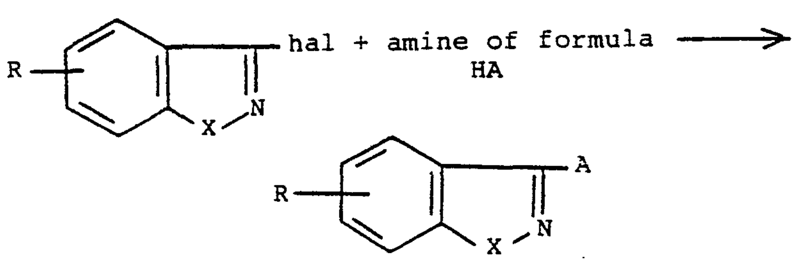

where R, X and A are as hereinbefore defined and hal represents a halogen. Alternatively "hal" may be replaced by a -S-alkyl or -S-aralkyl group. The benzisothiazole and isoindolinone starting materials are known compounds which are readily available or which can be prepared by known methods. Thus, for example, the compound

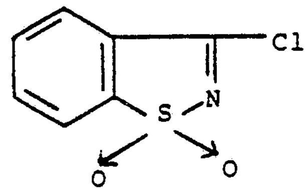

is pseudo saccharin chloride, and can be prepared according to the method of Stephen et al., *J. Chem. Soc.,* 1957, 490-92. The amines of formula HA are well known in the field of $H_2$-receptor antagonists and their preparation is reported in the following patent literature:

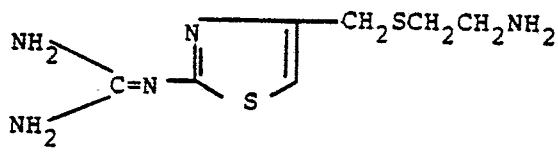

in U.S. Patent No. 4,165,378;

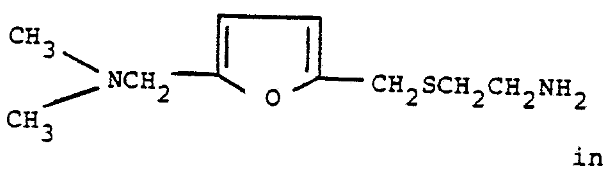

in U.S. Patent No. 4,128,658; and

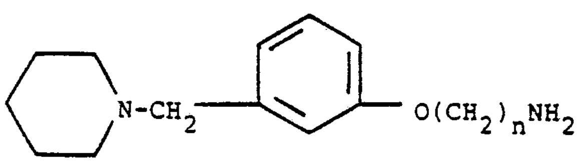

in U.K. Patent No. 2,023,133.

The amines having the formula

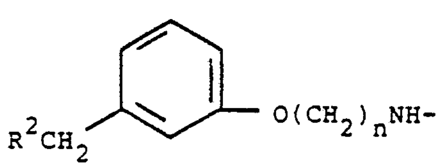

or

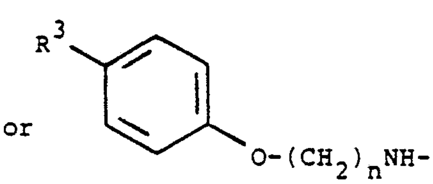

wheein $R^2$, $3^3$ and n are as defined hereinbefore, can be prepared according to the following reaction sequence (exemplified for the case where $R^3$ is hydrogen):

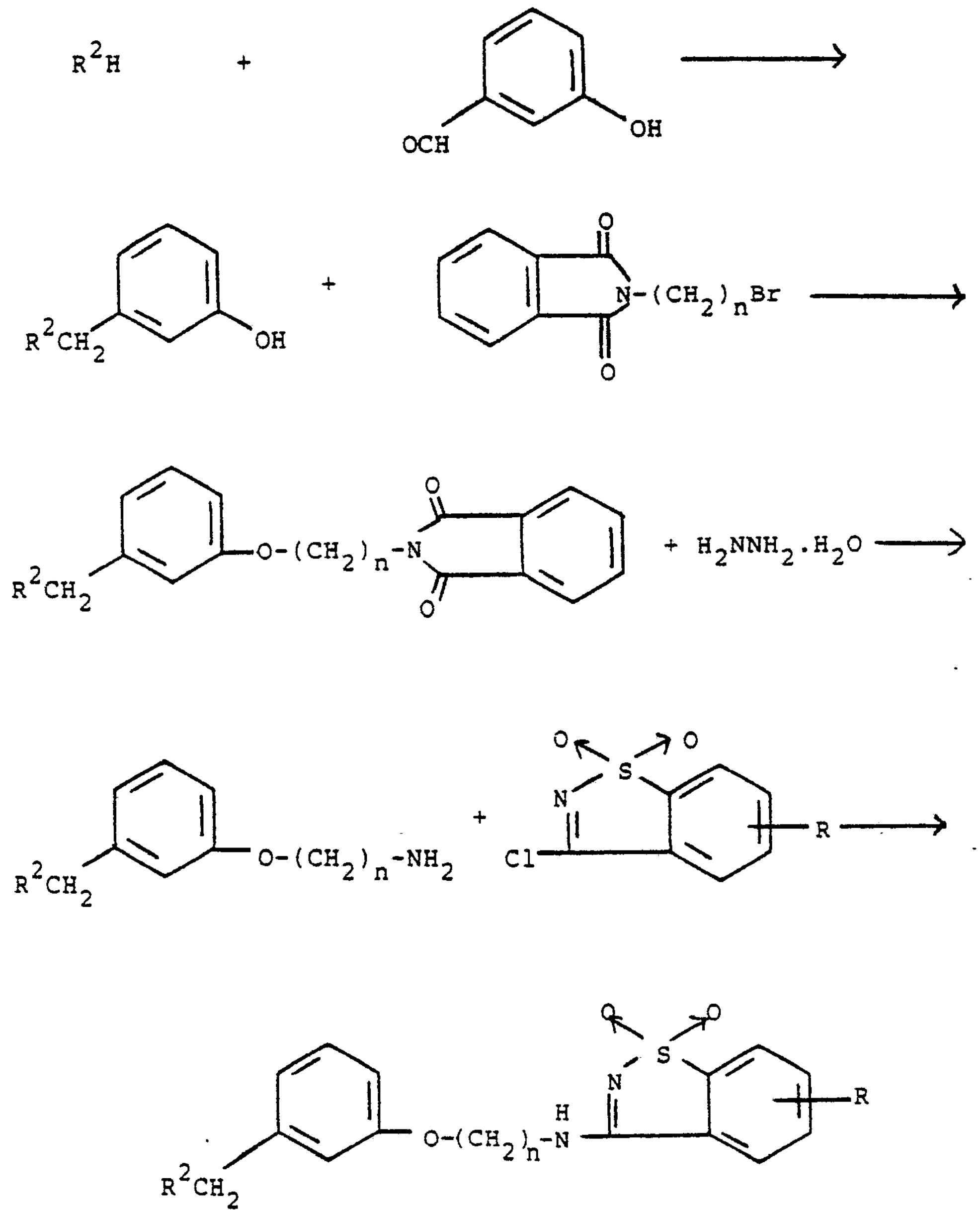

As an alternative procedure, the preparation of the isoindoledione reactant can be carried out as follows:

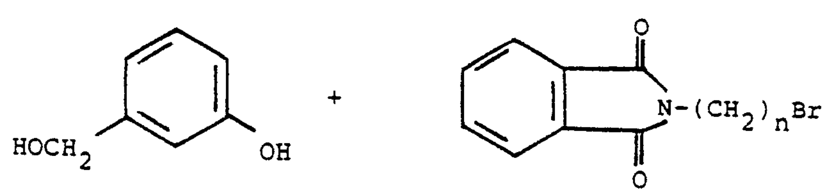

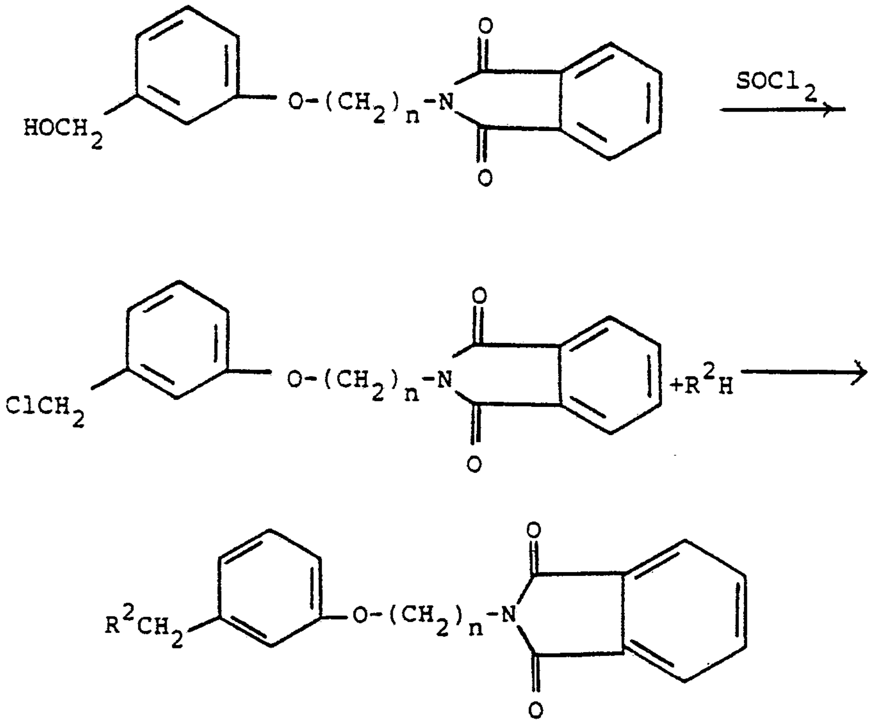


Accordingly this invention provides a process for preparing a compound of formula I as hereinbefore defined which comprises:

a) reacting a compound of formula

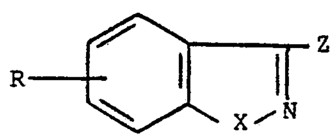

(II)

wherein X and R are as hereinbefore defined and Z is halogen or $SR^4$ where $R^4$ is alkyl or aralkyl especially alkyl of 1 to 6 carbon atoms or aralkyl of 7 to 10 carbon atoms, e.g. benzyl, with an amine of formula H—A wherein A is as hereinbefore defined to give a compound of formula I, or

b) reacting a compound of formula

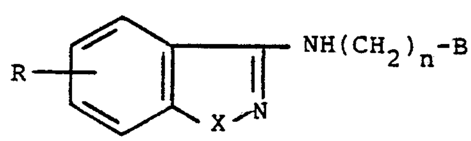

(III)

wherein X, R and n are as hereinbefore defined and B is OH or L where L represents a leaving group such as halogen or a sulphonyloxy radical e.g. alkyl or aryl sulphonyloxy, especially tosyloxy; with compound formula:

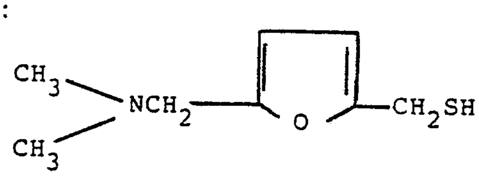

(IVa)

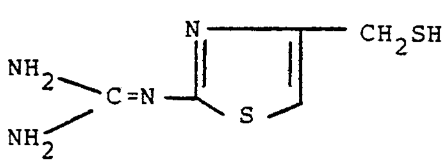

(IVb)

or

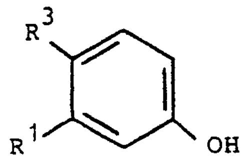

(IVc)

wherein $R^1$ and $R^2$ are as hereinbefore defined, with the proviso that (i) when a compound of formula IVa or IVb is used then n is 2 in the compound of formula III and (ii) when a compound of formula IVc is used then B is L;

c) reacting a compound of formula:

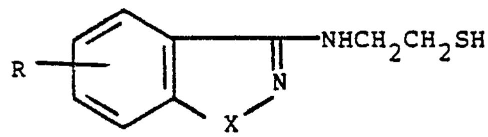

(V)

wherein R and X are as defined above, with a compound of formula:

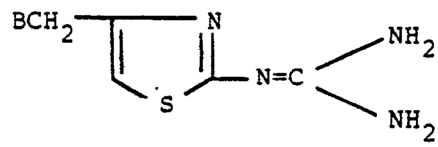

(VIa)

or

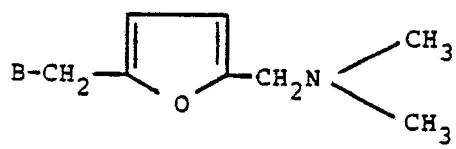

(VIb)

wherein B is OH or L as defined above to give a compound of formula I wherein A has the formula Ia or Ib; or

d) reacting an aldehyde of formula

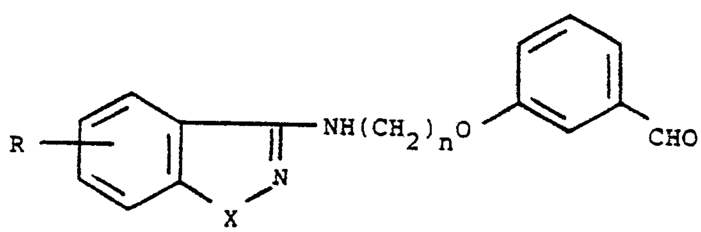

(VIIa)

with an appropriate amine of formula

$HR^2$ (VIIb)

in which formulae n, R, X and $R^2$ are as defined above, under reductive amination conditions to give a corresponding compound of formula I wherein $R^1$ is $R^2CH_2$- and $R^3$ is hydrogen; or

e) reacting an aldehyde of formula

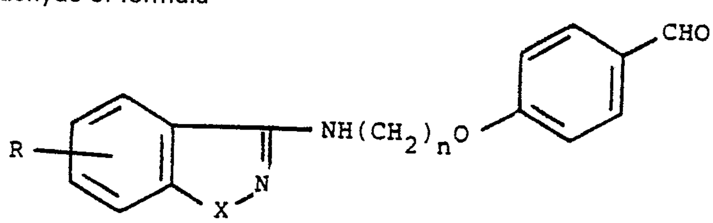

(VIIc)

wherein n, R anx X are as hereinbefore defined with piperidine under reductive amination conditions to give a compound of formula I wherein $R^3$ is (1-piperidinyl)-methyl; or

f) reacting an aldehyde of formula

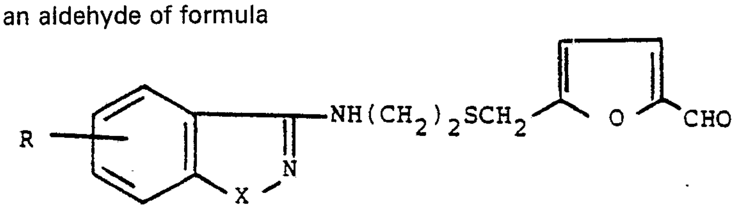

(VIId)

wherein X, R and X are as hereinbefore defined with dimethylamine under reductive amination conditions to give a compound of formula I wherein A has formula Ia; or

g) reacting a compound of formula

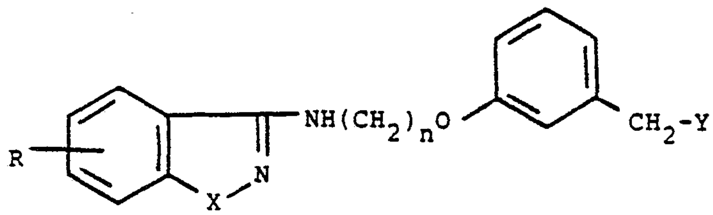

(VIII)

wherein n, R and X are as hereinbefore defined and Y is OH or L wherein L is a leaving group such as hereinbefore described, with an amine of formula $HR^2$ as defined above, the reaction being carried out in the presence of a nickel catalyst when Y is OH, to give a compound of formula I wherein A has the formula Ic and $R^1$ is $R^2CH_2$-; or

h) reacting a compound of formula

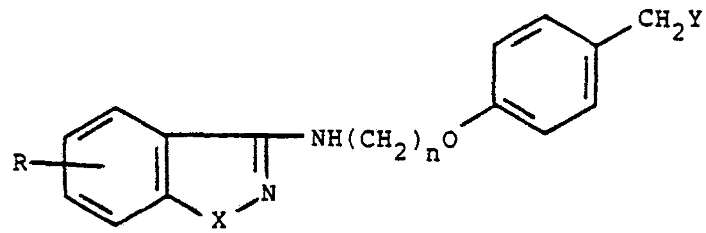

(VIIIa)

wherein Y, n, X and R are as hereinbefore defined, with piperidine, the reaction being carried out in the presence of a nickel catalyst when Y is OH, to give a compound of formula I wherein A has the formula Ic and $R^3$ is (1-piperidinyl)-methyl; or

i) reacting a compound of formula

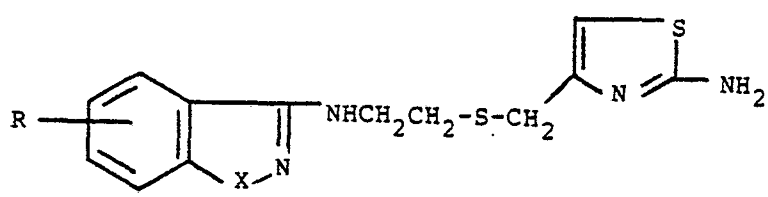

(IX)

wherein X and R are as hereinbefore defined a compound capable of converting $-NH_2$ to

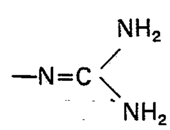

to give a compound of formula I wherein A has formula Ic,

j) reacting of compound of formula

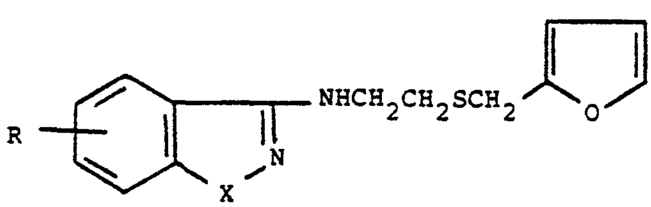

(X)

wherein X and R are hereinbefore defined with dimethylamine in the presence of formaldehyde or paraformaldehyde, to give a compound of formula I wherein A has formula Ia,

k) reacting a compound of formula

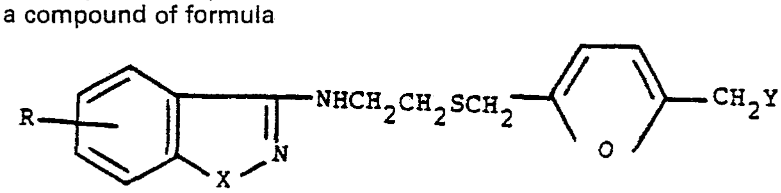

(XI)

wherein Y is OH or a leaving group L as hereinbefore described, with dimethylamine; the reaction being carried out in the presence of a nickel ctalyst when Y is OH; or

1) a compound of formula I having a reactive substituent group is converted to a compound of formula I having a different substituent gorup; or

m) a basic compound of formula I is converted to a pharmacologically acceptable salt or a pharmacologically acceptable salt is converted to the free base form.

Processes b) and c) may be carried out in an inert solvent such as an alkanol, acetonitrile, dimethylformamide, dimethylsulfoxide, acetone and the like; if desired with heating. Preferably the reaction is conducted in the presence of a base which acts as acid acceptor. Suitable bases include for example NaOH, KOH, LiOH, triethylamine, dimethylaniline, sodium ethoxide and the like. When B is OH in the compounds of formulae III or VI or VIb then the reaction is carried out in the presence of a mineral acid e.g. concentrated HC1.

Processes d), e) and f) may be carried out using a hydride transfer agent to effect reductive amination, e.g. an alkali metal borohydride such as sodium borohydride, and in an inert solvent, e.g. an alkanol such as ethanol.

When Y is a leaving group in the compound of formula VIII, VIIIa or XI then processes g), h) and k) may be carried out in inert solvent, e.g. acetonitrile, dimethylformamide preferably in the presence of an acid acceptor, e.g. triethylamine or $K_2CO_3$.

When Y is OH in the compounds of formulae VIII, VIIIa or XI then processes g), k) and h) may be carried out in the presence of a nickel catalyst such as Raney nickel e.g. Raney nickel W2. An organic solvent which is inert under the reaction conditions, is usually used, for example xylene, toluene or benzene. Preferably the reaction is carried out by heating the reactants under reflux in a water immiscible organic solvent, e.g. xylene, and removing the water formed during reaction by azeotropic distillation. If necessary reactive substituent groups can be blocked during reaction and released later.

Process i) is carried out by standard procedures known in the art for converting an amine function to a guanidine function, e.g. using urea, thiourea, cyanamide or a regent of formula
18.11

$$\underset{\displaystyle NH}{\overset{}{NH_2\underset{\|}{C}\text{-}L^1}}$$

where $L^1$ is a halogen, S-alkyl or alkoxy.

For example the reaction with an S-alkyl-isothiourea may be affected by heating the reactants in a suitable solvent, e.g. acetonitrile or an alkanol.

Process j) may be carried out using standard procedures well known in the art for effecting the Mannich reaction.

Many of the intermediates used in the reactions above are known compounds or are prepared by analogous methods for known compounds. Compounds of formula III can be prepared by reacting a compound of formula II with a corresponding amine of formula

$$NH_2(CH_2)_n\text{-}B \qquad (XII)$$

Compounds of formula IVa and IVb may be prepared by treating the corresponding chloromethyl analogues with NaSH. Similarly compounds of formula V may be prepared by reacting corresponding compounds of formula III where B is halogen, e.g. chlorine, with NaSH.

Compounds of formula VIa and VIb wherein B is a leaving group may be prepared for example from the corresponding alcohols by conventional means.

Compounds of formula VIIa, VIIc and VIId may be prepared by reacting the appropriate compound of formula III wherein B is chloro with 3- or 4-hydroxybenzaldehyde or 5-mercapto methylfuran-2-carboxaldehyde in the manner of process b).

Compounds of formula VIII, VIIIa and XI may be prepared by reacting a compound of formula II with the appropriate amine starting materials wherein Y is OH in the manner of process a) and if desired converting the hydroxy Y group to a leaving group by conventional means.

Similarly compounds of formulae IX and X may be prepared by reacting a compound of formula II with the appropriate compound of formula

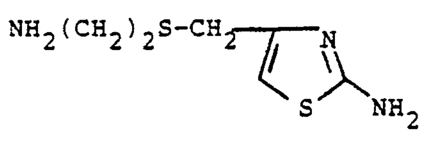

or

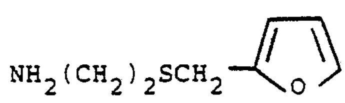

in the manner of process a).

Once a compound of formula I having a reactive substituent group has been prepared then that compound may be converted in known manner to other compounds of formula I. For example when R is a lower alkocxy substituent dealkylation produces a corresponding compound of formula I wherein R is a

hydroxy substituent. When R is nitro then reduction, e.g. using ammonium sulphide can convert the nitro group to an amino group. Such amino groups may be acylated.

In any of the aforementioned processes reactive substituent groups may be blocked and released at a later stage.

The compounds of the invention readily form pharmacologically acceptable salts with both inorganic and organic acids, such as hydrochloric, hydrobromic, sulfuric, nitric, phosphoric, acetic, maleic, fumaric, citric and the like.

The compounds of the invention have histamine $H_2$-blocking activity and can be used in the treatment of conditions where there is hypersecretion of gastric acid, such as in gastric and peptic ulceration, and other conditions caused or exacerbated by gastric acidity such as stress ulceration or gastric intestinal bleeding due to trauma.

The compounds of the invention can be administered orally or parenterally or by suppository, of which the preferred route is the oral route. They may be used in the form of the base or as a pharmacologically acceptable salt. They will in general be associated with a pharmaceutically acceptable carrier or diluent, to provide a pharmaceutical composition.

Accordingly this invention also provides a pharmaceutical composition comprising a compound of formula I or a pharmaceutically acceptable salt thereof and a pharmaceutically acceptable carrier.

The compounds of the invention can be administered in combination with other active ingredients, e.g. conventional antihistamines if required. For oral administration the pharmaceutical composition can be most conveniently be in the form of capsules or tablets, which may be slow release tablets. The composition may also take the form of a dragee or may be in syrup form.

A convenient daily dose by the oral route would be the order of 100 mg to 1.2 g per day, in the form or dosage units containing from 20 to 200 mg per dosage unit. A convenient regimen in the case of a slow release tablet would be twice or three times a day.

Parenteral administration may be by injections at intervals or as a continuous infusion. Injection solutions may contain from 10 to 100 mg/ml of active ingredient.

The histamine $H_2$-antagonist activity of the compounds of the invention may be demonstrated by the ability of the compounds to inhibit the histamine-induced positive chronotropic response in the spontaneously beating right atrium of the guinea pig heart, as well as by activity in other more generalised procedures, such as the modified Shay procedure of pylorus ligation for the study of rat gastric secretion and by the study of gastric secretion in the unanaethetised dog. The procedures for these tests and the results for some of the compounds of the invention are presented in the Examples (vide infra). The following illustrate the preparation of starting materials.

### Preparation A

### Preparation of 3-chlorobenzisothiazole-1,1-dioxide (ψ-saccharin chloride).

Following the procedure of Stephen et al., *J. Chem. Soc.* 1957, 490-92, 1 mol of saccharin (1,2-benzisothiazol-3(2*H*-one 1,1-dioxide) is heated with 1.1 mol phosphorus pentachloride at 170°C for 1.5 hours. Phosphorus oxychloride is removed at 60°/30 mm and the yellow crystalline residue of ψ-saccharin chloride and *o*-cyanobenzene sulfonyl chloride is treated with ether in which the latter is soluble. The sparingly soluble ψ-saccharin chloride in a yield of 28% is collected and crystallised from ether as white needles, m.p. 132-137°C.

### Preparation B

### Preparation of 3-Chloro-1H-isoindol-1-one

Following the procedure of U.K. Patent No. 704,595, 147 parts of phthalimide are refluxed while stirring with 208 parts of phosphorus pentachloride and 150 parts of *o*-dichlorobenzene. A light orange-yellow solution is formed at 130°C and the solution is stirred at 150°C for a 4 hour period. The phosphorus oxychloride produced is distilled off under reflux up to an internal temperature of 205°C and the *o*-dichlorobenzene is partly distilled off. The resulting solution is filtered and distilled in vacuo. After removing the *o*-dichlorobenzene and the first runnings from 105°C to about 143°C (under 15 mm pressure about 50 parts of an almost colourless distillate are obtained. Redistilling the solution produces a colourless distillate (b.p. 159-160°C/15 mm) which solidifies at 70-73°C as fine crystals. The product is recrystallised from dry cyclohexane to yield the title compound in long bright needles having a melting point of 77-78°C.

The following Examples illustrate the preparation of compounds of the invention; Examples 39 to 41 give details of pharmacological test procedures and results therein:'-

### Example 1

### N-[2-[[[5-[(dimethylamino)methyl]-2-furanyl]methyl]thiol]-ethyl]-1,2-benzisothiazol-3-amine 1,1-dioxide

To a refluxing solution of 2.14 g (10 mmol) of 2-[[[5-[(dimethylamino)methyl]-2-furanyl]methyl]-thio]ethylamine in 150 ml of chloroform is added incrementally 2.02 g (10 mmol) of 3-chlorobenzoisothiazole 1,1-dioxide. The reaction mixture is refluxed for an additional 30 minutes, is cooled at room temperature and then solvent is removed on a rotoevaporator. The resulting oil is subjected to

preparative column chromatography on silica gel. Elution with methylene chloride followed by methylene chloride: methanol (95:5 v/v) yields 420 mg (12% w/w) of a white solid. The solid is suspended in ethyl ether, separated by suction filtration and dried in vacuo to give 270 mg (7% w/w) of the title compound: m.p. 91-94°C.

IR $cm^{-1}$ 3300, 1612, 1585, 1275, 1150, 1119, 775, 748, and 707 $cm^{-1}$

NMR ($CDC1_3$) δ 2.19 (6H, s) 2.81 (2H, t, J=6Hz), 3.38 (2H, s), 3.54 (4H, m) 3.70 (2H, s) 6.08 (1H, d, J=4Hz), 6.15 (1H, d, J=4Hz), 7.70 (5H, m) $^{13}C$ :$CDC1_3$) δ 28.50, 30.59, 42.05, 45.02, 55.98, 109, 49, 109,92, 121.56, 121.70, 127.89, 132.62, 133.12, 142.62, 150.96, 151.84 and 159.85; MS m/e 380 ($MH^+$).

Example 2

N[3-[3-[(1-piperidinyl)methyl]phenoxy]propyl]-1,2-benzisothiazol-3-amine 1, 1-dioxide

To a gently refluxing solution of 3-[3-[(1-piperidinyl)methyl]phenoxy]propylamine (992 mg. 4 mmol) in 90 ml of chloroform is added dropwise a solution of 880 mg (4.4 mmol) of 3-chlorobenzoisothiazol 1,1-dioxide in 30 ml of chloroform. Following the addition, the reaction mixture is refluxed for an additional 15 min., cooled to room temperature and solvent removed in vacuo. Column chromatography of the resultant residue (foam) on silica gel utilizing methylene chloride:methanol: ammonium hydroxide (90:10:1 by volume) as eluting solvent affords after evaporation a white solid. The crude product is suspended in ethyl acetate, filtered and dried to give 620 mg (37.5% w/w) of the title compound, m.p., 138-140°C.

TLC [silica/methanol: ammonium hydroxide (99:1 by volume] Rf 0.33

IR(KBr) 3300, 1612, 1271, 1150, 1118, 770, 746 $cm^{-1}$;

NMR ($d_6$−DMSO) δ 9.44 [1H (removed by $D_3O$ exchange)], 8.13 [1H, m] 7.88 [3H, m] 7.15 [1H, m] 6.79 [3H, broad s), 4.18 (2H, t), 3.65 (2H, t), 3.26 (2H, s), 2.10 (6H, m), and 1.30 (6H, broad s). $^{13}$CNMR ($D_6$-DMSO) δ 160.06, 159.15, 142.98, 141.12, 134.03, 133.60, 129.71, 128.42, 123.56, 121.71, 115.52, 113.47, 65.72, 63.45, 54.57, 40.53, 28.63, 26.26 and 24.69.

Example 3

[4-[[[2-[(1,2-benzisothiazole-3yl]amino]ethyl]thio]-methyl]-2-thiazolyl]guanidine S′,S′-dioxide

To a refluxing solution of 1.3 g (6.6 mmol) of [4-[[[2-[amino]ethyl]thio]-methyl]-2-thiazol]guanidine in 100 ml of chloroform is added a solution of 1.33 g (6.6 mmol) of 3-chlorobenzisothiazol- 1,1-dioxide in 50 ml of chloroform over 20 min. The mixture is maintained at reflux for an additional 30 min. and is then allowed to come to room temperature. Decantation of the chloroform leaves a solid residue which is triturated with methanol and potassium carbonate. The methanol is removed in vacuo and the remaining material is subjected to column chromatography on silica gel. Elution with methylene chloride:methanol:ammonium hydroxide (90:10:1 by volume) followed by methylene chloride: methanol: ammonium hydroxide (65:35:1 by volume) gives 545 mg 20.8% w/w) of crude product. Recrystallisation from ethanol affords the title compound, m.p. 225-231°C (dec.)

IR (KBR) 3440, 3380, 3270, 1615, 1580, 1535, 1450, 1275, 1260, 1145, 1112, 775, 748 and 655 $cm^{-1}$;

NMR ($d^6$−DMSO) δ [1H, broad s (removed by $D_2O$ exchange]), 8.15 (1H, m) 7.85 (3H, m), 6.79 (4H, broad s (removed by $D_2O$ exchange)), 6.04 (1H, s) 3.67 (4H, m), 2.76 (2H, t J=6Hz).

| | | | |
|---|---|---|---|
| *Analysis for:* | $C_{14}H_{16}N_6O_2S_3$ | | |
| *Calculated:* | C, 42.40; | H, 4.07; | N, 21.20 |
| *Found:* | C, 42.25; | H, 4.23; | N, 20.87 |

Example 4

3-[3-[3-[(1-piperidinyl)methyl]phenoxy]-propyl]amino]-1*H*-isoindol-1-one.

A solution of 248 mg (1 mmol) of 3-[3-[(1-piperidinyl)methyl]phenoxy]propylamine in 20 ml alcohol-free chloroform is heated to reflux and a solution of 166 mg (1 mmol) of 3-chloro-1*H*-isoindol-1-one in 20 ml of chloroform is added dropwise over 15 min. The reaction mixture is refluxed for an additional 15 min. cooled to room temperature and solvent removed on a rotoevaporator. The residue is chromatographed on silica gel using methylene chloride:methanol (95:5 by volume) followed by methylene chloride: methanol:ammonium hydroxide (95:5:0.5 by volume] to yield an oil. Treatment with isopropanolic hydrochloric acid and subsequent crystallisation from acetonitrile/ethyl ether and drying in vacuo gives 67 mg (14.7% w/w) of the title compound as the dihydrochloride quarterhydrate salt. m.p. 211-216°C.

IR (KBr) 3410 (broad), 23745, 2495, 1770, 1643, 1270, 1280, 1040, 719 $cm^{-1}$;

NMR ($d^6$−DMSO) δ 8.57 (1H, m), 7.87 (3H, m) 7.22 (3H, m) 6.84 (1H, m) 4.12 (6H, m) 3.50 (2H, broad s (removed by $D_3$ exchange)), 3.25 (2H, m), 2.83 (2H, m), 2.09 (2H, m), 1.70 (6H, m).

| | | | |
|---|---|---|---|
| *Analysis for:* | $C_{23}H_{29}N_3O_2 \cdot \frac{1}{4}\ H_2O$ | | |
| *Calculated:* | C, 60.72; | H, 6.54; | N, 9.24 |
| *Found:* | C, 60.77; | H, 6.49; | N, 9.22 |

Example 5

N[3-[3-[(1-piperidinyl)methyl]phenoxyl]propyl]-6-nitro-1,2-benzisothiazol-3-amine 1,1-dioxide, hydrochloride

To a gently reluxing solution of 496 mg (2 mmol) of 3-[3-[(1-piperidinyl)methyl]phenoxy]propylamine in 25 ml of alumina-treated chloroform is added dropwise over 20 min. 492 mg (2 mmol) of 6-nitropseudosaccharyl chloride (prepared according to the method described in Japanese patent publication 7014302) in 25 ml of chloroform. The mixture is heated for 10 min. following the addition and then is rotoevaporated to an oily foam. Crystallization from absolute ethanol gives crude product which yields 272 mg (27.59) of the title compound upon recrystallization from 95% ethanol: m.p. 223-227°C, IR (KBr) 1603, 1550, 1318, 1175 $cm^{-1}$;

| | | | |
|---|---|---|---|
| *Analysis for:* | $C_{22}H_{26}N_4O_5S{\cdot}HC_1$ | | |
| *Calculated:* | C, 53.37; | H, 5.94; | N, 11.32 |
| *Found:* | C, 52.93; | H, 5.35; | N, 11.24 |

Example 6

N[3-[3-[(1-piperidinyl)methyl]phenoxy]propyl]-6-amino 1,2-benzisothiazol-3-amine 1,1 di-dioxide

To a solution of 1.98 g (4 mmol) of the compound of Example 5 in 30 ml of ethanol:ammonium hydroxide (5:1) is added 10 ml of an ammonium sulfide solution prepared by dissolving 540 mg (10 mmol) of ammonium chloride and 2.4 g (10 mmol of sodium sulfide monohydrate in 10 ml of water. The resulting solution is heated to reflux and maintained under a nitrogen atmosphere for 20 min. Removal of the solvent *in vacuo* is followed by an aqueous sodium bicarbonate:methylene chloride partition. The aqueous phase is subseuqently extracted with ethyl acetate and the organic phase dried over magnesium sulfate and evaporated to give 905 mg (52.8%) of the title compound: m.p. 191-193°C, IR (KBr) 3370, 1612, 1275, 1139 $cm^{-1}$.

| | | | |
|---|---|---|---|
| *Analysis for:* | $C_{22}H_{28}N_4O_3S{\cdot}HCl$ | | |
| *Calculated:* | C, 61.66; | H, 6.59; | N, 13.07 |
| *Found:* | C, 61.11; | H, 6.60; | N, 12.81 |

Example 7

N[3-[3-[(1-piperidinyl)methyl]phenoxy]propyl]-6-chloro-1,2-benzisothiazol-3-amine 1,1-dioxide, hydrochloride salt hemihydrate

To a solution of 248 mg (1 mmol) of 3-[3-[(1-piperidinyl)methyl]phenoxy]propylamine in 10 ml of alcohol-free chloroform is added dropwise a solution of 236 mg (1 mmol) of 6-chloropseudosaccharyl chloride (prepared according to the method described in Japanese Patent Publication 7014302) in 20 ml of chloroform. The reaction mixture is maintain at reflux for 1 hr, cooled to room temperature, and solvent removed in vacuo. The residue is dissolved in ethanol and treated with ethereal hydrochloric acid. Crystallization of the crude hydrochloride from ethanol gave 128 mg (25.9%) of the title compound: m.p. 210-215°C, IR (KBr) 2500, 1642, 1290, 1164 and 1145 $cm^{-1}$.

| | | | |
|---|---|---|---|
| *Analysis for:* | $C_{22}H_{26}ClN_3O_3S{\cdot}HCl\ \frac{1}{2}\ H_2O$ | | |
| *Calculated:* | C, 53.54; | H, 5.72; | N, 8.51 |
| *Found:* | C, 53.79; | H, 5.62; | N, 8.33 |

Example 8

N[3-[3-[(1-piperidinyl)methyl]phenoxy]propyl]-6-fluoro-1,2-benzisothiazol-3-amine 1,1-dioxide

A. 3-chloro-6-fluoro-1,2-benzisothiazole 1,1-dioxide

A mixture of 2.0 g (10 mmol) of 6-fluoro-saccharin and 2.1 g (10 mmol) of phosphorus pentachloride in 10 ml of *o*-dichlorobenzene is heated to reflux and maintained for 1 hr. The reaction mixture is cooled and evaporated to give crued 6-fluoropseudosaccharyl chloride which is used without characterization.

B. N[3-[3-[(1-piperidinyl)methyl]phenoxy]propyl]-6-fluoro- 1,2-benzisothiazol-3-amine 1,1-dioxide

A refluxing solution of 2.0 g (8 mmol) of 3-[3-[(1-piperidinyl)methyl]phenoxy]propylamine is treated dropwise with 2.19 g (10 mmol) of crude 6-fluoropseudosaccharyl chloride. The reaction mixture is maintained at reflux for 10 min. cooled and solvent removed *in vacuo.* The residual oil is subjected to

column chromatography to give after trituration with ethyl ether, and drying the title compound: m.p. 139-163°C, IR (KBr) 3300, 1642, 1479, 1273, 1145 and 1122 $cm^{-1}$.

| | | | |
|---|---|---|---|
| *Analysis for:* | $C_{22}H_{26}FN_3O_3S$ | | |
| *Calculated:* | C, 61.23; | H, 6.07; | N, 9.73 |
| *Found:* | C, 60.99; | H, 6.00; | N, 9.34 |

Example 9

N[3-[3-[(1-piperidinyl)methyl]phenoxy]propyl]-5,6-dichloro-1,2-benzisothiazol-3-amine 1,1-dioxide

To a refluxing solution of 1.24 g (5 mmol) of 3-[3-[(1-piperidinyl)methyl]phenoxy]propylamine in 30 ml of alumina-treated chloroform is added dropwise 1.35 g (5 mmol) of 5,6-dichloropseudosaccharyl chloride(prepared according to the method described in Japanese Patent Publication 7014302) in 30 ml of chloroform. The mixture is refluxed for an additional 10 min., cooled to room temperature and solvent removed by rotoevaporation. The resulting foam is crystallized for absolute ethanol and yields upon recrystallization from ethanol: water:tetrahydrofuran, the desired title compound as the monohydrochloride sale: m.p. 281-284°C, IR (KBr) 2942, 1628, 1298, 1171 $cm^{-1}$.

| | | | |
|---|---|---|---|
| *Analysis for:* | $C_{22}H_{25}FN_3O_3S{\cdot}Cl$ | | |
| *Calculated:* | C, 50.92; | H, 5.05; | N, 8.10 |
| *Found:* | C, 50.80; | H, 5.17; | N, 8.15 |

Example 10

N[3-[3-[(1-piperidinyl)methyl]phenoxy]propyl]-5-methyl-1,2-benzisothiazol-3-amine 1,1-dioxide
A. 3-chloro-5-methyl-1,2-benzisothiazole 1,1-dioxide

A slurry of 5-methyl-1,2-benzisothiazolin-3-one, 1,1-dioxide (1.80 g, 9.13 mmoles) and phosphorus pentachloride (2.00 g, 9.59 mmoles) in *o*-dichlorobenzene (9.0 ml) is heated under dry nitrogen. Complete solution occurs at ca. 85°C. The temperature is raised to 180°C and maintained at 180°C for 2 hours. After cooling to room temperature the brown solution is partially evaporated *in vacuo* at 70°C to remove the phosphorus oxychloride formed. Addition of pentane and standing overnight at −5°C gives a gum. Decantation and trituration with three 50 ml portions of hexane gives a gum which cannot be solidified and is used directly in the next step.

B. N[3-[3-[(1-piperidinyl)methyl]phenoxy]propyl]-5-methyl- 1,2-benzisothiazol-3-amine 1,1-dioxide

To a refluxing solution of 3-[3-(1-piperidinylmethyl)-phenoxy]propylamine (1.33 g, 5.36 mmoles) in dried chloroform (50 ml) under dry nitrogen is added over 45 minutes a solution of 3-chloro-5-methyl-1,2 benzisothiazole 1,1-dioxide (1.21 g 5.64 mmoles) in dried chloroform (25 ml) while maintaining reflux. After the addition is complete refluxing is continued for 15 minutes and the clear brown solution is allowed to come to room temperature Evaporation *in vacuo* gives a tan solid which cannot be crystallized. Column chromatography on silica gel using methylene chloride/methanol/ammonium hydroxide 95/5/0.5 as eluent gives the free methanol base a brown foam, 1.22 g. Trituration with ether gives an off-white coloured solid, 0.73 g, m.p. 120-124°C. Recrystallization from ethyl acetate/pentane and finally ethyl acetate gives the pure product, 0.169 tan solid m.p. 120-4°C.

IR (KBr) 3300, 2938, 1622, 1530 $cm^{-1}$;

NMR (in DMSO) 6.8-8.0 (7H, m), 4.05 (2H, t), 3.76 (2H, t), 1.37 (6H, s), 1.4 (6H, s), 2.0-2.4 (6H, m), 2.5 (3H, s), 3.4 (2H, s), 3.6 (2H, t), 4.1 (2H, t), 6.7-8.0 (7H, m), 9.3 (1H, m).

| | | | |
|---|---|---|---|
| *Analysis for:* | $C_{23}H_{29}N_3O_3S$ | | |
| *Calculated:* | C, 64.61; | H, 6.84; | N, 9.87 |
| *Found:* | C, 64.25; | H, 6.84; | N, 9.56 |

Example 11

N[3-[3-[(1-piperidinyl)methyl]phenoxy]propyl]-5-chloro-1,2-benzisothiazol-3-amine 1,1-dioxide
A. 3.5-dichloro-1,2-benzisothiazole 1,1-dioxide

A slurry of 5-methyl-1,2-benzisothiazoline-3-one, 1,1-dioxide (1.50 mg, 6.89 mmoles), and phosphorus pentachloride (1.51 g, 7.24 mmoles) in in *o*-dichlorobenzene (25 ml) is heated under dry nitrogen. At 80°C hydrogen chloride evolution appears to start. At ca. 90°C complete solution occurs. The temperature is raised and maintained at 180-185°C for 2 hours. After cooling to room temperature the tan solution is partially stripped *in vacuo* at 70°C to remove the phosphorus oxychloride formed. Addition of pentane

(100 ml) to the cooled residue gives a tan solid 0.767 g, m.p. 130-136°C, IR (KBr) 1528, 1344, 1173, 1530 $cm^{-1}$; NMR (in DMSO) 7.47-8.28 (m).

B. N[3-[3-[(1-piperidinyl)methyl]phenoxy]propyl]-5-chloro-1,2-benzisothiazol-3-amine 1,1-dioxide,

To a refluxing solution of 3-[3-(1-piperidinylmethyl)-phenoxy]propylamine (0.700 g, 2.82 mmoles) in dried chloroform (30 ml) under dry nitrogen is added over 45 minutes a solution of 3,5-dichloro-1,2-benzisothiazole 1,1-dioxide (0.699 g, 2.96 mmoles) in dried chloroform (15 ml) while maintaining reflux. After the addition is complete, refluxing is continued for 15 minutes and the clear tan solution allowed to come to room temperature. Evaporation *in vacuo* gives a yellow-orange foam. Crystallization from methylene chrloride/acetonitrile gives a yellow solid, 1.034 g, m.p. 242-245°C. Recrystallization from ethanol gives the hydrochloride salt of the title compound as a yellow solid 0.275 g, m.p. 242-244°C. IR (KBr) 2950, 2540, 1620; NMR (in DMSO) 1.8 (6H, s), 2.2 (2H, t), 2.9 (2H, m), 3.3 (2H, m), 3.7 (2H, m), 4.2 (4H, m); 6.9-8.5 (7H, m), 9.9 (1H, m); 10.1 (1H, m).

| | | | | |
|---|---|---|---|---|
| *Analysis for:* | $C_{22}H_{26}ClN_3O_3S{\cdot}HCl$ | | | |
| *Calculated:* | C, 54.55; | H, 5.62; | N, 8.67; | C1, 14.64 |
| *Found:* | C, 54.54; | H, 5.61; | N, 8.82; | C1, 14.92 |

Example 12

N[3-[3-[(1-piperidinyl)methyl]phenoxy]propyl]-5-Methoxy-1,2-benzisothiazol 1,1-dioxide

A. 3,-dichloro-5-methoxy-1,2-benzisothiazole 1,1-dioxide

A slurry of 5-methyl-1,2-benzisothiazoline-3-one, 1,1-dioxide (1.60 g, 7.504 mmoles) and phosphorus pentachloride (1.64 g, 7.879 mmoles) in *o*-dichlorobenzene (9.0 ml) is heated under dry nitrogen. At 100°C hydrogen chloride evolution appears to start and complete solution also occur. The temperature is raised to 180°C for 2 hours. After cooling to room temperature the solution is partially evaporated *in vacuo* at 70°C to remove the phosphorus oxychloride formed. Addition of pentane (50 ml), and stirring overnight at room temperature gives a tan gum. Decantation and trituration with three 50 ml portions of pentane gives on filtering a light tan solid, m.p. 50-85°C. IR (KBr) 1725 (shoulder) 1297, 1304, 1175. NMR (in DMSO) 3.9 (s, starting material), 4.0 (s), 7.2-8.2 (aromatic), 12.7 (s, starting material)

B. N-[3-[3-[(1-piperidinyl)methyl]phenoxy]propyl]-5-methoxy-1,2-benzisothiazol-3-amine 1,1-dioxide.

To a refluxing solution of 3-[3-(1-piperidinylmethyl]phenoxy]propylamine (1.33 g, 5.355 mmoles) in dried chloroform (50 ml) under dry nitrogen is added over 45 minutes a solution of 3-chloro-5-methoxy-1,2-benzisothiazole 1,1-dioxide (1.30 g), 5.632 mmoles) in dried chloroform (25 ml) while maintaining reflux. After the addition is complete, refluxing is continued for 45 minutes and the solution allowed to come to room temperature. Evaporation *in vacuo* gives sticky residue which cannot be crystallized. Column chromatography on silica gel using 95/5/0.5 methylene chloride/methanol/ammonium hydroxide as eluent gives the free base as a tan foam 1.703 g which cannot be crystallized. Treatment with anhydrous oxalic acid in ethanol gives the title compound as the 2/3 oxalate, ethanolate salt 0.302 g, tan solid, m.p. 114-120°C (from 0) and clear by 240°C.

IR (KBr) 2960, 1620, 1338, 1170 $cm^{-1}$ NMR (in DMSO) 1.5 (6H, s), 2.2 (2H, t) 2.7 (4H, s). 3.6 (2H, t), 3.9 (3H, s and 2H, s), 4.1 (2H, t) 6.9-7.9 (7H, m), 9.5 (1H, s). Also one mole EtOH.

*Analysis for:* $C_{23}H_{29}N_3O_3S.2/3CO_2H.C_2H_5OH$ (with $CO_2H$ printed below the first $CO_2H$, joined by a vertical bond)

| | | | |
|---|---|---|---|
| *Calculated:* | C, 57.54: | H, 6.66; | N, 7.64 |
| *Found:* | C, 57.30; | H, 6.73; | N. 7.71 |

Example 13

N-[3-[3-[(1-piperidinyl)methyl]phenoxy]propyl]-6-sulfamoyl-benzisothiazol-3-amine 1,1-dioxide

A. 6-sulfamoyl-benzisothiazoline-3-thione 1,1-dioxide.

To a mixture of 5.25 g (20 mmol) of 6-sulfamoyl-saccharin in 50 ml of diglyme is added 13,4 g (30 mmol) of phosphorus pentasulfide and 6.75 g (80 mmol) of sodium bicarbonate. The mixture is allowed to stand at room temperature for 30 min. and then is heated at 70°C for 2 hours.

The reaction mixture is diluted with water and is extracted with methylene chloride and ethyl ether sequentially. The aqueous phase is acidified with dilute aqueous hydrochloric acid and reextracted with methylene chloride and ethyl ether. All the organic extracts are dried over magnesium sulfate, rotoevaporated and placed under high vacuum. The resulting orange solid is triturated with benzene, filtered and dried overnight to give the title compound: m.p. 250—252°C (dec); IR (KBr) 3350, 3275, 1335, 1178, 1156 and 845 $cm^{-1}$.

| | |
|---|---|
| *Analysis for:* | $C_7H_6N_2O_4S_3$ |
| *Calculated:* | C, 30.20; H, 2.17; N, 10.07 |
| *Found:* | C, 34.64; H, 3.64; N, 8.01 |

B. 6-sulfamoyl-3-thiomethyl benzisothiazole 1,1-dioxide

To a solution of 2.8 g (10 mmol) of A. above in 10 ml of dimethylformamide is added 937 ml (15 mmol) of iodomethane. The reaction mixture is allowed to stand at room temperature for 40 minutes and then is partitioned between aqueous sodium bicarbonate and methylene chloride. A precipitate is observed, separated by filtration, and extracted with ethyl acetate to give 297 mg of the title compound.

The aqueous phase is then extracted with ethyl acetate to give, after combination with the methylene chloride extract, drying over magnesium sulfate and rotoevaporation, 285 mg of alkylated product: m.p. 280—282°C IR (KBr) 3365, 3230, 1489, 1332, 1320, 1160 and 825 $cm^{-1}$.

| | |
|---|---|
| *Analysis for:* | $C_8H_8N_2O_4S_3$ |
| *Calculated:* | C, 32.86; H, 2.76; N, 9.58 |
| *Found:* | C, 34.67; H, 3.06; N, 9.91 |

C. N-[3-[3-[(1-piperidinyl)methyl]phenoxyl]propyl]-6-Sulfamoyl-1,2-benzisothiazol-3-amine 1,1-dioxide

To a gently refluxing solution of 372 mg (1.5 mmol) of 3-[3-[1-piperidinyl)methyl]phenoxy]propylamine in 50 ml of alumina-treated chloroform is added a suspension of 3-thiomethyl-6-sulfamoyl benzisothiazole 1,1-dioxide (438 mg, 1.5 mmol) in 50 ml of chloroform:acetonitrile (1:1).

The reaction mixture is maintained at reflux for 1 hr, cooled to room temperature and solvent removed *in vacuo*. The residue is triturated with isopropyl ether and the resulting yellow solid filtered and dried (100°C, high vaccum) to give 482 mg of title adduct: m.p. 155—159°C, IR (KBr), 1612, 1280, 1140 and 822$cm^{-1}$.

| | |
|---|---|
| *Analysis for:* | $C_{22}H_{28}N_4O_5S_2$ |
| *Calculated:* | C, 53.64; H, 5.73; N, 11.39 |
| *Found:* | C, 52.96; H, 5.71; N, 11.02 |

## Example 14

N-[3-[3-[1-piperidinyl)methyl]phenoxy]propyl]-6-bromo-1,2-benzisothiazol-3-amine 1,1-dioxide.

A mixture of 4.00 g (15 mmol) 6-bromo-1,2-benzisothiazol-3(2*H*)-one 1,1-dioxide and 3.57, (17 mmol) phosphorus pentachloride in 5 ml *o*-dichlorobenzene is heated from ambient temperature to 160°C over one hour and is held at that temperature for one additional hour. Upon cooling liquids are removed *in vacuo* and the residual 6-bromo-3-chlorobenzisothiazole 1,1-dioxide is suspended in acetonitrile and added portionwise to a solution of 3.7 g (15 mmol) 3-[3-[(1-piperidinyl)methyl]phenoxy]propylamine in refluxing acetonitrile. Heating at reflux is continued for one hour after completion of the addition, after which the reaction mixture is cooled to room temperature. The solution is decanted away from a small quantity of gum and the solvent is removed on a rotoevaporator. The resulting oil is dissolved in absolute ethanol, from which the hydrochloride salt of the title compound precipitates. m.p. 217—220°C. IR (KBr) 1625, 1290, 1165, 1150 $cm^{-1}$.

| | |
|---|---|
| *Analysis for:* | $C_{22}H_{27}BrClN_3O_3S$ |
| *Calculated:* | C, 49.96; H, 5.15; N, 7.94 |
| *Found:* | C, 49.76; H, 5.21; N, 7.46 |

## Example 15

N-[3-[3-[1-piperidinyl)methyl]phenoxy]propyl]-5-fluoro-1,2-benzisothiazol-3-amine 1,1-dioxide

A. 3-chloro-5-fluoro-1,2-benzisothiazole 1,1-dioxide

A slurry of 5-fluoro-1,2-benzisothiazolin-3-one 1,1-dioxide (1.80 g, 8.948 mmoles) and phosphorus pentachloride (1.96 g, 9.412 mmoles) in *o*-dichlorobenzene (9.0 ml) is heated under dry nitrogen. A 70°C hydrogen chloride evolution appears to start. At ca. 105°C complete solution occurs. The temperature is raised and maintained at 175—185°C for 2 1/2 hours. After cooling to room temperature the solution is partially stripped at 70°C *in vacuo* to remove the phosphorus oxychloride formed. Addition of pentane (80 ml) with stirring gives a light tan solid, 0.91 g, m.p. 142—147°, IR (KBr) 1548, 1351, 1170 $cm^{-1}$ (in DMSO) 7.74—8.52(m).

B. N-[3-[3-[1-piperidinyl)methyl]phenoxy]propyl]-5-fluoro-1,2-benzisothiazol-3-amine, 1,1-dioxide

To a refluxing solution of 3-[3-(1-piperidinylmethyl)phenoxy]propylamine (0.870 g, 3.503 mmoles) in dried chloroform (35 ml) under dry nitrogen is added over 45 minutes a solution of 3-chloro-5-fluoro-1,2-benzisothiazole 1,1-dioxide (0.808 g, 3.679 mmoles) in dried chloroform (20 ml) while maintaining reflux. After the addition is complete refluxing is continued for 15 minutes and the solution allowed to come to room temperature. Evaporation *in vacuo* gives an orange solid. Recrystallization from isopropanol gives the hydrochloride salt of the title compound as a light tan solid. m.p. 224—226° after softening from 200°C. IR (KBr) 2950, 1600, 1302, 1171 $cm^{-1}$; NMR (in DMSO) 1.76 (6H, s), 2.2 (2H, t), 2.8 (2H, m), 3.3 (3H, m), 3.6 (2H, t) 4.2 (3H, m) 7.0—8.5 (7H, m), 10.1 (1H, s), 10.6 (1H, m)

*Analysis for:* $C_{22}H_{26}FN_3O_3S_3.HCl$
*Calculated:* C, 56.46; H, 5.81; N, 8.98; Cl, 7.58
*Found:* C, 56.26; H, 5.87; N, 9.04; Cl, 7.50

Example 16

N-[3-[3-(4-morpholinylmethyl)phenoxy]propyl)-1,2-benzisothiazol-3-amine 1,1-dioxide

A. 3-(4-morpholinylmethyl)phenol

To a solution of 15 g (.123 mole) of 3-hydroxybenzaldehyde in 150 ml of ethanol is added 26.5 g (.304 mole) of morpholine. Then 4.7 g (.124 mole) of sodium borohydride is added portionwise over 1 hour. The solution is stirred for 1 hour and allowed to sit overnight. The solvent is then evaporated via a rotary evaporator and the residue triturated with 200 ml cold water. After acidification with concentrated HCl the solution is extracted with ethyl acetate. The aqueous layer is basified with ammonium hydroxide and extracted again with 100 ml ethyl acetate. The organic layer is dried over $MgSO_4$, filtered and evaporated. The residue is recrystallized twice from acetonitrile to give 6.3 g of product m.p. 113—15°C.

*Analysis for:* $C_{11}H_{15}NO_2$
*Calculated:* C, 68.37; H, 7.82; N, 7.25
*Found:* C, 68.17; H, 7.73; N, 7.35

B. 2-[3-[3-(4-morpholinylmethyl)phenoxy]propyl]-1H-isoindole-1,3(2H)-dione

To 1.58 g (.033 moles) of 50% sodium hydride is added 15 ml DMF: 6.3 g (.033 moles) of 3-(4-morpholinylmethyl)phenol, dissolved in 30 ml DMB, is added dropwise to the stirred suspension at room temperature. This mixture is stirred 20 minutes and then N-(3-bromopropyl)-phthalimide, 9 g (.033 moles) is added to the mixture and stirred. A clear, amber solution results. After 2 hours, this solution is poured into ice-water and extracted with 100 ml of chloroform. The organic layer is washed once with 100 ml 5% NaOH, and once with 100 ml water, dried over $MgSO_4$, filtered and evaporated. The residue is dissolved in ether and washed again with 50 ml water. Then the organic layer is dried over $MgSO_4$, filtered and evaporated. The residue rapidly forms a white solid. This recrystallized from acetonitrile. m.p. 90—93°C (yield=39 g).

*Analysis for:* $C_{22}H_{24}N_2O_4$
*Calculated:* C, 69.45; H, 6.36; N, 7.37
*Found:* C, 69.07; H, 6.45; N, 7.21

C. 3-[3-(4-morpholinylmethyl)phenoxy]-1-propanamine

A solution of 4.35 g (.011 moles) of 2-[3-[3-(4-morpholinylmethyl)phenoxy]propyl]-1*H*-isoindole-1, 2(2*H*)-dione in 85 ml of ethanol is added to 7 ml (.14 moles) of hydrazine hydrate and stirred at room temperature for 2 hours. The precipitate is filtered off and the filtrate evaporated to dryness. Benzene is added and distilled off to azeotrope water. Then chloroform is added and the precipitate formed is filtered off. The filtrate is dried over $MgSO_4$, filtered and evaporated, leaving 1.7 g of low viscosity, yellow-liquid. This crude product is used without further purification.

D. N-[3-[3-(4-morpholinylmethyl)phenoxy]propyl]-1,2-benzisothiazol-3-amine 1,1-dioxide

A solution of 1.37 g (.0068 moles) of 3-chlorobenzoisothiazole 1,1-dioxide is added dropwise to 1.7 g (.0068 moles) of 3-[3-(4-morpholinylmethyl)phenoxy]-1-propanamine in 150 ml of gently refluxing chloroform, over 30 minutes. The reflux is continued for 15 minutes after the end of the addition, then the reaction mixture is evaporated to dryness. The residue is treated with ethanolethyl acetate, causing the formation of a yellow-white solid, m.p. 210°C (dec). Recrystallization of the crude product from ethanol gave the hydrochloride salt of the title compound having a m.p. of 215°C (dec) (1.7 g yield).

*Analysis for:* $C_{21}H_{26}ClN_3O_4$
*Calculated:* C, 55.80; H, 5.80; N, 9.30
*Found:* C, 55.98; H, 6.03; N, 9.55

Example 17

Preparation of Amines

Following the procedures of Example 16A—C, there are prepared the following intermediate amines:

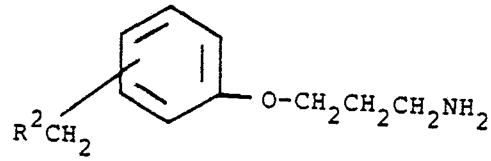

(1) 3-(3-aminopropoxy)-*N,N*-dipropylbenzenemethanamine, dihydrochloride.
$R^2H$:dipropylamine

Melting point of amine: 200°C (dec.)

| | | | |
|---|---|---|---|
| *Analysis for:* | $C_{16}H_{30}Cl_2N_2O$ | | |
| *Calculated:* | C, 56.97; | H, 8.97; | N, 8.31 |
| *Found:* | C, 56.78; | H, 8.67; | N, 8.26 |

(2) 3-(3-aminopropoxy)-*N,N*-diethylbenzenemethanamine
$R^2H$:diethylamine
Melting point of amine: 147°C (dec.)

| | | | |
|---|---|---|---|
| *Analysis for:* | $C_{14}H_{26}Cl_2N_2O$ | | |
| *Calculated:* | C, 54.37; | H, 8.47; | N, 9.06 |
| *Found:* | C, 54.58; | H, 8.29; | N, 8.93 |

(3) 3-[3-(1-pyrrolidinylmethyl)phenoxy]-1-propanamine
$R^2H$:pyrrolidine
Melting point of amine: product is an oil

(4) 3-(3-aminopropoxy)-*N,N*-dimethylbenzenemethanamine, dihydrochloride
$R^2H$:dimethylamine
Melting point of amine: 208—10°C,

| | | | |
|---|---|---|---|
| *Analysis for:* | $C_{12}H_{22}Cl_2N_2O$ | | |
| *Calculated:* | C, 51.25; | H, 7.89; | N, 9.96 |
| *Found:* | C, 51.57; | H, 7.75; | N, 9.91 |

(5) 3(3-aminopropoxy)-*N,N*-dibutylbenzenemethanamine
$R^2H$:n-dibutylamine
Melting point of amine:product is an oil

(6) 3-[3-(4-thiomorpholinylmethyl)phenoxy]-1-propanamine
$R^2H$:thiomorpholine

(7) 3-[3-(hexahydro-1*H*-azepin-1-ylmethyl)phenoxy]-1-propanamine
$R^2H$:hexamethyleneimine

| | | | |
|---|---|---|---|
| *Analysis for:* | $C_{16}H_{26}N_2O$ | | |
| *Calculated:* | C, 73.25; | H, 9.99; | N, 10.68 |
| *Found:* | C, 73.04; | H, 10.12; | N, 10.27 |

(8) 3-[3-(3-thiazolidinylmethyl)phenoxy]-1-propanamine, dihydrochloride hemihydrate
$R^2H$:thiazolidine
Melting point of amine: salt has m.p. of 160—5°C free base is an oil

| | | | |
|---|---|---|---|
| *Analysis for:* | $C_{13}H_{22}Cl_2N_2OS$ 1/$H_2O$ | | |
| *Calculated:* | C, 46.70; | H, 6.93; | N, 8.38 |
| *Found:* | C, 46.92; | H, 6.37; | N, 8.07 |

(9) 3-[3-[(octahydro-1 (2*H*)-azocinyl)methyl)phenoxy]-1-propanamine
$R^2H$:heptamethyleneimine

(10) 3-(3-aminopropoxy)-N-cyclohexylbenzenemethanamine, dihydrochloride
$R^2H$:cyclohexylamine
Melting point of amine: salt has m.p. of 183—7°C

(11) 3-(3-aminopropoxy)-*N*-cyclopentylbenzenemethanamine, dihydrochloride
$R^2H$-cyclopentylamine
Melting point of amine: salt has m.p. of 183—5°C

| | | | |
|---|---|---|---|
| *Analysis for:* | $C_{15}H_{26}Cl_2N_2O$ | | |
| *Calculated:* | C, 56.07; | H, 8.16; | N, 8.72 |
| *Found:* | C, 56.03; | H, 8.04; | N, 8.63 |

(12) N-[[3-(3-aminopropoxy)phenyl]methyl]-2-furanmethanamine, dihydrochloride
$R^2H$:furfurylamine
Melting point of amine:salt has m.p. of 238—40°C

| | | | |
|---|---|---|---|
| *Analysis for:* | $C_{15}H_{22}Cl_2N_2O_2$ | | |
| *Calculated:* | C, 54.06; | H, 6.66; | N, 8.41 |
| *Found:* | C, 53.88; | H, 6.55; | N, 8.46 |

(13) 3-(3-aminopropoxy)-*N*-(phenylmethyl)benzenemethanamine, dihydrochloride
$R^2H$:benzylamine
Melting point of amine:salt has m.p. of 248—50°C

| | | | |
|---|---|---|---|
| *Analysis for:* | $C_{17}H_{24}Cl_2N_2O$ | | |
| *Calculated:* | C, 59.47; | H, 7.05; | N, 8.16 |
| *Found:* | C, 59.13; | H, 6.90; | N, 8.16 |

(14) 3-(3-aminopropoxy)-N,N-bis(2-methylpropyl)benzenemethanamine
$R^2H$:diisobutylamine
Melting point of amine:product is an oil

(15) 3-(3-aminopropoxy)-N-butylbenzenemethanamine, dihydrochloride
$R^2H$:n-butylamine
Melting point of amine:salt has m.p. of 235—9°C

| | | | |
|---|---|---|---|
| *Analysis for:* | $C_{14}H_{26}Cl_2N_2O$ | | |
| *Calculated:* | C, 54.37; | H, 8.47; | N, 9.06 |
| *Found:* | C, 54.26; | H, 8.34; | N, 9.02 |

(16) 3-3(3-aminopropoxy)-*N*-phenylaminobenzenemethanamine, dihydrochloride
$R^2H$:aniline
Melting point of amine:salt has m.p. of 157—60°C

| | | | |
|---|---|---|---|
| *Analysis for:* | $C_{16}H_{22}N_2Cl_2O$ | | |
| *Calculated:* | C, 55.36; | H, 6.74; | N, 8.51 |
| *Found:* | C, 58.11; | H, 6.62; | N, 8.40 |

(17) 3-(3-aminopropoxy)-*N*-methylpentylbenzenemethanamine
$R^2H$:*N*-methyl-*N*-amylamine
Melting point of amine:product is an oil

(18) 3-(3-aminopropoxy)-*N*-butyl-propylbenzenemethanamine
$R^2H$:n-butyl-n-propylamine
Melting point of amine:product is an oil

(19) 3-(3-aminopropoxy)-*N*-butyl-*N*-methylbenzenemethanamine
$R^2H$:N-methyl-n-butylamine
Melting point of amine:product is an oil

| | | | |
|---|---|---|---|
| *Analysis for:* | $C_{15}H_{26}N_2O$ | | |
| *Calculated:* | C, 71.95; | H, 10.47; | N, 11.19 |
| *Found:* | C, 71.53; | H, 10.22; | N, 10.55 |

(20) 3-(3-aminopropoxy)-*N,N*-dipentylbenzenemethanamine
$R^2H$:*N,N*-diamylamine
Melting point of amine:product is an oil

| | | | |
|---|---|---|---|
| *Analysis for:* | $C_{20}H_{36}N_2O$ | | |
| *Calculated:* | C, 74.95; | H, 11.32; | N, 8.74 |
| *Found:* | C, 74.97; | H, 10.81; | N, 8.49 |

(21) 3-[4-(1-piperidinylmethyl)phenoxy]-1-propanamine
Starting materials:piperidine and 4-hydroxybenzaldehyde
Melting point of amine:product is an oil

| | | | |
|---|---|---|---|
| *Analysis for:* | $C_{15}H_{24}N_2O$ | | |
| *Calculated:* | C, 72.54; | H, 9.74; | N, 11.28 |
| *Found:* | C, 72.31; | H, 9.78; | N, 11.72 |

Examples 18—38

Following the procedure of Example 16D, there are prepared the following final products:

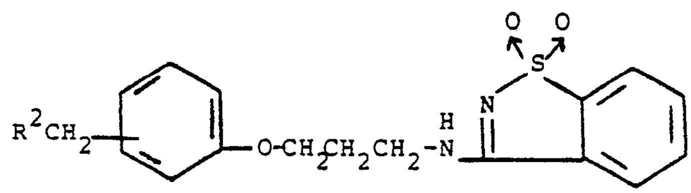

18. *N*-[3-[3-[(dipropylamino)methyl]phenoxy]propyl]-1,2-benzisothiazol-3-amine 1,1-dioxide, hydrochloride
Starting amine: Example 17(1)
Melting point: 205—8°C (dec.)

| | | | |
|---|---|---|---|
| *Analysis for:* | $C_{23}H_{32}N_3O_3SCl$ | | |
| *Calculated:* | C, 59.27; | H, 6.92; | N, 9.02 |
| *Found:* | C, 59.38; | H, 6.96; | N, 8.87 |

19. *N*-[3-[3-[(diethylamino)methyl]phenoxy]propyl]-1,2-benzisothiazol-3-amine 1,1-dioxide, hydrochloride
Starting amine: Example 17(2)
Melting Point: 184—7°C (dec.)

| | | | |
|---|---|---|---|
| *Analysis for:* | $C_{21}H_{28}ClN_3O_3S$ | | |
| *Calculated:* | C, 57.59; | H, 6.44; | N, 9.60 |
| *Found:* | C, 57.74; | H, 6.54; | N, 9.39 |

20. *N*-[3-[3-(1-pyrrolidinylmethyl)phenoxy]propyl]-1,2-benzisothiazol-3-amine 1,1-dioxide, hydrochloride, 1/3 hydrate
Starting amine Example 17(3)
Melting point: 193—5°C

| | | | |
|---|---|---|---|
| *Analysis for:* | $C_{21}H_{25}H_3O_3S \cdot HCl \cdot 1/2\ H_2O$ | | |
| *Calculated:* | C, 57.07; | H, 6.06; | N, 9.50 |
| *Found:* | C, 57.14; | H, 5.97; | N, 9.40 |

21. *N*-[3-[3-[(dimethylamino)methyl]phenoxy]propyl]-1,2-benzisothiazol-3-amine 1,1-dioxide, hydrochloride, hemihydrate
Starting amine: Example 17(4)
Melting Point: 188—92°C (dec.)

| | | | |
|---|---|---|---|
| *Analysis for:* | $C_{19}H_{24}ClN_3O_3S \cdot 1/2\ H_2O$ | | |
| *Calculated:* | C, 54.47; | H, 6.02; | N, 10.03 |
| *Found:* | C, 54.32; | H, 5.71; | N, 10.17 |

22. *N*-[3-[3-[(dibutylamino)methyl]phenoxy]propyl]-1,2-benzisothiazol-3-amine 1,1-dioxide, hydrochloride, hemihydrate
Starting amine: Example 17(5)
Melting Point: 157°C (dec.)

| | | | |
|---|---|---|---|
| *Analysis for:* | $C_{25}H_{36}ClN_3O_3S \cdot 1/2\ H_2O$ | | |
| *Calculated:* | C, 59.68; | H, 7.41; | N, 8.35 |
| *Found:* | C, 59.57; | H, 7.16; | N, 8.42 |

23. N-[3-[3-[(4-thiomorpholinyl)methyl]phenoxy]propyl]-1,2-benzisothiazol-3-amine 1,1-dioxide, hydrochloride
Starting amine: Example 17(6)
Melting point: 208—12°C

| | | | |
|---|---|---|---|
| *Analysis for:* | $C_{21}H_{26}ClN_3O_3S_2$ | | |
| *Calculated:* | C, 53.89; | H, 5.60; | N, 8.98 |
| *Found:* | C, 53.93; | H, 5.69; | N, 8.78 |

24. *N*-[3-[3-[(hexahydro-1*H*-azepin-1-yl)methyl]phenoxy)propyl]-1,2-benzisothiazol-3-amine 1,1-dioxide, hydrochloride
Starting amine: Example 17(7)
Melting point: 1902°C

| | | | |
|---|---|---|---|
| *Analysis for:* | $C_{23}H_{29}N_3O_3S \cdot HCl$ | | |
| *Calculated:* | C, 59.52; | H, 6.52; | N, 9.06 |
| *Found:* | C, 59.11; | H, 6.70; | N, 8.84 |

25. *N*-[3-[3-[(3-thiazolidinyl)methyl]phenoxy]propyl]-1,2-benzisothiazol-3-amine 1,1-dioxide, hydrochloride
Starting amine: Example 17(8)
Melting point: 162—6°C

| | | | |
|---|---|---|---|
| *Analysis for:* | $C_{24}H_{20}ClN_3O_3S_2$ | | |
| *Calculated:* | C, 52.91; | H, 5.33; | N, 9.26 |
| *Found:* | C, 52.91; | H, 5.22; | N, 9.10 |

26. *N*-[3-[3-[(octahydro-1(2H)-azocinyl)methyl]phenoxy]-propyl]-1,2-benzisothiazol-3-amine 1,1-dioxide, hydrochloride
Starting amine: Example 17(9)
Melting point: 163—5°C

| | | | |
|---|---|---|---|
| *Analysis for:* | $C_{24}H_{32}ClN_3O_3S$ | | |
| *Calculated:* | C, 60.30; | H, 6.75; | N, 8.79 |
| *Found:* | C, 60.03; | H, 6.86; | N, 8.74 |

27. *N*-[3-[3-[(cyclohexylamino)methyl]phenoxy]propyl]-1,2-benzisothiazol-3-amine 1,1-dioxide, hydrochloride
Starting amine: Example 17(10)
Melting point: 198—203°C

| | | | |
|---|---|---|---|
| *Analysis for:* | $C_{23}H_{30}ClN_3O_3S$ | | |
| *Calculated:* | C, 59.63; | H, 6.52; | N, 9.06 |
| *Found:* | C, 59.42; | H, 6.63; | N, 8.97 |

28. *N*-[3-[3-[(cyclopentylamino)methyl]phenoxy]propyl]-1,2-benzisothiazol-3-amine 1,1-dioxide, hydrochloride
Starting amine: Example 17(11)
Melting point: 198—201°C

| | | | |
|---|---|---|---|
| *Analysis for:* | $C_{22}H_{28}ClN_3O_3S$ | | |
| *Calculated:* | C, 58.82; | H, 6.27; | N, 9.34 |
| *Found:* | C, 58.77; | H, 6.46; | N, 9.08 |

29. *N*-[3-[3-[[[(2-furanyl)methyl]amino]methyl)phenoxy]-propyl]-1,2-benzisothiazol-3-amine 1,1-dioxide, hydrochloride.
Starting amine: Example 17(12)
Melting point: 169-71°C

| | | | |
|---|---|---|---|
| *Analysis for:* | $C_{22}H_{24}ClN_3O_4S$ | | |
| *Calculated:* | C, 57.20; | H, 5.24; | N, 9.10 |
| *Found:* | C, 57.02; | H, 5.29; | N, 8.99 |

30. *N*-[3-[3-[(benzylamino)methyl]phenoxy]propyl]-1,2-benzisothiazol-3-amine 1,1-dioxide, hydrochloride, quarter hydrate
Starting amine: Example 17(13)
Melting point: 119°C (dec.)

| | | | |
|---|---|---|---|
| *Analysis for:* | $C_{24}H_{26}ClN_3O_3S$ 1/4 $H_2O$ | | |
| *Calculated:* | C, 60.49; | H, 5.61; | N, 8.82 |
| *Found:* | C, 60.38; | H, 5.61; | N, 8.54 |

31. *N*-[3-[[3-[bis(2-methylpropyl)amino]methyl]phenoxy]propyl]-1,2-benzisothiazol-3-amine 1,1-dioxide
Starting amine: Example 17(14)
Melting Point: 141—3°C

| | | | |
|---|---|---|---|
| *Analysis for:* | $C_{22}H_{35}N_3O_3S$ | | |
| *Calculated:* | C, 65.61; | H, 7.71; | N, 9.18 |
| *Found:* | C, 65.39; | H, 7.63; | N, 9.16 |

32. *N*-[3-[3-[(butylamino)methyl]phenoxy]propyl]-1,2-benzisothiazol-3-amine 1,1-dioxide, hydrochloride, hemihydrate.
Starting amine: Example 17(15)
Melting point: 95—100°C

| | | | |
|---|---|---|---|
| *Analysis for:* | $C_{21}H_{28}ClN_3O_3S \cdot 1/2\ H_2O$ | | |
| *Calculated:* | C, 56.43; | H, 6.54; | N, 9.40 |
| *Found:* | C, 56.47; | H, 6.42; | N, 9.19 |

33. *N*-[3-[3-[(phenylamino)methyl]phenoxy]propyl]-1,2-benzisothiazol-3-amine 1,1-dioxide.
Starting amine: Example 17(16)
Melting point: 177—80°C

| | | | |
|---|---|---|---|
| *Analysis for:* | $C_{23}H_{23}N_3O_3S$ | | |
| *Calculated:* | C, 65.54; | H, 5.50; | N, 9.97 |
| *Found:* | C, 65.40; | H, 5.59; | N, 9.94 |

34. *N*-[3-[3-[(methylpentylamino)methyl)phenoxy]propyl]-1,2-benzisothiazol-3-amine 1,1-dioxide
Starting amine: Example 17(17)
Melting Point: 113—15°C

| | | | |
|---|---|---|---|
| *Analysis for:* | $C_{23}H_{31}N_3O_3S$ | | |
| *Calculated:* | C, 64.31; | H, 7.27; | N, 9.78 |
| *Found:* | C, 64.17; | H, 7.24; | N, 9.51 |

35. *N*-[3-[3-[(butylpropylamino)methyl]phenoxy]propyl]-1,2-benzisothiazol-3-amine 1,1-dioxide
Starting amine: Example 17(18)
Melting point: 124—8°C

| | | | |
|---|---|---|---|
| *Analysis for:* | $C_{24}H_{33}N_3O_3S$ | | |
| *Calculated:* | C, 64.98; | H, 7.50; | N, 9.47 |
| *Found:* | C, 65.21; | H, 7.42; | N, 9.70 |

36. *N*-[3-[3-[(butylmethylamino)methyl]phenoexy]propyl]-1,2-benzisothiazol-3-amine 1,1-dioxide
Starting amine: Example 17(19)
Melting point: 114—6°C

| | | | |
|---|---|---|---|
| *Analysis for:* | $C_{22}H_{29}N_3O_3S$ | | |
| *Calculated:* | C, 63.58; | H, 7.03; | N, 10.11 |
| *Found:* | C, 63.60; | H, 7.12; | N, 10.10 |

37. *N*-[3-[3-[(dipentylamino)methyl]phenoxy]propyl]-1,2-benzisothiazol-3-amine, 1,1-dioxide
Starting amine: Example 17(20)
Melting point: 109—11°C

| | | | |
|---|---|---|---|
| *Analysis for:* | $C_{27}H_{39}N_3O_3S$ | | |
| *Calculated:* | C, 66.77; | H, 8.09; | N, 8.65 |
| *Found:* | C, 66.44; | H, 8.07; | N, 8.41 |

38. N-[3-[4-[(1-piperidinyl)methyl]phenoxy]propyl]-1,2-benzisothiazol-3-amine 1,1-dioxide, hydrochloride
Starting amine: Example 17(21)
Melting point: 211—3°C

| | | | |
|---|---|---|---|
| *Analysis for:* | $C_{22}H_{27}N_3O_3S \cdot HCl$ | | |
| *Calculated:* | C, 58.72; | H, 6.27; | N, 9.34 |
| *Found:* | C, 58.66; | H, 6.33; | N, 9.33 |

## Example 39

The guinea pig heart atrium test is carried out as follows:

A guinea pig right atrium is suspended at 1 g tension (isometric) in a thermostatically controlled (32°C) tissue bath (10 ml) containing oxygenated (95% $O_2$; 5% $CO_2$) Krebs-Haenseleit buffer (pH 7.4). The tissue is allowed to stabilise over 1 hour. Individual contractions are recorded with a force-displacement transducer through a strain gauge coupler. A control dose-response curve to histamine in the above described tissue bath is obtained after which the tissue is washed 3 times and allowed to re-equilibrate to basal rate. The test compound is added to the tissue bath at the desired final concentration. Thirty minutes after addition of the compound, a fresh histamine dose response curve is again obtained. Then the response to histamine in the presence of antagonist is compared to the histamine control response. This procedure is repeated, using fresh tissues, for each concentration of antagonist tested. The result is expressed as the apparent dissociation constant ($pA_2$) of the $H_2$ antagonist as determined by standard procedures. Cimetidine is used as the standard for this test.

The results for a series of compounds of the invention are as follows:—

| Compound of Example No. | $pA_2$ Value |
|---|---|
| Cimetidine | 6.5 |
| 1 | 7.0 |
| 2 | 8.1 |
| 3 | 8.7 |
| 4 | 6.6 |
| 5 | 7.3 |
| 6 | 7.7 |
| 7 | 7.5 |
| 8 | 7.7 |
| 9 | 6.7 |
| 10 | 7.3 |
| 11 | 7.1 |
| 12 | 8.0 |
| 16 | <7.0 |
| 18 | 7.8 |
| 19 | ~6.7 |
| 20 | 7.6 |
| 21 | 7.1 |
| 22 | 8.2 |
| 23 | 7.6 |
| 24 | 8.24 |
| 25 | 7.39 |
| 26 | 7.7 |
| 27 | 7.35 |
| 28 | 7.6 |
| 29 | 7.16 |
| 30 | 7.2 |
| 31 | ~7.2 |
| 32 | 7.2 |
| 34 | ~ 8.0 |
| 35 | $1.4 \times 10^{-8}$M* |
| 36 | $4.8 \times 10^{-8}$M* |
| 38 | 7.63 |

*These values are the $K_B$ values for the compounds tested, and the $K_B$ differs from the $A_2$ value only by the fact that the $A_2$ value reflects the results of three experiments, while the $K_B$ value represents the results of only one experiment.

The results show that the compounds of the invention are extremely active $H_2$ antagonists, being significantly more active than the standard compound cimetidine.

Example 40

The procedure for testing gastric secretion in the rat, a modification of the procedure of Shay et al., *Gastroenterology*, 26, 906—13 (1954) is carried out as follows:

Male Charles River rats weighing 200—300 grams are deprived of good but not water for 24 hours prior to use. Water is, however, withheld during the experiment. The rats are weighed, anesthetized, and the pylorus ligated according to the method of Shay et al. Treatment or vehicle control is then administered interduodenally (i.d.) Rats are housed 2/cage and sacrificed with $CO_2$ four hours after ligation. The stomachs are removed, rinsed, and contents emptied into a graduated centrifuge tube. The tubes are centrifuged for 20 minutes for 2,000 RPM and the volume of gastric juice recorded. Any samples obviously contaminated by feces, food or hemolysis are eliminated. An aliquot of each is frozen for later analysis of $Na^+$, $K^+$ and $C1^-$ concentration. The pH is measured and 1 ml of gastric juice is titrated with 0.1N NaOH to a pH of 7.0—7.4. Titratable acid output is calculated in microequivalents and the percent inhibition of acid output is calculated as follows:

$$\text{\% Inhibition of Acid Output} = \frac{\text{Acid Output (Control)} - \text{Acid Output (Drug)}}{\text{Acid Output (Control)}} \times 100$$

The test results for some of the compounds of the invention are for the known $H_2$ antagonists ranitidine and tiotidine are as follows:

| Compound of Example No. | Dose (mg/kg) | % Inhibition |
|---|---|---|
| 1 | 4 | 79 |
| 16 | 32 | 39 |
| 18 | 32 | 90 |
| | 16 | 89 |
| | 8 | 67 |
| 19 | 32 | 78 |
| | 16 | 52 |
| 20 | 2 | 50 |
| 21 | 32 | 72 |
| 22 | 32 | 68 |
| | 16 | 53 |
| | 8 | 62 |
| | 4 | 42 |
| 24 | 8 | 50 |
| 25 | 4 | 48 |
| 28 | 32 | 49 |
| | 16 | 75 |
| 29 | 32 | 50 |
| 35 | 32 | 55 |
| 36 | 32 | 84 |
| 37 | 32 | 38 |
| 38 | 16 | 93 |
| | 4 | 52 |
| | 1 | 34 |
| Ranitidine | 4 | 43 |
| tiotidine | 4 | 16 |

The results show the compounds of the invention to have significant activity in inhibiting gastric acid secretion.

Example 41

The procedure for testing the ability of the compounds of the invention to inhibit the secretion of acidic gastric juice in dogs as follows:

A female pure bred beagle (7—10 kg) having Pavlov gastric pouches is fasted overnight with water *ab lib.* The animal is orally dosed and thirty minutes later it is fed to induce gastric acid secretions. Gastric acid samples are then collected every 15 minutes. The volume of each sample is measured and aliquots are titrated to neautrality with 0.1 N NaOH to determine acid concentration. The results are reported as the dose at which there is obtained a 50% inhibition of the total gastric acid output ($ID_{50}$).

The results for some compounds of the invention and the known $H_2$ antagonists cimetidine and ranitidine are presented below:

| Compound of Example No. | $ID_{50}$ (mg/kg) |
|---|---|
| 1 | 0.8 |
| 18 | 1.2 |
| 20 | 1.4 |
| 24 | 1.2 |
| 38 | 2.0 |
| cimetidine | 6.0 |
| ranitidine | 2.3 |

The results show that the compounds of the invention are extremely active in reducing gastric acid secretions in the dog at very low dosage levels, and that the levels are below the levels at which the same reduction in gastric acid secretions is attained by the known $H_2$ antagonists cimetidine and ranitidine.

**Claims for the Contracting States: BE CH DE FR IT LI LU NL SE**

1. A compound having the formula:

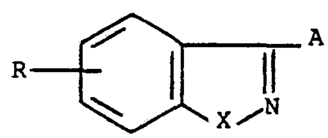

(I)

wherein R is hydrogen, mono- or dihalo, amino, nitro, cyano, hydroxy, trifluoromethyl, mercapto, lower alkyl, lower alkoxy, alkanoyl having 2 to 5 carbon atoms, cycloalkyl of 5 to 7 carbon atoms, carboxy, alkoxycarbonyl having 2 to 7 carbon atoms, mono- or di-lower alkyl substituted amino, alkanoylamino having 2 to 5 carbon atoms, lower alkyl thio, lower-alkylsulfonyl, sulfamoyl, lower alkyl substituted sulfamoyl, phenyl or phenyl substituted with halo, lower alkyl, lower alkoxy, trifluoromethyl, hydroxy, amino, cyano or nitro; X is $So_2$, SO, S or C=O; and A is an amine selected from

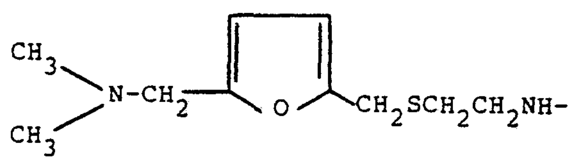

(Ia)

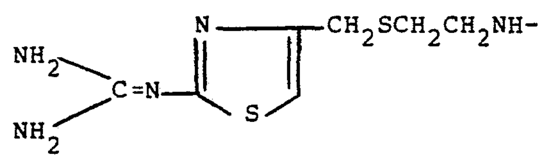

(Ib)

or

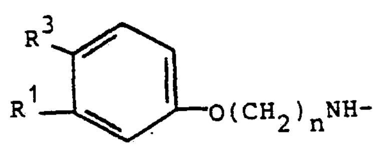

(Ic)

wherein $R^1$ is hydrogen or $R^2CH_2$ wherein $R^2$ is mono- or diloweralkylamino, mono- or di-N-lower alkylaminoloweralkyl, (2-furyl)methylamino, benzylamino, cycloalkylamino having 5 to 7 carbon atoms, 1-pyrrolidinyl, 1-piperidinyl, 1-hexahydroazepinyl, 1-octahydroazocynyl, 3-thiazolidinyl, 4-morpholinyl or 4-thiomorpholinyl; $R^3$ is hydrogen or (1-piperidinyl)methyl; the term, "halo" means fluoro, chloro or bromo and the term "lower" used in connection with alkyl or alkoxy means such groups containing 1 to 6 carbon atoms; with the proviso that when $R^3$ is (1-piperidinyl)methyl, $R^1$ is hydrogen; n is 1 to 4 and the pharmacologically acceptable salts thereof.

2. A compound of formula I as claimed in Claim 1 wherein A has formula Ic and $R^3$ is hydrogen.

3. A compound of formula I as claimed in Claim 1 or Claim 2 wherein A has formula Ic and n is 3.

4. A compound of formula I as claimed in any one of Claims 1 to 3 wherein A has formula Ic and $R^1$ is $R^2CH_2$ in which $R^2$ is diloweralkylamino, 1-pyrrolidinyl, 2-piperidinyl or 1-hexahydroazepinyl.

5. A compound of formula I as claimed in any one of Claims 1 to 4 wherein X is $SO_2$.

6. A compound of formula I as defined in Claim 1 selected from
N-[2-[[[5-[(dimethylamino)methyl]-2-furanyl]methyl]thio]ethyl]-1,2-benzisothiazol-3-amine 1, 1-dioxide;
N-[3-[3-[(1-piperidinyl)methyl)]phenoxy]propyl]-6-nitro-1,2-benzisothiazol-3-amine 1,1-dioxide;
N-[3-[3-[(1-piperidinyl)methyl]phenoxy]propyl]-6-amino-1,2-benzisothiazol-3-amine 1,1-dioxide;
N-[3-[3-[(1-piperidinyl)methyl]phenoxy]propyl]-6-chloro-1,2-benzisothiazol-3-amine 1, 1-dioxide;
N-[3-[3-[(1-piperidinyl)methyl]phenoxy]propyl]-6-fluoro-1,2-benzisothiazol-3-amine 1,1-dioxide;
N-[3-[3-[(1-piperidinyl)methyl]phenoxy]propyl]-5-6-dichloro-1,2-benzisothiazol-3-amine 1,1-dioxide;
N-[3-[3-[(1-piperidinyl)methyl]phenoxy]propyl]-5-methyl-1,2-benzisothiazol-3-amine 1,1-dioxide;
N-[3-[3-[(1-piperidinyl)methyl]phenoxy]propyl]-5-chloro-1,2-benzisothiazol-3-amine 1,1-dioxide;
N-[3-[3-[(1-piperidinyl)methyl]phenoxy]propyl]-5-methoxy-1,2-benzisothiazol-3-amine 1,1-dioxide;
N-[3-[3-[(1-piperidinyl)methyl]phenoxy]propyl]-6-sulfamoyl-1,2-benzisothiazol-3-amine 1,1-dioxide;
N-[3-[3-[(1-piperidinyl)methyl]phenoxy]propyl]-6-bromo-1,2-benzisothiazol-3-amine 1,1-dioxide;
N-[3-[3-[(1-piperidinyl)methyl]phenoxy]propyl]-5-fluoro-1,2-benzisothiazol-3-amine 1,1-dioxide;
N-[3-[3-[(4-morpholinylmethyl)phenoxy]propyl]-1,2-benzisothiazol-3-amine 1,1-dioxide;
N-[3-[3-[(diethylamino)methyl]phenoxy]propyl]-1,2-benzisothiazol-3-amine 1,1-dioxide;
N-[3-[3-[(dimethylamino)methyl]phenoxy]propyl]1,2-benzisothiazol-3-amin 1,1-dioxide;
N-[3-[3-[(dibutylamino)methyl]phenoxy]propyl]-1,2-benzisothiazol-3-amine 1,1-dioxide;
N-[3-[3-[(4-thiomorpholinyl)methyl]phenoxy]propyl]-1,2-benzisothiazol-3-amine 1,1-dioxide;
N-[3-[3-[(3-thiazolidinyl)methyl]phenoxy]propyl]-1,2-benzisothiazol-3-amine 1,1-dioxide;
N-[3-[3-[(octahydro-1(2H)-azocinyl)methyl]phenoxy]-propyl]-1,2-benzisothiazol-3-amine 1,1-dioxide;
N-[3-[3-[(cyclohexylamino)methyl]phenoxy]propyl]-1,2-benzisothiazol-3-amine 1,1-dioxide;
N-[3-[3-[(cyclopentylamino)methyl]phenoxy]propyl]-1,2-benzisothiazol-3-aminoe 1,1-dioxide;
N-[3-[3-[[[(2-furanyl)methyl]amino]methyl]phenoxy]-propyl]-1,2-benzisothiazol-3-amine 1,1-dioxide;
N-[3-[3-[(benzylamino)methyl]phenoxy]propyl]-1,2-benzisothiazol-3-amine 1,1-dioxide;
N-[3-[3-[[bis(2-methylpropyl)amino]methyl]phenoxy]-propyl]-1,2-benzisothiazol-3-amine 1,1-dioxide;
N-[3-[3-[(butylamino)methyl]phenoxy]propyl]-1,2-benzisothiazol-3-amine 1,1-dioxide;
N-[3-[3-[(methylpentylamino)methyl]phenoxy]propyl]-1,2-benzisothiazol-3-amine 1,1-dioxide;
N-[3-[3-[(butylpropylamino)methyl]-phenoxy]propyl]-1,2-benzisothiazol-3-amine 1,1-dioxide;
N-[3-[3-[(butylmethylamino)methyl]phenoxy]propyl]-1,2-benzisothiazol-3-amine 1,1-dioxide;
N-[3-[3-[(dipentylamino)methyl]phenoxy]propyl]-1,2-benzisothiazol-3-amine 1,1-dioxide;
N-[3-[4-[(1-piperidinyl)methyl]phenoxy]propyl]-1,2-benzisothiazol-3-amine 1,1-dioxide;

or a pharmacologically acceptable salt thereof.

7. A compound of formula I as defined in Claim 1 selected from [4-[[[2-[(1,2-benzisothiazol-3-yl)amino]ethyl]-thio]methyl]-2-thiazolyl]guanidine S,S'-dioxide;
3-[[3-[3-[(1-piperidinyl)methyl]phenoxy]propyl]-amino]]-1H-isoindol-1-one;
or a pharmacologically acceptable salt thereof.

8. A compound of formula I as defined in Claim 1 selected from
N-[3-[3-[(1-piperidinyl)methyl]-phenoxy]propyl]-1,2-benzisothiazol-3-amine-1,1-dioxide;
N-[3-[3-[(dipropylamino)methyl]phenoxy]propyl]-1,2-benzisothiazol-3-amine 1,1-dioxide;
N-[3-[3-[(1-pyrrolidinylmethyl)phenoxy]propyl]-1,2-benzisothiazol-3-amine, 1,1-dioxide;
N-[3-[3-[(hexahydro-1H-azepin-1-yl)methyl]phenoxy]-propyl]-1,2-benzisothiazol-3-amine 1,1-dioxide;
or a pharmacologically acceptable salt thereof.

9. A process for preparing a compound of formula I as defined in Claim 1 or a pharmacologically acceptable salt thereof which comprises:
a) reacting a compound of formula

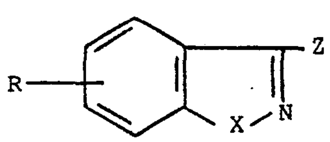

(II)

wherein X and R are as defined in Claim 1 and Z is halogen or $SR^4$ where $R^4$ is alkyl or aralkyl with an amine of formula H—A wherein A is as defined in Claim 1 to give a compound of formula I, or
b) reacting a compound of formula

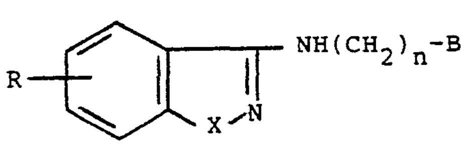

(III)

wherein X, R and n are as defined in Claim 1 and B is OH or L where L represents a leaving group with a compound of formula:

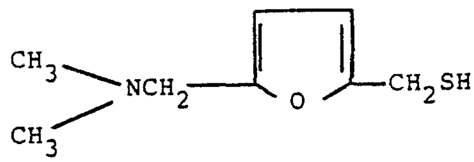

(IVa)

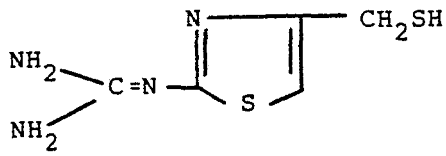

(IVb)

or

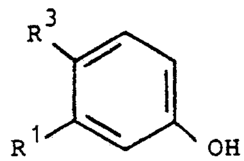

(IVc)

wherein $R^1$ and $R^3$ are as defined in Claim 1, with the provisos that (i) when a compound of formula IVa or IVb is used then n is 2 in the compound of formula III and (ii) when a compound of formula IVc is used then B is L; or

c) reacting a compound of formula

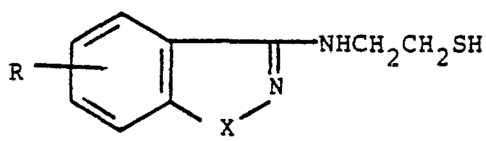

(V)

wherein R and X are as defined in Claim 1 with a compound of formula:

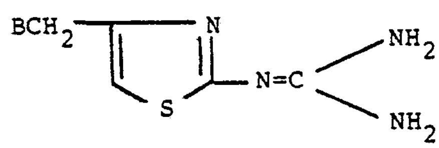

(VIa)

or

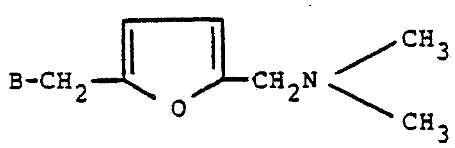

(VIb)

wherein B is OH or L as defined above to give a compound of formula I wherein A has the formula Ia or Ib; or

d) reacting an aldehyde of formula

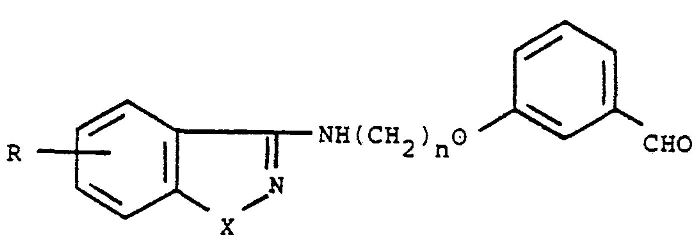

(VIIa)

with an appropriate amine of formula

$HR^2$ (VIIb)

in which formulae n, R, X and $R^2$ are as defined in Claim 1, under reductive amination conditions to give a corresponding compound of formula I wherein $R^1$ is $R^2CH_2$— and $R^3$ is hydrogen; or

e) reacting an aldehyde of formula

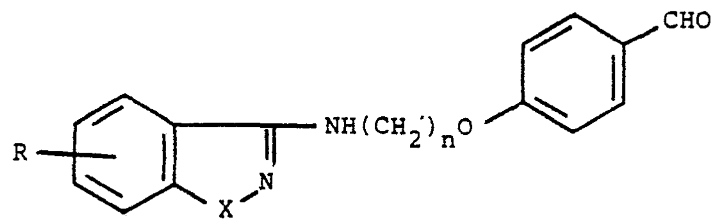

(VIIc)

wherein n, R and X are as defined in Claim 1 with piperidine under reductive amination conditions to give a compound of formula I wherein $R^3$ is (1-piperidinyl)methyl; or

f) reacting an aldehyde of formula

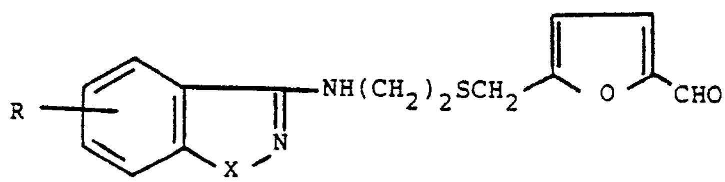

(VIId)

wherein X, R and N are as defined in Claim 1 with dimethylamine under reductive amination conditions to give a compound of formula I wherein A has formula Ia; or

g) reacting a compound of formula

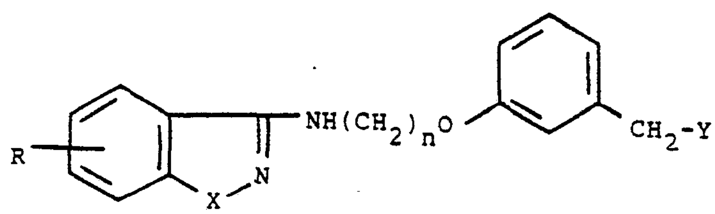

(VIII)

wherein n, X and R are as defined in Claim 1 and Y is OH or L wherein L is a leaving group as hereinbefore described, with an amine of formula $HR^2$ as defined above, the reaction being carried out in the presence of a nickel catalyst when Y is OH, to give a compound of formula I wherein A has the formula Ic and $R^1$ is $R^2CH_2$—; or

h) reacting a compound of formula

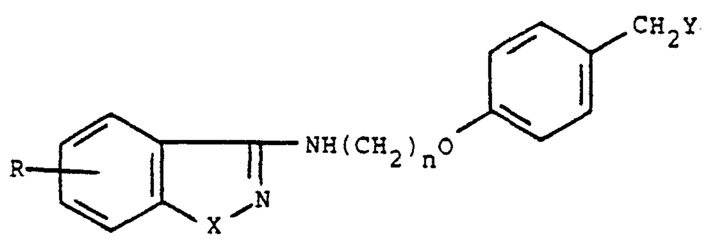

(VIIIa)

wherein Y, n, X and R are as hereinbefore defined with piperidine, the reaction being carried out in the presence of a nickel catalyst when Y is OH, to give a compound of formula I wherein A has the formula Ic and $R^3$ is (1-piperidinyl)methyl; or

i) reacting a compound of formula

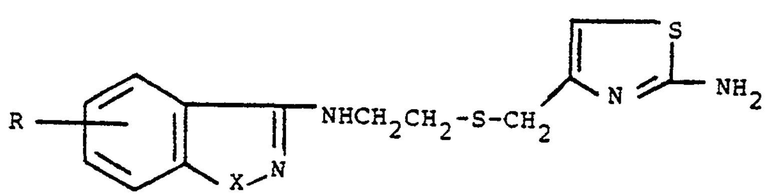

(IX)

wherein X and R are as defined in Claim 1 with a compound capable of converting —$NH_2$a to

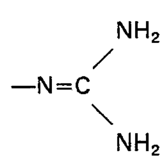

to give a compound of formula I wherein A has formula Ic; or

j) reacting a compound of formula

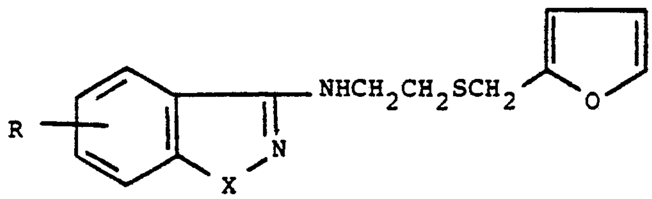

(X)

wherein X and R are as defined in Claim 1 with dimethylamine in the presence of formaldehyde or paraformaldehyde, to give a compound of formula I wherein A has formula Ia; or

k) reacting a compound of formula

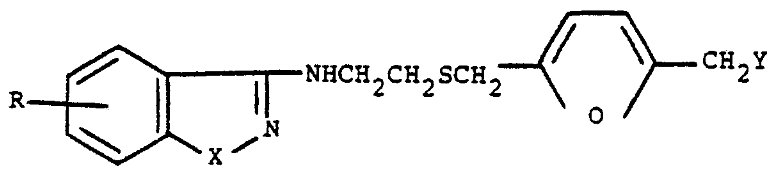

(XI)

wherein Y is OH or a leaving group L as hereinbefore defined, with dimethylamine, the reaction being carried out in the presence of a nickel catalyst when Y is OH; or

l) a basic compound of formula I is converted to a pharmacologically acceptable salt or a pharmacologically acceptable salt is converted to the free base form.

10. A compound of formula I or a pharmacologically acceptable salt thereof as claimed in any one of Claims 1 to 8 for use as an inhibitor of gastric acid secretion.

11. A pharmaceutical composition comprising a compound of formula I as claimed in any one of Claims 1 to 8 or a pharmaceutically acceptable carrier.

**Claims for the Contracting State: AT**

1. A process for preparing a compound having the formula

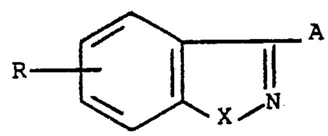

(I)

wherein R is hydrogen, mono- or dihalo, amino, nitro, cyano, hydroxy, trifluoromethyl, mercapto, lower alkyl, lower alkoxy, alkanoyl having 2 to 5 carbon atoms, cycloalkyl of 5 to 7 carbon atoms, carboxy, alkoxycarbonyl having 2 to 7 carbon atoms, mono- or di-lower alkyl substituted amino, alkanoylamino having 2 to 5 carbon atoms, lower alkyl thio, lower alkylsulfonyl, sulfamoyl, lower alkyl substituted sulfamoyl, phenyl or phenyl substituted with halo, lower alkyl, lower alkoxy, trifluoromethyl, hydroxy, amino, cyano or nitro; X is $SO_2$, SO, S or C=O; and A is an amine selected from

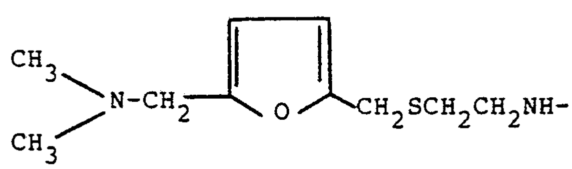

(Ia)

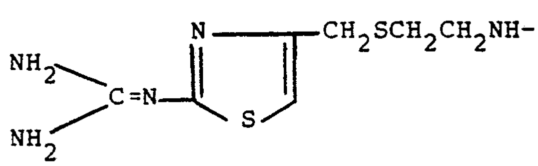

(Ib)

or

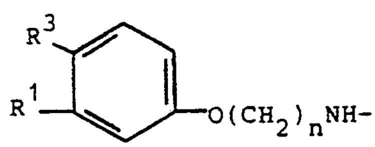

(Ic)

wherein $R^1$ is hydrogen or $R^2CH_2$ wherein $R^2$ is mono- or diloweralkylamino, mono- or di-N-lower alkylaminoloweralkyl, (2-furyl)methylamino, benzylamino, cycloalkylamino having 5 to 7 carbon atoms, 1-pyrrolidinyl, 1-piperidinyl, 1-hexahydroazepinyl, 1-octahydroazocinyl, 3-thiazolidinyl, 4-morpholinyl or 4-thiomorpholinyl; $R^3$ is hydrogen or (1-piperidinyl)methyl; the term "halo" means fluoro, chloro or bromo and the term "lower" used in connection with alkyl or alkoxy means such groups containing 1 to 6 carbon atoms; with the proviso that when $R^3$ is (1-piperidinyl)methyl, $R^1$ is hydrogen; n is 1 to 4 and the pharmacologically acceptable salts thereof, which comprises

a) reacting a compound of formula

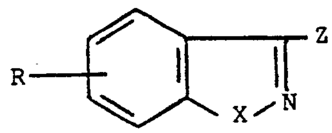

(II)

wherein X and R are as defined above and Z is halogen or $SR^4$ where $R^4$ is alkyl or aralkyl with an amine of formula H—A wherein A is as defined above to give a compound of formula I, or

b) reacting a compound of formula

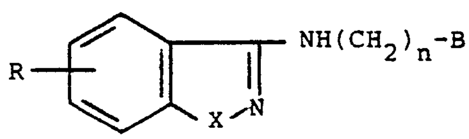

(III)

wherein X, R and n are as defined above and B is OH or L where L represents a leaving group with a compound of formula:

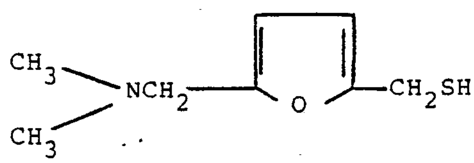

(IVa)

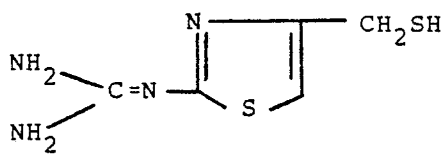

(IVb)

or

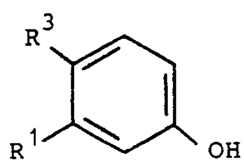

(IVc)

wherein $R^1$ and $R^3$ are as defined above with the provisos that (i) when a compound of formula IVa and IVb is used then n is 2 in the compound of formula III and (ii) when a compound of formula IVc is used then B is L; or

c) reacting a compound of formula

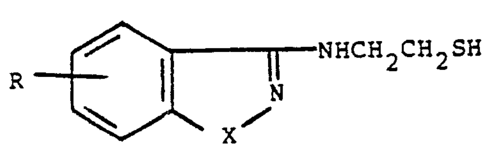

(V)

wherein R and X are as defined above with a compound of formula:

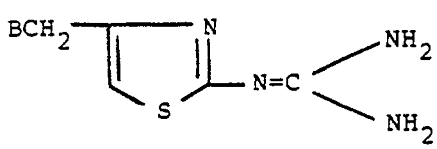

(VIa)

or

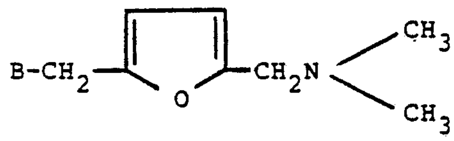

(VIb)

wherein B is OH or L as defined above to give a compound of formula I wherein A has the formula Ia or Ib; or

d) reacting an aldehyde of formula

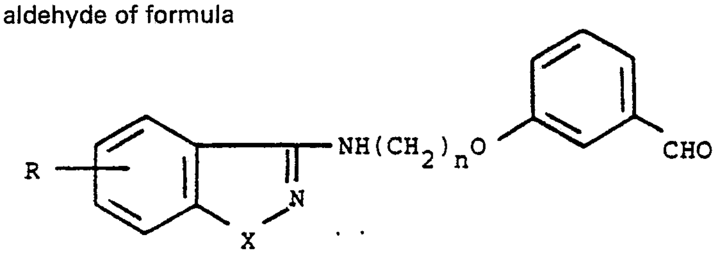

(VIIa)

with an appropriate amine of formula

$$HR^2 \qquad \text{(VIIb)}$$

in which formulae n, R, X and $R^2$ are as defined above, under reductive amination conditions to give a corresponding compound of formula I wherein $R^1$ is $R^2CH_2$— and $R^3$ is hydrogen; or

e) reacting an aldehyde of formula

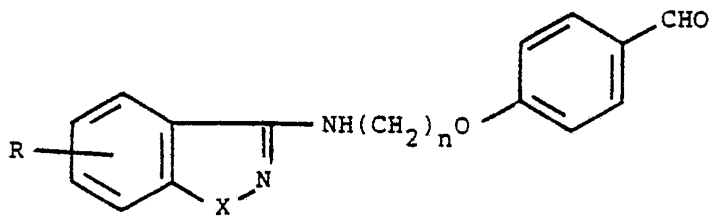

(VIIc)

wherein n, R and X are as defined in Claim 1 with piperidine under reductive amination conditions to give a compound of formula I wherein $R^3$ is (1-piperidinyl)methyl; or

f) reacting an aldehyde of formula

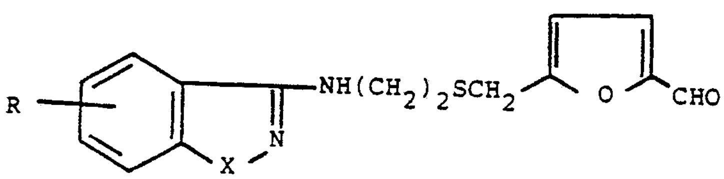

(VIId)

wherein X and R are as defined above with dimethylamine under reductive amination conditions to give a compound of formula I wherein A has formula Ia; or

g) reacting a compound of formula

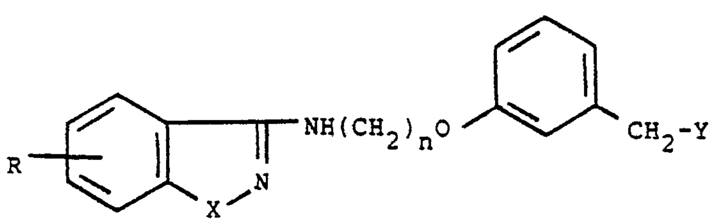

(VIII)

wherein n, X and R are as defined above and Y is OH or L wherein L is a leaving group as hereinbefore described, with an amine of formula $HR^2$ as defined above, the reaction being carried out in the presence of a nickel catalyst when Y is OH, to give a compound of formula I wherein A has the formula Ic and $R^1$ is $R^2CH_2$—; or

h) reacting a compound of formula

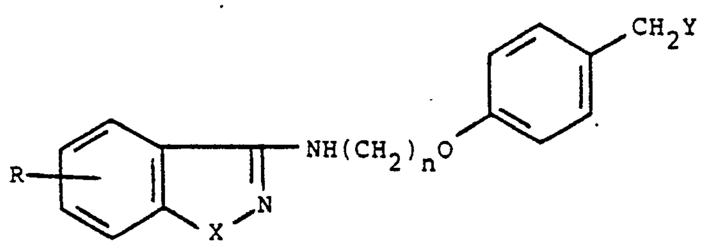

(VIIIa)

wherein Y, n, X and R are as hereinbefore defined with piperidine, the reaction being carried out in the presence of a nickel catalyst when Y is OH, to give a compound of formula I wherein A has the formula Ic and $R^3$ is (1-piperidinyl)methyl; or

i) reacting a compound of formula

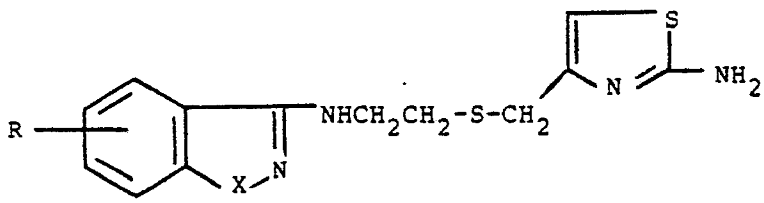

(IX)

wherein X and R are as defined above with a compound capable of converting $—NH_2$ to

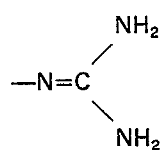

to give a compound of formula I wherein A has formula Ic; or

j) reacting a compound of formula

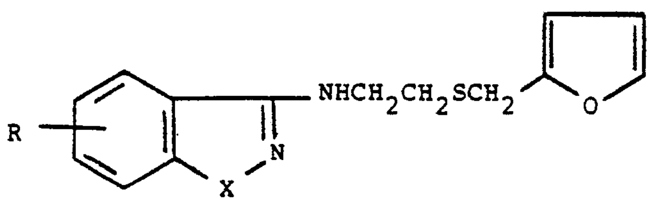

(X)

wherein X and R are as defined above with dimethylamine in the presence of formaldehyde or paraformaldehyde, to give a compound of formula I wherein A has formula Ia; or

k) reacting a compound of formula

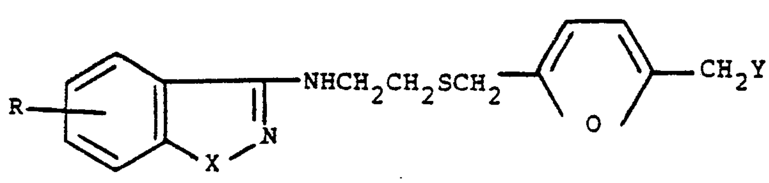

(XI)

wherein Y is OH or a leaving group L as hereinbefore defined, with dimethylamine, the reaction being carried out in the presence of a nickel catalyst when Y is OH; or

l) a basic compound of formula I is converted to a pharmacologically acceptable salt or a pharmacologically acceptable salt is converted to the free base form.

2. A process as claimed in Claim 1 wherein the compound prepared A has formula Ic and $R^3$ is hydrogen.

3. A process as claimed in Claim 1 or Claim 2 wherein the compound prepared A has formula Ic and n is 3.

4. A process as claimed in any one of Claims 1 to 3 wherein the compound prepared A has formula Ic and $R^1$ is $R^2CH_2$ in which $R^2$ is diloweralkylamino, 1-pyrrolindinyl, 1-piperidinyl or 1-hexahydroazepinyl.

5. A process as claimed in any one of Claims 1 to 4 wherein the compound prepared X is $SO_2$.

6. A process as claimed in Claim 1 in which the compound prepared is selected from

N-[2-[[[5-[(dimethylamino)-methyl]-2-furanyl]methyl]thio]ethyl]-1,2-benzisothiazol-3-amine 1,1-dioxide;
N-[3-[3-[(1-piperidinyl)methyl]phenoxy]propyl]-6-nitro-1,2-benzisothiazol-3-amine 1,1-dioxide;
N-[3-[3-[(1-piperidinyl)methyl]phenoxy]propyl]-6-amino-1,2-benzisothiazol-3-amine 1,1-dioxide;
N-[3-[3-[(1-piperidinyl)methyl]phenoxy]propyl]-6-chloro-benzisothiazol-3-amine 1,1-dioxide;
N-[3-[3-[(1-piperidinyl)methyl]phenoxy]propyl]-6-fluoro-1,2-benzisothiazol-3-amine 1,1-dioxide;
N-[3-[3-[(1-piperidinyl)methyl]phenoxy]propyl]-5,6-dichloro-1,2-benzisothiazol-3-amine 1,1-dioxide;
N-[3-[3-[(1-piperidinyl)methyl]phenoxy]propyl]-5-methyl-1,2-benzisothiazol-3-amine 1,1-dioxide;
N-[3-[3-[(1-piperidinyl)methyl]phenoxy]propyl]-5-chloro-1,2-benzisothiazol-3-amine 1,1-dioxide;
N-[3-[3-[(1-piperidinyl)methyl]phenoxy]propyl]-5-methoxy-1,2-benzisothiazol-3-amine 1,1-dioxide;
N-[3-[3-[(1-piperidinyl)methyl]phenoxy]propyl]-6-sulfamoyl-1,2-benzisothiazol-3-amine 1,1-dioxide;
N-[3-[3-[(1-piperidinyl)methyl]phenoxy]propyl]-6-bromo-1,2-benzisothiazol-3-amine 1,1-dioxide;
N-[3-[3-[(1-piperidinyl)methyl]phenoxy]propyl]-5-fluoro-1,2-benzisothiazol-3-amine 1,1-dioxide;
N-[3-[3-(4-morpholinylmethyl)phenoxy]propyl]-1,2-benzisothiazol-3-amine 1,1-dioxide;
N-[3-[3-[(diethylamino)methyl]phenoxy]propyl]-1,2-benzisothiazol-3-amine 1,1-dioxide;
N-[3-[3-[(dimethylamino)methyl]phenoxy]propyl]-1,2-benzisothiazol-3-amine 1,1-dioxide;
N-[3-[3-[(dibutylamino)methyl]phenoxy]propyl]-1,2-benzisothiazol-3-amine 1,1-dioxide;
N-[3-[3-[(4-thiomorpholinyl)methyl]phenoxy]propyl]-1,2-benzisothiazol-3-amine 1,1-dioxide;
N-[3-[3-[(3-thiazolidinyl)methyl]phenoxy]propyl]-1,2-benzisothiazol-3-amine 1,1-dioxide;
N-[3-[3-[(octahydro-1(2H)-azocinyl)methyl]phenoxy]propyl]-1,2-benzisothiazol-3-amine 1,1-dioxide;
N-[3-[3-[(cyclohexylamino)methyl]phenoxy]propyl]-1,2-benzisothiazol-3-amine 1,1-dioxide;
N-[3-[3-[(cyclopentylamino)methyl]phenoxy]propyl]-1,2-benzisothiazol-3-amine 1,1-dioxide;
N-[3-[3-[[[(2-furanyl)methyl]amino]methyl]phenoxy]propyl]-1,2-benzisothiazol-3-amine 1,1-dioxide;
N-[3-[3-[(benzylamino)methyl]phenoxy]propyl]-1,2-benzisothiazol-3-amine 1,1-dioxide;
N-[3-[3-[[bis(2-methylpropyl)amino]methyl]phenoxy]propyl]-1,2-benzisothiazol-3-amine 1,1-dioxide;
N-[3-[3-[(butylamino)methyl]phenoxy]propyl]-1,2-benzisothiazol-3-amine 1,1-dioxide;
N-[3-[3-[(methylpentylamino)methyl]phenoxy]propyl]-1,2-benzisothiazol-3-amine 1,1-dioxide;
N-[3-[3-[(butylpropylamino)methyl]phenoxy]propyl]-1,2-benzisothiazol-3-amine 1,1-dioxide;
N-[3-[3-[(butylmethylamino)methyl]phenoxy]propyl]-1,2-benzisothiazol-3-amine 1,1-dioxide;
N-[3-[3-[(dipentylamino)methyl]phenoxy]propyl]-1,2-benzisothiazol-3-amine 1,1-dioxide;
N-[3-[4-[(1-piperidinyl)methyl]phenoxy]propyl]-1,2-benzisothiazol-3-amine 1,1-dioxide;
or a pharmacologically acceptable salt thereof.

7. A process as claimed in claim 1 in which the compound prepared is from [4-[[[2-[(1,2-benzisothiazol-3-yl)amino]-ethyl] thio]methyl]-2-thiazolyl]guanidine S,S'-dioxide;
3-[[3-[3-(1-piperidinyl)methyl]phenoxy]propyl] amino-1H-isoindol-1-one;
or a pharmacologically acceptable salt thereof.

8. A process as claimed in claim 1 in which the compound prepared is selected from
N-[3-[3-[(1-piperidinyl)methyl]phenoxy]propyl]-1,2-benzisothiazol-3-amine 1,1-dioxide;
N-[3-[3-[(dipropylamino)methyl]phenoxy]propyl]-1,2-benzisothiazol-3-amine 1,1-dioxide;
N-[3-[3-[(1-pyrrolidinylmethyl)phenoxy]propyl]-1,2-benzisothiazol-3-amine 1,1-dioxide;
N-[3-[3-[(hexahydro-1H-azepin-1-yl)methyl]phenoxy]propyl]-1,2-benzisothiazol-3-amine 1,1-dioxide;
or a pharmacologically acceptable salt thereof.

9. A process for preparing a pharmaceutical composition characterised in that a compound of formula I as defined in any one of Claims 1 to 8 or a pharmaceutically acceptable salt thereof is brought into a form suitable for therapeutic administration.

10. A process as claimed in Claim 9 in which the compound of formula I is prepared by a process as claimed in any one of Claims 1 to 8.

**Patentansprüche für die Vertragsstaaten: BE CH DE FR GB IT LI LU NL SE**

1 Ein Verbindung der Formel

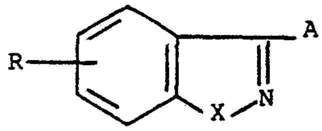

(I)

worin R Wasserstoff, Mono- oder Dihalogen, Amino, Nitro, Cyano, Hydroxy, Trifluormethyl, Mercapto, nied.Alkyl, nied.Alkoxy, Alkanoyl mit 2 bis 5 C-Atomen, Cycloalkyl mit 5 bis 7 C-Atomen, Carboxy, Alkoxycarbonyl mit 2-bis 7 C-Atomen, mono- oder di-nied.alkylsubstituiertes Amino, Alkanoylamino mit 2 bis 5 C-Atomen, nied.Alkylthio, nied.Alkylsulfonyl, Sulfamoyl, nied.alkyl-substituiertes Sulfamoyl, Phenyl oder Phenyl substituiert mit Halogen, nied.Alkyl, nied.Alkoxy, Trifluormethyl, Hydroxy, Amino, Cyano oder Nitro bedeutet; X $SO_2$, SO S oder C=O darstellt und A ein Amin ausgewählt aus

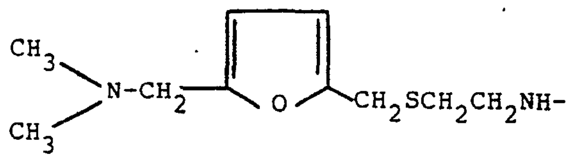

(Ia)

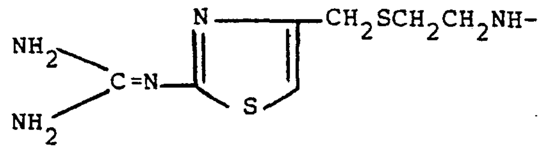

(Ib)

oder

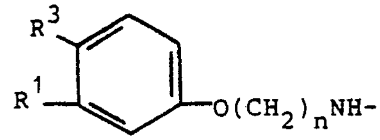

(Ic)

ist; $R^1$ Wasserstoff ist oder die Bedeutung $R^2CH_2$ hat, wobei $R^2$ Mono- oder Di-nied.alkylamino, Mono- oder Di-N-nied.-alkylamino-nied.alkyl, (2-Furyl)-methylamino, Benzylamino, Cycloalkylamino mit 5 bis 7 C-Atomen, 1-Pyrrolidinyl, 1-Piperidinyl, 1-Hexahydroazepinyl, 1-Oktahydrazocinyl, 3-Thiazolidinyl, 4-Morpholinyl oder 4-Thiomorpholinyl darstellt; $R^3$ Wasserstoff oder (1-Piperidinyl)-methyl ist; wobei der Ausdruck "Halogen" Fluor, Chlor oder Brom bezeichnet und der in Verbindung mit Alkyl oder Alkoxy verwendete Ausdruck "nied." bedeutet, daß derartige Gruppen 1 bis 6 C-Atome aufweisen; mit der Maßgabe, daß, wenn $R^3$ (1-Piperidinyl)-methyl ist, $R^1$ Wasserstoff darstellt; n 1 bis 4 ist, und die pharmakologisch annehmbaren Salze hievon.

2. Verbindung der Formel (I), wie in Anspruch 1 beansprucht, worin A die Formel (Ic) aufweist und $R^3$ Wasserstoff ist.

3. Verbindung der Formel (I), wie in Anspruch 1 oder Anspruch 2 beansprucht, worin A die Formel (Ic) aufweist und n 3 ist.

4. Verbindung der Formel (I), wie in einem der Ansprüche 1 bis 3 beansprucht, worin A die Formel (Ic) aufweist und $R^1$ die Bedeutung $R^2CH_2$ besitzt, wobei $R^2$ Di-nied.alkylamino, 1-Pyrrolidinyl, 2-Piperidinyl oder 1-Hexahydrcoazepinyl ist.

5. Verbindung der Formel (I), wie in einem der Ansprüche 1 bis 4 beansprucht, worin X $SO_2$ darstellt.

6. Verbindung der Formel (I), wie in Anspruch 1 definiert, ausgewählt aus

N-[2-[[[5-[(Dimethylamino)-methyl)]-2-furanyl]-methyl]-thio]-äthyl]-1,2-benzisothiazol-3-amin-1,1-dioxid;

N-[3-[3-[(1-Piperidinyl)-methyl)]-phenoxy]-propyl]-6-nitro-1,2-benzisothiazol-3-amin-1,1-dioxid;
N-[3-[3-[(1-Piperidinyl)-methyl)]-phenoxy]-propyl]-6-amino-1,2-benzisothiazol-3-amin-1,1-dioxid;
N-[3-[3-[(1-Piperidinyl)-methyl)]-phenoxy]-propyl]-6-chlor-1,2-benzisothiazol-3-amin-1,1-dioxid;
N-[3-[3-[(1-Piperidinyl)-methyl)]-phenoxy]-propyl]-6-fluor-1,2-benzisothiazol-3-amin-1,1-dioxid;
N-[3-[3-[(1-Piperidinyl)-methyl)]-phenoxy]-propyl]-5,6-dichlor-1,2-benzisothiazol-3-amin-1,1-dioxid;
N-[3-[3-[(1-Piperidinyl)-methyl)]-phenoxy]-propyl]-5-methyl-1,2-benzisothiazol-3-amin-1,1-dioxid;
N-[3-[3-[(1-Piperidinyl)-methyl)]-phenoxy]-propyl]-5-chlor-1,2-benzisothiazol-3-amin-1,1-dioxid;
N-[3-[3-[(1-Piperidinyl)-methyl)]-phenoxy]-propyl]-5-methoxy-1,2-benzisothiazol-3-amin-1,1-dioxid;
N-[3-[3-[(1-Piperidinyl)-methyl)]-phenoxy]-propyl]-6-sulfamoyl-1,2-benzisothiazol-3-amin-1,1-dioxid;
N-[3-[3-[(1-Piperidinyl)-methyl)]-phenoxy]-propyl]-6-brom-1,2-benzisothiazol-3-amin-1,1-dioxid;
N-[3-[3-[(1-Piperidinyl)-methyl)]-phenoxy]-propyl]-5-fluor-1,2-benzisothiazol-3-amin-1,1-dioxid;
N-[3-[3-[(4-Morpholinylmethyl)-phenoxy]-propyl]-1,2-benzisothiazol-3-amin-1,1-dioxid;
N-[3-[3-[(Diäthylamino)-methyl]-phenoxy]-propyl]-1,2-benzisothiazol-3-amin-1,1-dioxid;
N-[3-[3-[(Dimethylamino)-methyl]-phenoxy]-propyl]-1,2-benzisothiazol-3-amin-1,1-dioxid;
N-[3-[3-[(Dibutylamino)-methyl]-phenoxy]-propyl]-1,2-benzisothiazol-3-amin-1,1-dioxid;
N-[3-[3-[(4-Thiomorpholinyl)-methyl]-phenoxy]-propyl]-1,2-benzisothiazol-3-amin-1,1-dioxid;
N-[3-[3-[(3-Thiazolidinyl)-methyl]-phenoxy]-propyl]-1,2-benzisothiazol-3-amin-1,1-dioxid;
N-[3-[3-[(Octahydro-1(2H)-azocinyl)-methyl]-phenoxy]-propyl]-1,2-benzisothiazol-3-amin-1,1-dioxid;
N-[3-[3-[(Cyclohexylamino)-methyl]-phenoxy]-propyl]-1,2-benzisothiazol-3-amin-1,1-dioxid;
N-[3-[3-[(Cyclopentylamino)-methyl]-phenoxy]-propyl]-1,2-benzisothiazol-3-amin-1,1-dioxid;
N-[3-[3-[[[(2-Furanyl)-methyl]-amino]-methyl]-phenoxy]-propyl]-1,2-benzisothiazol-3-amin-1,1-dioxid;
N-[3-[3-[(Benzylamino)-methyl]-phenoxy]-propyl]-1,2-benzisothiazol-3-amin-1,1-dioxid;
N-[3-[3-[(Bis-(2-methylpropyl)-amino]-methyl]-phenoxy]-propyl]-1,2-benzisothiazol-3-amin-1,1-dioxid;
N-[3-[3-[(Butylamino)-methyl]-phenoxy]-propyl]-1,2-benzisothiazol-3-amin-1,1-dioxid;
N-[3-[3-[(Methylpentylamino)-methyl]-phenoxy]-propyl]-1,2-benzisothiazol-3-amin-1,1-dioxid;
N-[3-[3-[(Butylpropylamino)-methyl]-phenoxy]-propyl]-1,2-benzisothiazol-3-amin-1,1-dioxid;
N-[3-[3-[(Butylmethylamino)-methyl]-phenoxy]-propyl]-1,2-benzisothiazol-3-amin-1,1-dioxid;

N-[3-[3-[(Dipentylamino)-methyl]-phenoxy]-propyl]-1,2-benzisothiazol-3-amin-1,1-dioxid;
N-[3-[4-[(1-Piperidinyl)-methyl]-phenoxy]-propyl]-1,2-benzisothiazol-3-amin-1,1-dioxid;
oder ein pharmakologisch annehmbares Salz hievon.

7. Verbindung der Formel (I), wie in Anspruch 1 definiert, ausgewählt aus [4-[[[2-[(1,2-Benzisothiazol-3-yl)-amino]-äthyl]-thio]-methyl]-2-thiazolyl]-guanidin-S,S'-dioxid;
3-[[3-[3-[(1-Piperidinyl)-methyl]-phenoxy]-propyl]-amino]]-1H-isoindol-1-on;
oder ein pharmakologisch annehmbares Salz hievon.

8. Verbindung der Formel (I), wie in Ansprüche 1 definiert, ausgewählt aus N-[3-[3-[(1-Piperidinyl)-methyl]-phenoxy]-propyl]-1,2-benzisothiazol-3-amin-1,1-dioxid;
N-[3-[3-[(Dipropylamino)-methyl]-phenoxy]-propyl]-1,2-benzisothiazol-3-amin-1,1-dioxid;
N-[3-[3-[(1-Pyrrolidinylmethyl)-phenoxy]-propyl]-1,2-benzisothiazol-3-amin-1,1-dioxid;
N-[3-[3-[(Hexahydro-1H-azepin-1-yl)-methyl]-phenoxy]-propyl]-1,2-benzisothiazol-3-amin-1,1-dioxid;
oder ein pharmakologisch annehmbares Salz hievon.

9. Verfahren zur Herstellung einer Verbindung der Formel (I), wie in Anspruch 1 definiert, oder eines pharmakologisch annehmbaren Salzes hievon, welches umfaßt:

a) das Umsetzen einer Verbindung der Formel

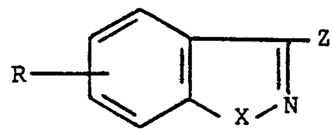

(II)

worin X und R wie in Anspruch 1 definiert sind und Z Halogen oder $SR^4$ ist, wobei $R^4$ Alkyl oder Aralkyl bedeutet, mit einem Amin der Formel H—A, worin A wie in Anspruch 1 definiert ist, zu einer Verbindung der Formel (I); oder

b) das Umsetzen einer Verbindung der Formel

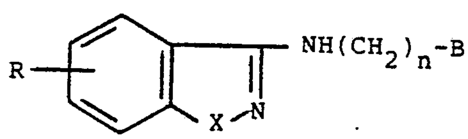

(III)

worin X, R und n wie in Anspruch 1 definiert sind und B OH oder L bedeutet, wobei L eine abspaltbare Gruppe darstellt, mit einer Verbindung der Formel

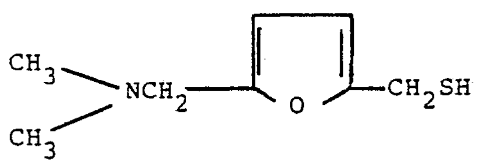

(IVa)

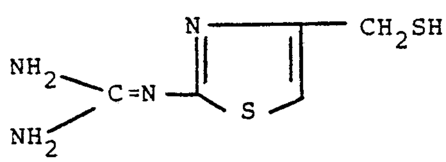

(IVb)

or

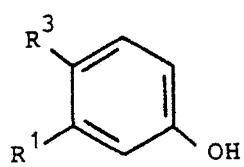

(IVc)

worin $R^1$ und $R^3$ wie in Anspruch 1 definiert sind, mit den Maßgaben, daß, (i) wenn eine Verbindung der Formel (IVa) oder (IVb) verwendet wird, n in der Verbindung der Formel (III) 2 ist, und, (ii) wenn eine Verbindung der Formel (IVc) verwendet wird, B L ist; oder

c) das Umsetzen einer Verbindung der Formel

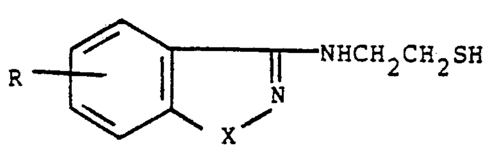

(V)

worin R und X wie in Anspruch 1 definiert sind, mit einer Verbindung der Formel

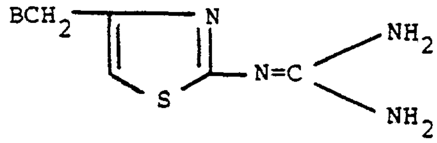

(VIa)

oder

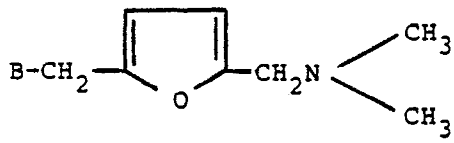

(VIb)

worin B OH oder L, wie oben definiert, ist, zu einer Verbindung der Formel (I), worin A die Formel (Ia) oder (Ib) aufweist; oder

d) das Umsetzen einer Aldehyds der Formel

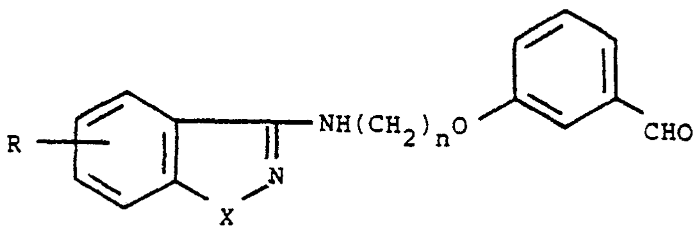

(VIIa)

mit einem geeigneten Amin der Formel

$HR^2$ (VIIb),

in welchen Formeln n, R, X und $R^2$ wie in Anspruch 1 definiert sind, unter reduktiven Aminierungsverbindungen zu einer entsprechenden Verbindung der Formel (I), worin $R^1$ $R^2CH_2$— bedeutet und $R^3$ Wasserstoff ist; oder

e) das Umsetzen einer Aldehyds der Formel

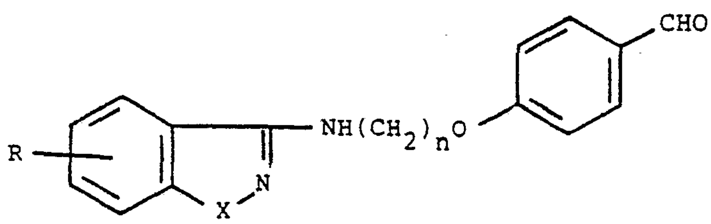

(VIIc)

worin n, R und X wie in Anspruch 1 definiert sind, mit Piperidin unter reduktiven Aminierungsbedingungen zu einer Verbindung der Formel (I), worin $R^3$ (1-Piperidinyl)-methyl bedeutet; oder

f) das Umsetzen eines Aldehyds der Formel

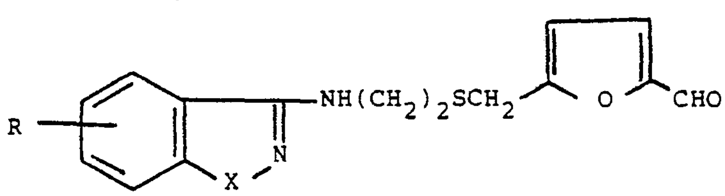

(VIId)

worin X und R wie in Anspruch 1 definiert sind, mit Dimethylamin unter reduktiven Aminierungsbedingungen zu einer Verbindung der Formel (I), worin A die Formel (Ia) aufweist; oder

g) das Umsetzen einer Verbindung der Formel

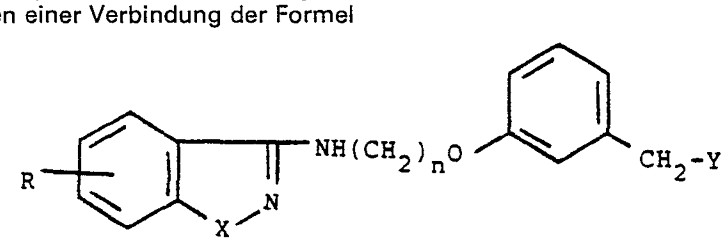

(VIII)

worin n, X und R wie in Anspruch 1 definiert sind, Y OH oder L bedeutet, wobei L eine abspaltbare Gruppe ist, wie oben beschrieben, mit einem Amin der Formel $HR^2$, wie oben definiert, wobei die Reaktion in Anwesenheit eines Nickelkatalysators durchgeführt wird, wenn Y OH ist, zu einer Verbindung der Formel (I), worin A die Formel (Ic) aufweist und $R^1$ die Bedeutung $R^2CH_2$— hat; oder

h) das Umsetzen einer Verbindung der Formel

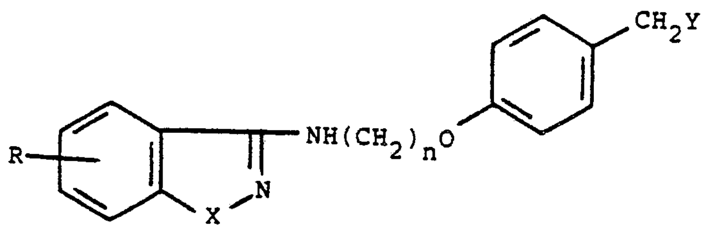

(VIIIa)

worin Y, n, X und R wie oben definiert sind, mit Piperidin, wobei die Reaktion in Anwesenheit eines Nickelkatalysators durchgeführt wird, wenn Y OH ist, zu einer Verbindung der Formel (I), worin A die Formel (Ic) aufweist und $R^3$ (1-Piperidinyl)methyl ist; oder

i) das Umsetzen einer Verbindung der Formel

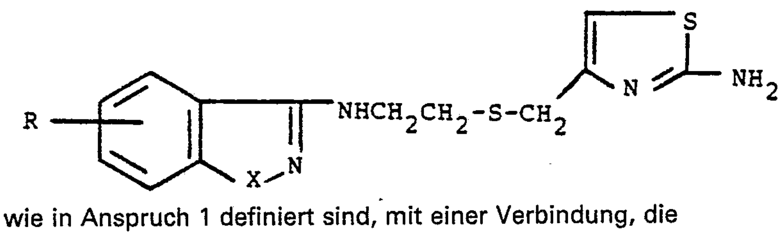

(IX)

worin X und R wie in Anspruch 1 definiert sind, mit einer Verbindung, die

$—NH_2$ in 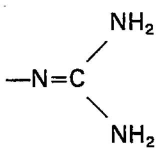

überzuführen imstande ist, zu einer Verbindung der Formel (I), worin A die Formel (Ic) aufweist; oder

j) das Umsetzen einer Verbindung der Formel

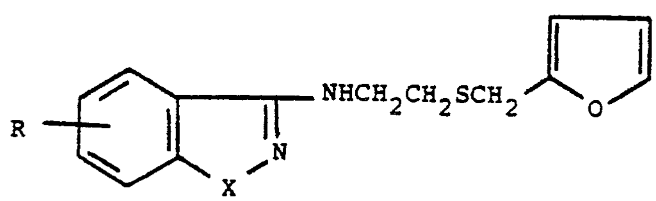

(X)

worin X und R wie in Anspruch 1 definiert sind, mit Dimethylamin in Anwesenheit von Formaldehyd oder p-Formaldehyd zu einer Verbindung der Formel (I), worin A die Formel (Ia) aufweist; oder

k) das Umsetzen einer Verbindung der Formel

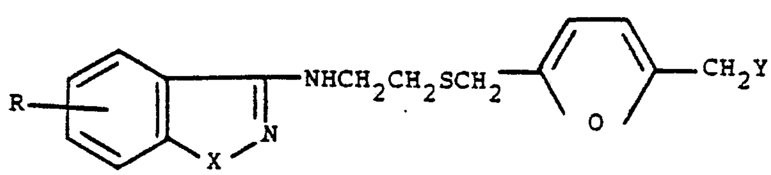

(XI)

worin Y OH oder eine abspaltbare Gruppe L, wie oben definiert, ist, mit Dimethylamin, wobei die Reaktion in Anwesenheit eines Nickelkatalysators durchgeführt wird, wenn Y OH ist; oder

l) das Überführen einer basischen Verbindung der Formel (I) in ein pharmakologisch annehmbares Salz oder eines pharmakologisch annehmbaren Salzes in die Form de freien Base.

10. Verbindung der Formel (I) oder ein pharmakologisch annehmbares Salz hievon, wie in einem der Ansprüche 1 bis 8 beansprucht, zur Verwendung als ein Inhibitor von Magensäuresekretion.

11. Pharmazeutische Zusammensetzung, die eine Verbindung der Formel (I), wie in einem der Ansprüche 1 bis 8 beansprucht, oder ein pharmazeutisch annehmbares Salz hievon und einen pharmazeutisch annehmbaren Träger umfaßt.

**Patentansprüche für den Vertragsstaat AT:**

1. Verfahren zur Herstellung einer Verbindung der Formel

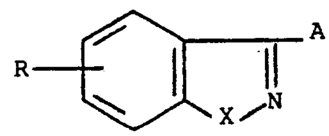

(I)

worin R Wasserstoff, Mono- oder Dihalogen, Amino, Nitro, Cyano, Hydroxy, Trifluormethyl, Mercapto, nied.Alkyl, nied.Alkoxy, Alkanoyl mit 2 bis 5 C-Atomen, Cycloalkyl mit 5 bis 7 C-Atomen, Carboxy, Alkoxycarbonyl mit 2 bis 7 C-Atomen, mono- oder di-nied.alkylsubstituiertes Amino, Alkanoylamino mit 2 bis 5 C-Atomen, nied.Alkylthio, nied.Alkylsulfonyl, Sulfamoyl, nied.alkyl-substituiertes Sulfamoyl, Phenyl oder Phenyl substituiert mit Halogen, nied.Alkyl, nied.Alkoxy, Trifluormethyl, Hydroxy, Amino, Cyano oder Nitro bedeutet; X $SO_2$, SO, S oder C=O darstellt und A ein Amin ausgewählt aus

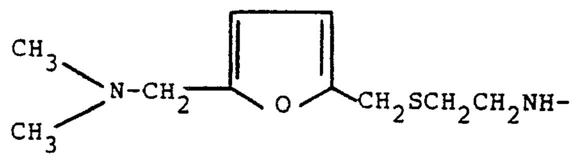

(Ia)

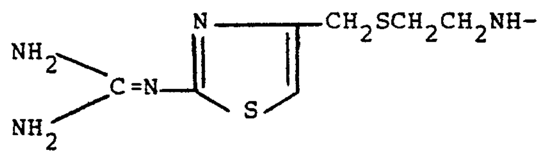

(Ib)

oder

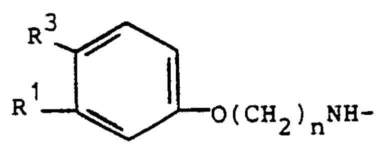

(Ic)

ist; $R^1$ Wasserstoff ist oder die Bedeutung $R^2CH_2$ hat, wobei $R^2$ Mono- oder Di-nied.alkylamino, Mono- oder Di-N-nied.-alkylamino-nied.alkyl, (2-Furyl)-methylamino, Benzylamino, Cycloalkylamino mit 5 bis 7 C-Atomen, 1-Pyrrolidinyl, 1-Piperidinyl, 1-Hexahydroazepinyl, 1-Oktahydrazocinyl, 3-Thiazolidinyl, 4-Morpholinyl oder 4-Thiomorpholinyl darstellt; $R^3$ Wasserstoff oder (1-Piperidinyl)-methyl ist; wobei der Ausdruck "Halogen" Fluor, Chlor oder Brom bezeichnet und der in Verbindung mit Alkyl oder Alkoxy verwendete Ausdruck "nied." bedeutet, daß derartige Gruppen 1 bis 6 C-Atome aufweisen; mit der Maßgabe, daß, wenn $R^3$ (1-Piperidinyl)-methyl ist, $R^1$ Wasserstoff darstellt; n 1 bis 4 ist, und der pharmakologisch annehmbaren Salze hievon, welches umfaßt:

a) das Umsetzen einer Verbindung der Formel

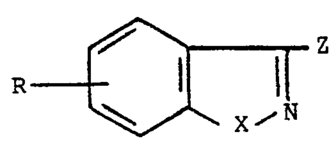

(II)

worin X und R wie oben definiert sind und Z Halogen oder $SR^4$ ist, wobei $R^4$ Alkyl oder Aralkyl bedeutet, mit einem Amin der Formel H—A, worin A wie oben definiert ist, zu einer Verbindung der Formel (I); oder

b) das Umsetzen einer Verbindung der Formel

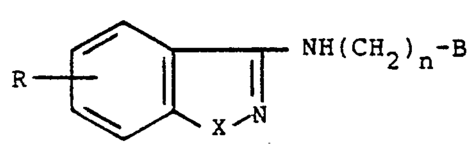

(III)

worin X, R und n wie oben definiert sind und B OH oder L bedeutet, wobei L eine abspaltbare Gruppe darstellt, mit einer Verbindung der Formel

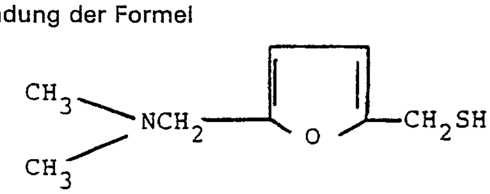

(IVa)

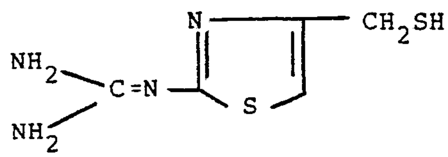 (IVb)

oder

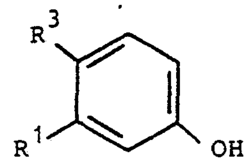 (IVc)

worin $R^1$ und $R^3$ wie oben definiert sind, mit den Maßgaben, daß, (i) wenn eine Verbindung der Formel (IVa) oder (IVb) verwendet wird, n in der Verbindung der Formel (III) 2 ist, und (ii) wenn eine Verbindung der Formel (IVc) verwendet wird, B L ist; oder

c) das Umsetzen einer Verbindung der Formel

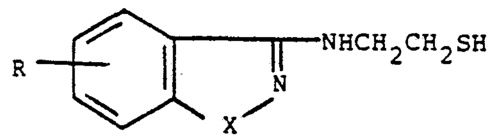 (V)

worin R und X wie oben definiert sind, mit einer Verbindung der Formel

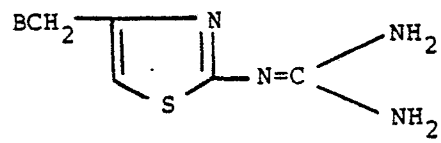 (VIa)

oder

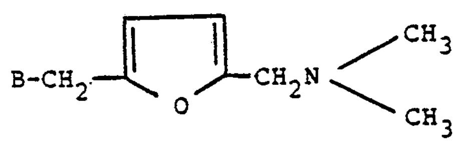 (VIb)

worin B OH oder L, wie oben definiert, ist, zu einer Verbindung der Formel (I), worin A die Formel (Ia) oder (Ib) aufweist; oder

d) das Umsetzen eines Aldehyds der Formel

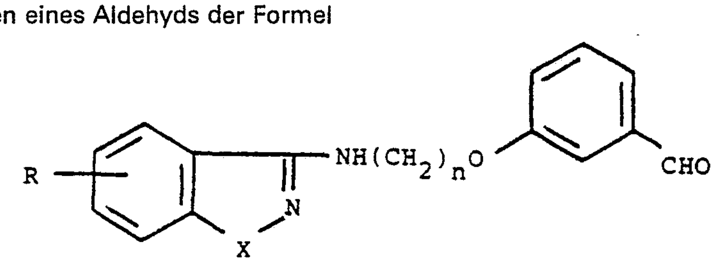 (VIIa)

mit einem geeigneten Amin der Formel

$HR^2$ (VIIb),

in welchen Formeln n, R, X und $R^2$ wie oben definiert sind, unter reduktiven Aminierungsverbindungen zu einer entsprechenden Verbindung der Formel (I), worin $R^1$ $R^2CH_2$— bedeutet und $R^3$ Wasserstoff ist; oder

e) das Umsetzen einer Aldehyds der Formel

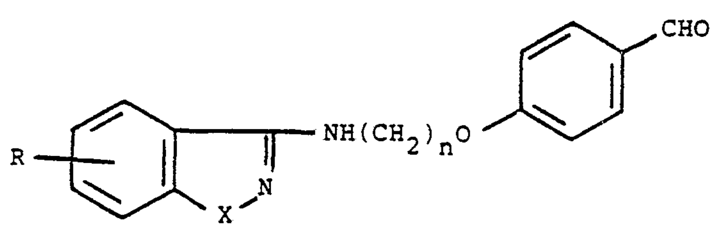 (VIIc)

worin n, R und X wie in Anspruch 1 definiert sind, mit Piperidin unter reduktiven Aminierungsbedingungen zu einer Verbindung der Formel (I), worin $R^3$ (1-Piperidinyl)-methyl bedeutet; oder

f) das Umsetzen eines Aldehyds der Formel

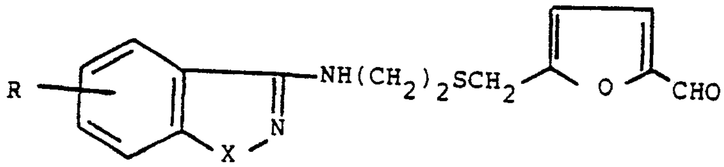

(VIId)

worin X und R X wie oben definiert sind, mit Dimethylamin unter reduktiven Aminiergunsbedingungen zu einer Verbindung der Formel (I), worin A die Formel (Ia) aufweist; oder

g) das Umsetzen einer Verbindung der Formel

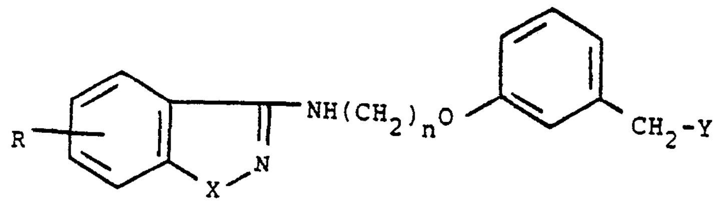

(VIII)

worin n, X und R wie oben definiert sind und Y OH oder L bedeutet, wobei L eine abspaltbare Gruppe ist, wie oben beschrieben, mit einem Amin der Formel $HR^2$, wie oben definiert, wobei die Reaktion in Anwesenheit eines Nickelkatalysators durchgeführt wird, wenn Y OH ist, zu einer Verbindung der Formel (I), worin A die Formel (Ic) aufweist und $R^1$ die Bedeutung $R^2CH_2$— hat; oder

h) das Umsetzen einer Verbindung der Formel

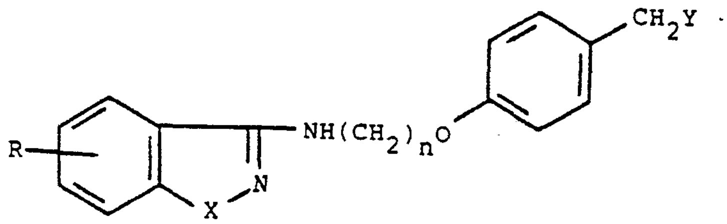

(VIIIa)

worin Y, n, X und R wie oben definiert sind, mit Piperidin, wobei die Reaktion in Anwesenheit eines Nickelkatalysators durchgeführt wird, wenn Y OH ist, zu einer Verbindung der Formel (I), worin A die Formel (Ic) aufweist und $R^3$ (1-Piperdinyl)methyl ist; oder

i) das Umsetzen einer Verbindung der Formel

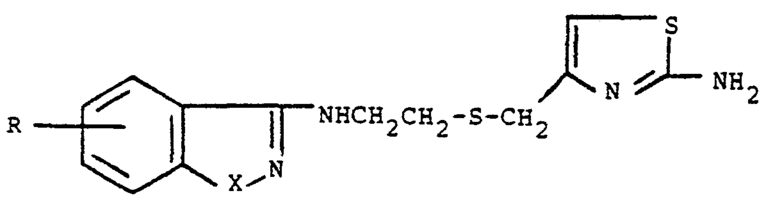

(IX)

worin X und R wie oben definiert sind, mit einer Verbindung, die

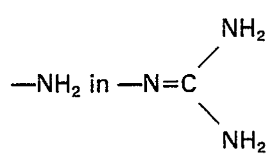

überzuführen imstande ist, zu einer Verbindung der Formel (I), worin A die Formel (Ic) aufweist; oder

j) das Umsetzen einer Verbindung der Formel

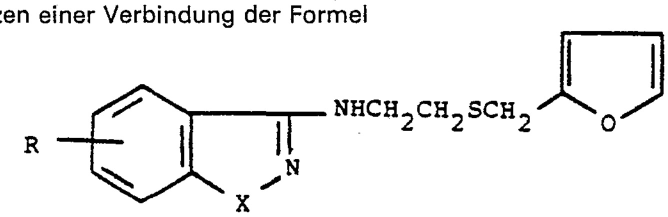

(X)

worin X und R wie oben definiert sind, mit Dimethylamin in Anwesenheit von Formaldehyd oder p-Formaldehyd zu einer Verbindung der Formel (I), worin A die Formel (Ia) aufweist; oder

k) das Umsetzen einer Verbindung der Formel

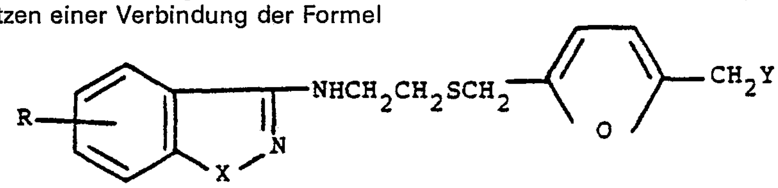

(XI)

worin Y OH oder eine abspaltbare Gruppe, wie oben definiert, ist, mit Dimethylamin, wobei die Reaktion in Anwesenheit eines Nickelkatalysators durchgeführt wird, wenn Y OH ist; oder

l) das Überführen einer basischen Verbindung der Formel (I) in ein pharmakologisch annehmbares Salz oder eines pharmakologisch annehmbaren Salzes in die Form de freien Base.

2. Verfahren, wie in Anspruch 1 beansprucht, worin in der Hergestellten Verbindung A die Formel (Ic) aufweist und $R^3$ Wasserstoff ist.

3. Verfahren, wie in Anspruch 1 oder Anspruch 2 beansprucht, worin in der hergestellten Verbindung A die Formel (Ic) aufweist und n 3 ist.

4. Verfahren, wie in einem der Ansprüche 1 bis 3 beansprucht, worin in der hergestellten Verbindung A die Formel (Ic) aufweist und $R^1$ die Bedeutung $R^2CH_2$ aufweist, wobei $R^2$ Di-nied.alkylamino, 1-Pyrrolidinyl, 1-Piperidinyl oder 1-Hexahydroazepinyl ist.

5. Verfahren, wie in einem der Ansprüche 1 bis 4 beansprucht, worin in der hergestellten Verbindung X die Bedeutung $SO_2$ hat.

6. Verfahren, wie in Anspruch 1 beansprucht, worin die hergestellte Verbindung ausgewählt ist aus
N-[2-[[[5-[(Dimethylamino)-methyl)]-2-furanyl]-methyl]-thio]-äthyl]-1,2-benzisothiazol-3-amin-1,1-dioxid;
N-[3-[3-[(1-Piperidinyl)-methyl)]-phenoxy]-propyl]-6-nitro-1,2-benzisothiazol-3-amin-1,1-dioxid;
N-[3-[3-[(1-Piperidinyl)-methyl)]-phenoxy]-propyl]-6-amino-1,2-benzisothiazol-3-amin-1,1-dioxid;
N-[3-[3-[(1-Piperidinyl)-methyl)]-phenoxy]-propyl]-6-chlor-1,2-benzisothiazol-3-amin-1,1-dioxid;
N-[3-[3-[(1-Piperidinyl)-methyl)]-phenoxy]-propyl]-6-fluor-1,2-benzisothiazol-3-amin-1,1-dioxid;
N-[3-[3-[(1-Piperidinyl)-methyl)]-phenoxy]-propyl]-5,6-dichlor-1,2-benzisothiazol-3-amin-1,1-dioxid;
N-[3-[3-[(1-Piperidinyl)-methyl)]-phenoxy]-propyl]-5-methyl-1,2-benzisothiazol-3-amin-1,1-dioxid;
N-[3-[3-[(1-Piperidinyl)-methyl)]-phenoxy]-propyl]-5-chlor-1,2-benzisothiazol-3-amin-1,1-dioxid;
N-[3-[3-[(1-Piperidinyl)-methyl)]-phenoxy]-propyl]-5-methoxy-1,2-benzisothiazol-3-amin-1,1-dioxid;
N-[3-[3-[(1-Piperidinyl)-methyl)]-phenoxy]-propyl]-6-sulfamoyl-1,2-benzisothiazol-3-amin-1,1-dioxid;
N-[3-[3-[(1-Piperidinyl)-methyl)]-phenoxy]-propyl]-6-brom-1,2-benzisothiazol-3-amin-1,1-dioxid;
N-[3-[3-[(1-Piperidinyl)-methyl)]-phenoxy]-propyl]-5-fluor-1,2-benzisothiazol-3-amin-1,1-dioxid;
N-[3-[3-[(4-Morpholinylmethyl)-phenoxy]-propyl]-1,2-benzisothiazol-3-amin-1,1-dioxid;
N-[3-[3-[(Diäthylamino)-methyl]-phenoxy]-propyl]-1,2-benzisothiazol-3-amin-1,1-dioxid;
N-[3-[3-[(Dimethylamino)-methyl]-phenoxy]-propyl]-1,2-benzisothiazol-3-amin-1,1-dioxid;
N-[3-[3-[(Dibutylamino)-methyl]-phenoxy]-propyl]-1,2-benzisothiazol-3-amin-1,1-dioxid;
N-[3-[3-[(4-Thiomorpholinyl)-methyl]-phenoxy]-propyl]-1,2-benzisothiazol-3-amin-1,1-dioxid;
N-[3-[3-[(3-Thiazolidinyl)-methyl]-phenoxy]-propyl]-1,2-benzisothiazol-3-amin-1,1-dioxid;
N-[3-[3-[(Octahydro-1(2H)-azocinyl)-methyl]-phenoxy]-propyl]-1,2-benzisothiazol-3-amin-1,1-dioxid;
N-[3-[3-[(Cyclohexylamino)-methyl]-phenoxy]-propyl]-1,2-benzisothiazol-3-amin-1,1-dioxid;
N-[3-[3-[(Cyclopentylamino)-methyl]-phenoxy]-propyl]-1,2-benzisothiazol-3-amin-1,1-dioxid;
N-[3-[3-[[[(2-Furanyl)-methyl]-amino]-methyl]-phenoxy]-propyl]-1,2-benzisothiazol-3-amin-1,1-dioxid;
N-[3-[3-[(Benzylamino)-methyl]-phenoxy]-propyl]-1,2-benzisothiazol-3-amin-1,1-dioxid;
N-[3-[3-[(Bis-(2-methylpropyl)-amino]-methyl]-phenoxy]-propyl]-1,2-benzisothiazol-3-amin-1,1-dioxid;
N-[3-[3-[(Butylamino)-methyl]-phenoxy]-propyl]-1,2-benzisothiazol-3-amin-1,1-dioxid;
N-[3-[3-[(Methylpentylamino)-methyl]-phenoxy]-propyl]-1,2-benzisothiazol-3-amin-1,1-dioxid;
N-[3-[3-[(Butylpropylamino)-methyl]-phenoxy]-propyl]-1,2-benzisothiazol-3-amin-1,1-dioxid;
N-[3-[3-[(Butylmethylamino)-methyl]-phenoxy]-propyl]-1,2-benzisothiazol-3-amin-1,1-dioxid;
N-[3-[3-[(Dipentylamino)-methyl]-phenoxy]-propyl]-1,2-benzisothiazol-3-amin-1,1-dioxid;
N-[3-[4-[(1-Piperidinyl)-methyl]-phenoxy]-propyl]-1,2-benzisothiazol-3-amin-1,1-dioxid;
oder einem pharmakologisch annehmbaren Salz hievon.

7. Verfahren, wie in Anspruch 1 beansprucht, worin die hergestellte Verbindung ausgewählt ist aus
4-[[[2-[(1,2-Benzisothiazol-3-yl)-amino]-äthyl]-thio]-methyl]-2-thiazolyl]-guanidin-S,S'-dioxid;
3-[[3-[3-[(1-Piperidinyl)-methyl]-phenoxy]-propyl]-amino]]-1H-isoindol-1-on;
oder einem pharmakologisch annehmbaren Salz hievon.

8. Verfahren, wie in Anspruch 1 beansprucht, worin die hergestellte Verbindung ausgewählt ist aus
N-[3-[3-[(1-Piperidinyl)-methyl]-phenoxy]-propyl]-1,2-benzisothiazol-3-amin-1,1-dioxid;
N-[3-[3-[(Dipropylamino)-methyl]-phenoxy]-propyl]-1,2-benzisothiazol-3-amin-1,1-dioxid;
N-[3-[3-[(1-Pyrrolidinylmethyl)-phenoxy]-propyl]-1,2-benzisothiazol-3-amin-1,1-dioxid;
N-[3-[3-[(Hexahydro-1H-azepin-1-yl)-methyl]-phenoxy]-propyl]-1,2-benzisothiazol-3-amin-1,1-dioxid;
oder einem pharmakologisch annehmbaren Salz hievon.

9. Verfahren zur Herstellung einer pharmazeutischen Zusammensetzung, dadurch gekennzeichnet, daß eine Verbindung der Formel (I), wie in einem der Ansprüche 1 bis 8 definiert, oder ein pharmazeutisch annehmbares Salz hievon in eine für therapeutische Verabreichung geeignete Form gebracht wird.

10. Verfahren, wie in Anspruch 9 beansprucht, in dem die Verbindung der Formel (I) hergestellt ist nach einem Verfahren, wie in einem der Ansprüche 1 bis 8 beansprucht.

**Revendications pour les Etats contractants: BE CH DE FR GB IT LI LU NL SE**

1. Composant répondant à la formule:

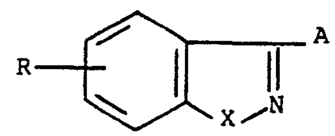

(I)

dans laquelle R est l'hydrogène, un radical mono- ou dihalogéno, amino, nitro, cyano, hydroxy, trifluorométhyle, mercapto, alkyle inférieur, alkoxy inférieur, alcanoyle ayant 2 à 5 atomes de carbone, cycloalkyle ayant 5 à 7 atomes de carbone, carboxy, alkoxycarbonyle ayant 2 à 7 atomes de carbone, amino portant 1 ou 2 substituants alkyle inférieur, alcanoylamino ayant 2 à 5 atomes de carbone, alkylthio, inférieur, alkylsulfonyle inférieur, sulfamoyle, sulfamoyle à substituant alkyle inférieur, phényle ou phényle à substituant halogéno, alkyle inférieur, alkoxy inférieur, trifluorométhyle, hydroxy, amino, cyano ou nitro; X représente $SO_2$, SO, S ou C=O; et A est une amine de formule

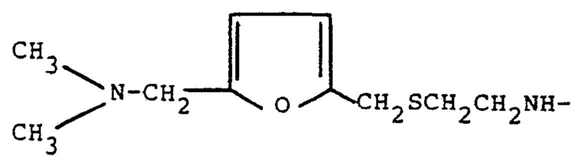

(Ia)

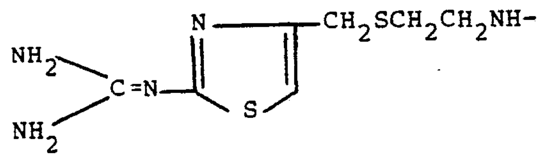

(Ib)

ou

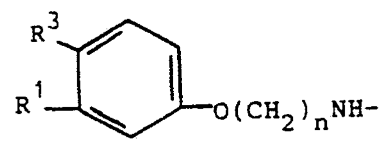

(Ic)

dans laquelle $R^1$ est l'hydrogène ou un groupe $R^2CH_2$ dans lequel $R^2$ est un radical mono- ou di(alkyle inférieur)amino, mono- ou di-N(alkyle inférieur)aminoalkyle inférieur, (2-furyl)méthylamino, benzylamino, cycloalkylamino ayant 5 à 7 atomes de carbone, 1-pyrrolidinyle, 1-pipéridinyle, 1-hexahydroazépinyle, 1-octahydroazocinyle, 3-thiazolidinyle, 4-morpholinyle ou 4-thiomorpholinyle; $R^3$ est l'hydrogène ou le groupe (1-pipéridinyl)méthyle; le terme "halogéno" désigne un radical fluoro, chloro ou bromo et le terme "inférieur" utilisé pour qualifier un groupe alkyle ou alkoxy signifie que ce groupe contient 1 à 6 atomes de carbone; sous réserve que, lorsque $R^3$ est le groupe (1-pipéridinyl)méthyle, $R^1$ soit un atome d'hydrogène; n a une valeur de 1 à 4, et ses sels pharmacologiquement acceptables.

2. Composé de formule I suivant la revendication 1 dans lequel A répond à la formule Ic et $R^3$ est l'hydrogène.

3. Composé de formule I suivant la revendication 1 ou la revendication 2, dans lequel A répond à la formule Ic et n est égal à 3.

4. Composé de formule I suivant l'une quelconque des revendications 1 à 3, dans lequel A répond à la formule Ic et $R^1$ est un groupe de formule $R^2CH_2$ dans laquelle $R^2$ est un radical di(alkyle inférieur)amino, 1-pyrrolidinyle, 2-pipéridinyle ou 1-hexahydro-azépinyle.

5. Composé de formule I suivant l'une quelconque des revendications 1 à 4, dans lequel X est le groupe $SO_2$.

6. Composé de formule I suivant la revendication 1, choisi entre les composés suivants:

1,1-dioxyde de *N*-[2-[[[5-[(diméthylamino)méthyl]-2-furannyl]méthyl]thio]éthyl]-1,2-benzisothiazole-3-amine;

1,1-dioxyde de *N*-[3-[3-[(1-pipéridinyle)méthyl)]phénoxyl]propyl]-6-nitro-1,2-benzisothiazole-3-amine;

1,1-dioxyde de *N*-[3-[3-[(1-pipéridinyl)méthyl]phénoxy]propyl]-6-amino-1,2-benzisothiazole-3-amine;
1,1-dioxyde de *N*-[3-[3-[(1-pipéridinyl)méthyl]phénoxy]propyl]-6-chloro-1,2-benzisothiazole-3-amine;
1,1-dioxyde de *N*-[3-[3-[(1-pipéridinyl)méthyl]phénoxy]propyl]-6-fluoro-1,2-benzisothiazole-3-amine;
1,1-dioxyde de *N*-[3-[3-[(1-pipéridinyl)méthyl]phénoxy]propyl]-6-fluoro-1,2-benzisothiazole-3-amine;
1,1-dioxyde de *N*-[3-[3-[(1-pipéridinyl)méthyl]phénoxy]propyl]-5,6-dichloro-1,2-benzisothiazole-3-amine;
1,1-dioxyde de *N*-[3-[3-[(1-pipéridinyl)méthyl]phénoxy]propyl]-5-méthyl-1,2-benzisothiazole-3-amine;
1,1-dioxyde de *N*-[3-[3-[(1-pipéridinyl)méthyl]phénoxy]propyl]-5-chloro-1,2-benzisothiazole-3-amine;
1,1-dioxyde de *N*-[3-[3-[(1-pipéridinyl)méthyl]phénoxy]propyl]-5-méthoxy-1,2-benzisothiazole-3-amine;
1,1-dioxyde de *N*-[3-[3-[(1-pipéridinyl)méthyl]phénoxy]propyl]-6-sulfamoyl-1,2-benzisothiazole-3-amine;
1,1-dioxyde de *N*-[3-[3-[(1-pipéridinyl)méthyl]phénoxy]propyl]-6-bromo-1,2-benzisothiazole-3-amine;
1,1-dioxyde de *N*-[3-[3-[(1-pipéridinyl)méthyl]phénoxy]propyl]-5-fluoro-1,2-benzisothiazole-3-amine;
1,1-dioxyde de *N*-[3-[3-(4-morpholinylméthyl]phénoxy]propyl]-1,2-benzisothiazole-3-amine;
1,1-dioxyde de *N*-[3-[3-[(diéthylamino)méthyl]phénoxy]propyl]-1,2-benzisothiazole-3-amine;
1,1-dioxyde de *N*-[3-[3-[(diméthylamino)méthyl]phénoxy]propyl]-1,2-benzisothiazole-3-amine;
1,1-dioxyde de *N*-[3-[3-[(dibutylaminométhyl]phénoxy]propyl]-1,2-benzisothiazole-3-amine;
1,1-dioxyde de *N*-[3-[3-[(4-thiomorpholinyl)méthyl]phénoxy]propyl]-1,2-benzisothiazole-3-amine;
1,1-dioxyde de *N*-[3-[3-[(3-thiazolidinylméthyl]phénoxy]propyl]-1,2-benzisothiazole-3-amine;
1,1-dioxyde de *N*-[3-[3-[(octahydro-1(2*H*)-azocinyl)méthyl]phénoxy]propyl]-1,2-benzisothiazole-3-amine;
1,1-dioxyde de *N*-[3-[3-[(cyclohexylamino)méthyl]phénoxy]propyl]-1,2-benzisothiazole-3-amine;
1,1-dioxyde de *N*-[3-[3-[(cyclopentylamino)méthyl]phénoxy]propyl]-1,2-benzisothiazole-3-amine;
1,1-dioxyde de *N*-[3-[3-[[[(2-furannyl)méthyl]amino]méthyl]phénoxy]propyl]-1,2-benzisothiazole-3-amine;
1,1-dioxyde de *N*-[3-[3-[(benzylamino)méthyl]phénoxy]propyl]-1,2-benzisothiazole-3-amine;
1,1-dioxyde de *N*-[3-[3-[[bis(2-méthylpropyl)amino]méthyl]phénoxy]propyl]-1,2-benzisothiazole-3-amine;
1,1-dioxyde de *N*-[3-[3-[(butylamino)méthyl]phénoxy]propyl]-1,2-benzisothiazole-3-amine;
1,1-dioxyde de *N*-[3-[3-[(méthylpentylamino)méthyl]phénoxy]propyl]-1,2-benzisothiazole-3-amine;
1,1-dioxyde de *N*-[3-[3-[(butylpropylamino)méthyl]phénoxy]propyl]-1,2-benzisothiazole-3-amine;
1,1-dioxyde de *N*-[3-[3-[(butylméthylamino)méthyl]phénoxy]propyl]-1,2-benzisothiazole-3-amine;
1,1-dioxyde de *N*-[3-[3-[(dipentylamino)méthyl]phénoxy]propyl]-1,2-benzisothiazole-3-amine;
1,1-dioxyde de *N*-[3-[4-[(1-pipéridinyl)méthyl]phénoxy]propyl]-1,2-benzisothiazole-3-amine;
ou un sel pharmacologiquement acceptable de ce composé.

7. Composé de formule I suivant la revendication 1, choisi entre le S,S'-dioxyde de,
4-[[[2-[(1,2-benzisothiazole-3-yl)amino]éthyl]thio]méthyl]-2-thiazolyl]guanidine;
la 3-[[3-[3-[(1-pipéridinyl)méthyl]phénoxy]propyl]-amino]]-1*H*-iso-indole-1-one;
ou un sel pharmacologiquement acceptable de ce composé.

8. Composé de formule I suivanbt la revendication 1, choisi entre le 1,1-dioxyde de *N*-[3-[3-[(1-pipéridinyl)méthyl]phénoxy]propyl]-1,2-benzisothiazole-3-amine;
le 1,1-dioxyde de *N*-[3-[3-[(dipropylamino)méthyl]phénoxy]propyl]-1,2-benzisothiazole-3-amine;
le 1,1-dioxyde de *N*-[3-[3-[(1-pyrrolidinylméthyl]phénoxy]propyl]-1,2-benzisothiazole-3-amine;
le 1,1-dioxyde de *N*-[3-[3-[(hexahydro-1*H*-azépine-1-ylméthylphénoxy]propyl]-1,2-benzisothiazole-3-amine;
ou un sel pharmacologiquement acceptable de ce composé.

9. Procédé de préparation d'un composé de formule I suivant la revendication 1 ou d'un sel pharmacologiquement acceptable de ce composé, qui consiste:

a) à faire réagir un composé de formule:

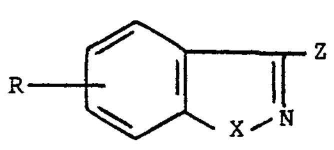

(II)

dans laquelle X et R sont tels que définis dans la revendication 1 et Z est un halogène ou un groupe $SR^4$ dans lequel $R^4$ est un radical alkyle ou aralkyle, avec une amine de formule H—A dans laquelle A est tel que défini dans la revendication 1, pour former un composé de formule I, ou bien

b) à faire réagir un composé de formule

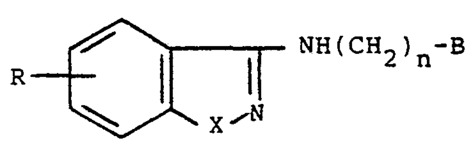

(III)

dans laquelle X, R et n sont tels que définis dans la revendication 1 et B est un groupe OH ou un groupe L, le groupe L représentant un groupe partant, avec un composé de formule:

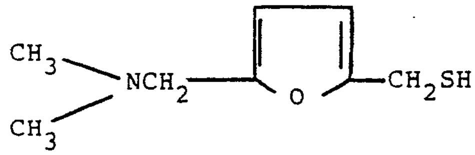

(IVa)

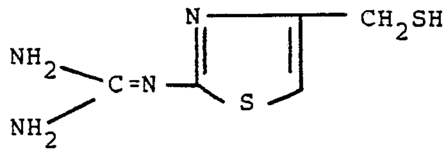

(IVb)

ou

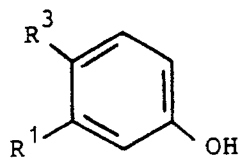

(IVc)

dans laquelle $R^1$ et $R^3$ sont tels que définis dans la revendication 1, sous réserve que (i) lorsqu'un composé de formule IVa ou IVb est utilisé, n soit égal à 2 dans le composé de formule III et (ii) lorsqu'un composé de formule IVc est utilisé, B représente L; ou bien

c) à faire réagir un composé de formule:

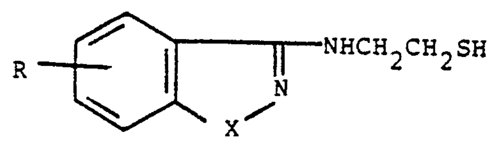

(V)

dans laquelle R et X sont tels que définis dans la revendication 1, avec un composé de formule:

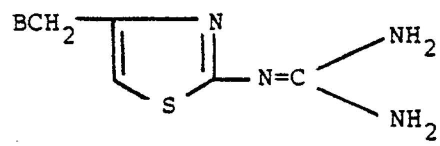

(VIa)

ou

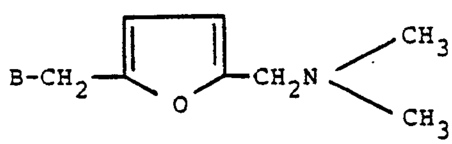

(VIb)

dans laquelle B représente OH ou L tel que défini ci-dessus pour former un composé de formule I dans laquelle A répond à la formule Ia ou Ib; ou bien

d) à faire réagir un aldéhyde de formule

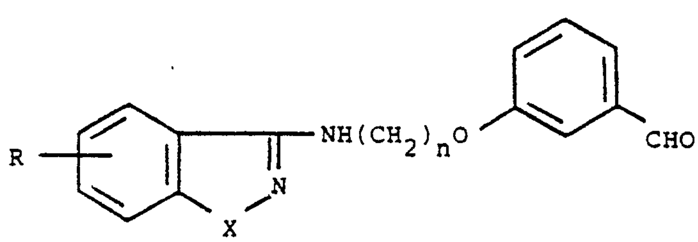

(VIIa)

avec un amine appropriée de formule

$HR^2$ (VIIb)

formules dans lesquelles n, R, X et $R^2$ sont tels que définis dans la revendication 1, dans des conditions

d'amination par voie de réduction pour former un composé correspondant de formule I dans laquelle $R^1$ est un groupe $R^2CH_2$— et $R^3$ est l'hydrogène; ou bien

e) à faire réagir un aldéhyde de formule

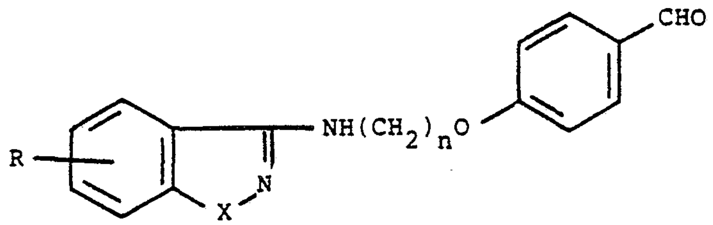

(VIIc)

dans laquelle n, R et X sont tels que définis dans la revendication 1, avec la pipéridine dans des conditions d'amination par voie de réduction pour former un composé de formule I dans laquelle $R^3$ est un groupe (1-pipéridinyl)méthyle; ou bien

f) à faire réagir un aldéhyde de formule

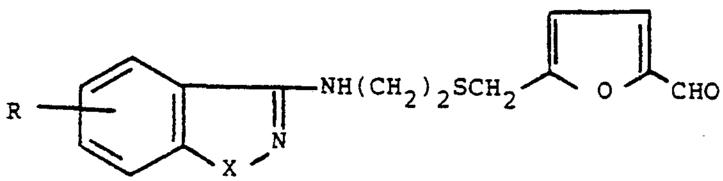

(VIId)

dans laquelle X, R et N sont tels que définis dans la revendication 1, avec la diméthylamine dans des conditions d'amination par voie de réduction pour former un composé de formule I dans laquelle A répond à la formule Ia; ou bien

g) à faire réagir un composé de formule

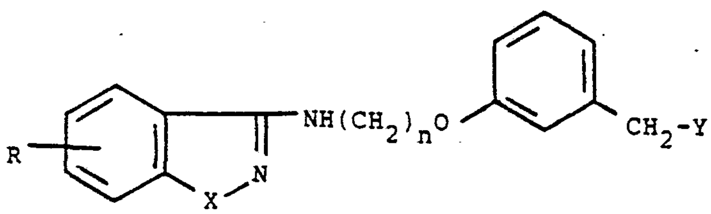

(VIII)

dans laquelle n, X et R sont tels que définis dans la revendication 1 et Y représente OH ou L, le groupe L étant un groupe partant tel que défini ci-dessus, avec une amine de formule $HR^2$ tells que définie ci-dessus, la réaction étant conduite en présence d'un catalyseur au nickel lorsque Y représente OH, pour former un composé de formule I dans laquelle A répond à la formule Ic et $R^1$ est un groupe $R^2CH_2$—; ou bien

h) à faire réagir un composé de formule

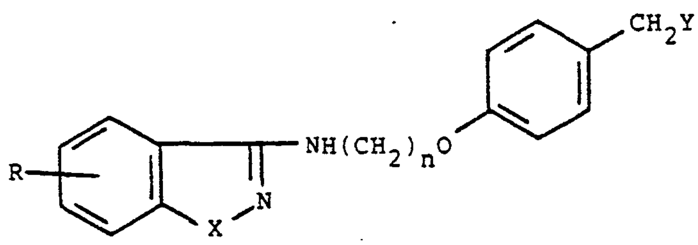

(VIIIa)

dans laquelle Y, n, X et R sont tels que définis ci-dessus, avec la pipéridine, la réaction étant conduite en présence d'un catalyseur au nickel lorsque Y représente OH, pour former un composé de formule I dans laquelle A répond à la formule Ic et $R^3$ est le groupe (1-pipéridinyl)méthyle; ou bien

i) à faire réagir un composé de formule

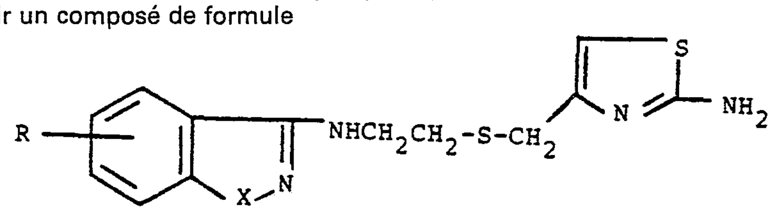

(IX)

dans laquelle X et R sont tels que définis dans la revendication 1, avec un composé capable de convertir —$NH_2$ en

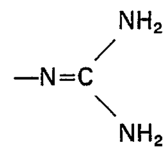

pour former un composé de formule I dans laquelle A répond à la formule Ic; ou bien

j) à faire réagir un composé de formule

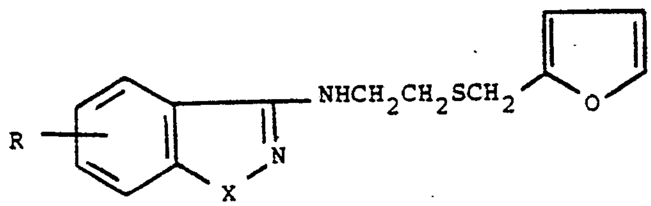

(X)

dans laquelle X et R sont tels que définis dans la revendication 1, avec la diméthylamine en présence de formaldéhyde ou de paraformaldéhyde pour former un composé de formule I dans laquelle A répond à la formule Ia; ou bien

k) à faire réagir un composé de formule

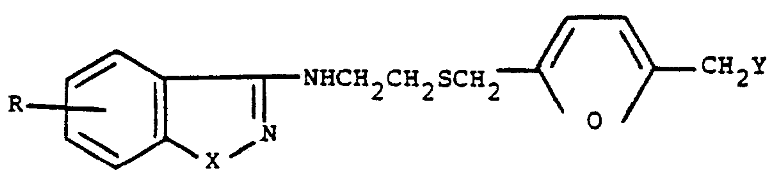

(XI)

dans laquelle Y représente OH ou un groupe partant L tel que défini ci-dessus, avec la diméthylamine, la réaction étant conduite en présence d'un catalyseur au nickel lorsque Y représente OH; ou bien

l) à convertir un composé basique de formule I en un sel pharmacologiquement acceptable ou à convertir un sel pharmacologiquement acceptable en la forme base libre.

10. Composé de formule I ou sel pharmacologiquement acceptable de ce composé suivant l'une quelconque des revendications 1 à 8, destiné à être utilisé commeinhibiteur de sécrétion d'acide gastrique.

11. Composition pharmaceutique comprenant un composé de formule I suivant l'une quelconque des revendications 1 à 8 ou un sel pharmaceutiquement acceptable de ce composé et un support acceptable du point de vue pharmaceutique.

**Revendications pour l'Etat contractant: AT**

1. Procédé de préparation d'un composé de formule:

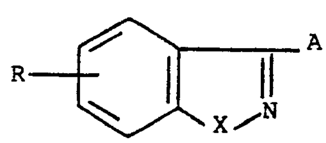

(I)

dans laquelle R est l'hydrogène, un radical mono- ou dihalogéno, amino, nitro, cyano, hydroxy, trifluorométhyle, mercapto, alkyle inférieur, alkoxy inférieur, alcanoyle ayant 2 à 5 atomes de carbone, cycloalkyle ayant 5 à 7 atomes de carbone, carboxy, alkoxycarbonyle ayant 2 à 7 atomes de carbone, amino portant 1 ou 2 substituants alkyle inférieur, alcanoylamino ayant 2 à 5 atomes de carbone, alkylthio, inférieur, alkylsulfonyle inférieur, sulfamoyle, sulfamoyle à substituant alkyle inférieur, phényle ou phényle à substituant halogéno, alkyle inférieur, alkoxy inférieur, trifluorométhyle, hydroxy, amino, cyano ou nitro; X représente $SO_2$, SO, S ou C=O; et A est une amine de formule;

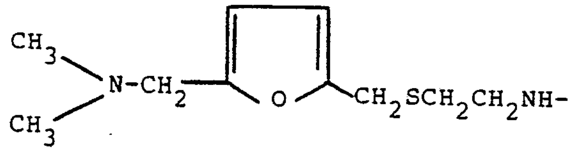

(Ia)

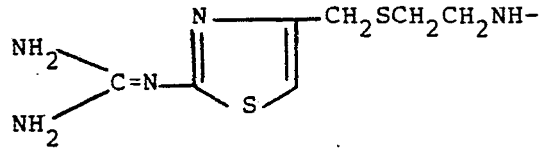

(Ib)

ou

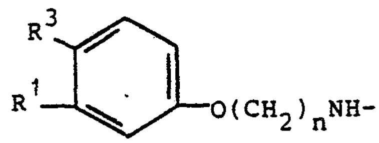

(Ic)

dans laquelle $R^1$ est l'hydrogène ou un groupe $R^2CH_2$ dans lequel $R^2$ est un radical mono- ou di(alkyle inférieur)amino, mono- ou di-N(alkyle inférieur)aminoalkyle inférieur, (2-furyl)méthylamino, benzylamine, cycloalkylamino ayant 5 à 7 atomes de carbone, 1-pyrrolidinyle, 1-pipéridinyle, 1-hexahydroazépinyle, 1-octahydroazocinyle, 3-thiazolidinyle, 4-morpholinyle ou 4-thiomorpholinyle; $R^3$ est l'hydrogène ou le groupe (1-pipéridinyl)méthyle; le terme "halogéno" désigne un radical fluoro, chloro ou bromo et le terme "inférieur" utilisé pour qualifier un groupe alkyle ou alkoxy signifie que ce groupe contient 1 à 6 atomes de carbone; sous réserve que, lorsque $R^3$ est le groupe (1-pipéridinyl)méthyle, $R^1$ soit un atome d'hydrogène; n a une valeur de 1 à 4, et de ses sels pharmacologiquement acceptables, procédé qui consiste:

a) à faire réagir un composé de formule:

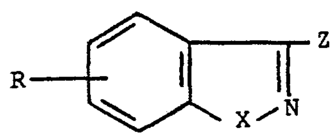

(II)

dans laquelle X et R sont tels que définis ci-dessus et Z est un halogène ou un groupe $SR^4$ dans lequel $R^4$ est un radical alkyle ou aralkyle, avec une amine de formule H—A dans laquelle A est tel que défini dans la revendication 1, pour former un composé de formule I, ou bien

b) à faire réagir un composé de formule

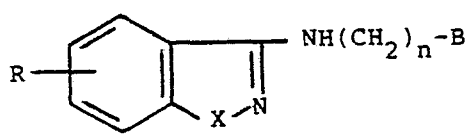

(III)

dans laquelle X, R et n sont tels que définis ci-dessus et B est un groupe OH ou un groupe L, le groupe L représentant un groupe partant, avec un composé de formule:

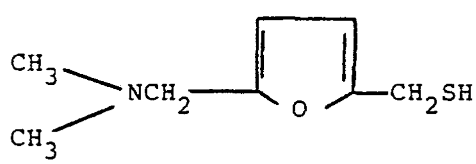

(IVa)

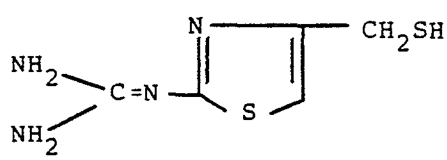

(IVb)

or

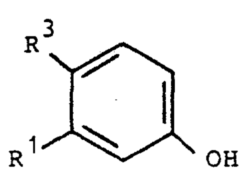

(IVc)

dans laquelle $R^1$ et $R^3$ sont tels que définis ci-dessus, sous réserve que (i) lorsqu'un composé de formule IVa ou IVb est utilisé, n soit égal à 2 dans le composé de formule III et (ii) lorsqu'un composé de formule IVc est utilisé, B représente L; ou bien

c) à faire réagir un composé de formule:

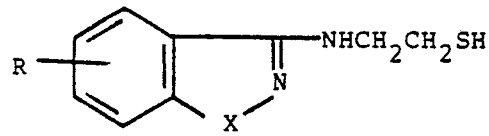

(V)

dans laquelle R et X sont tels que définis ci-dessus, avec un composé de formule:

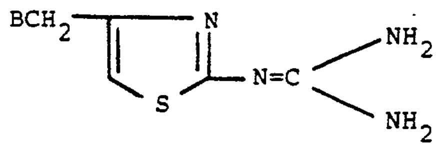

(VIa)

ou

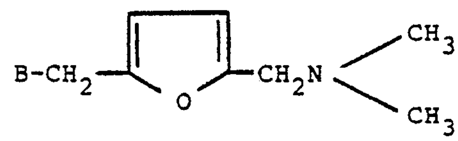

(VIb)

dans laquelle B représente OH ou L tel que défini ci dessus pour former un composé de formule I dans laquelle A répond à la formule Ia ou Ib; ou bien

d) à faire réagir un aldéhyde de formule

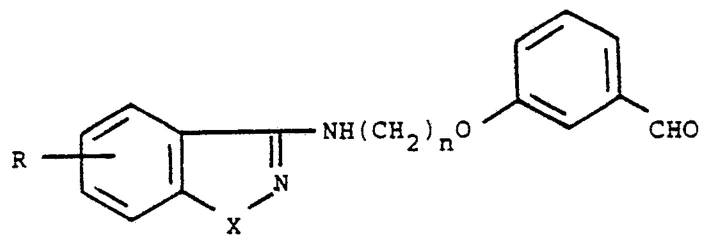

(VIIa)

avec un amine appropriée de formule

$HR^2$ (VIIb)

formules dans lesquelles n, R, X et $R^2$ sont tels que définis ci-dessus, dans des conditions d'amination par voie de réduction pour former un composé correspondant de formule I dans laquelle $R^1$ est un groupe $R^2CH_2$— et $R^3$ est l'hydrogène; ou bien

e) à faire réagir un aldéhyde de formule

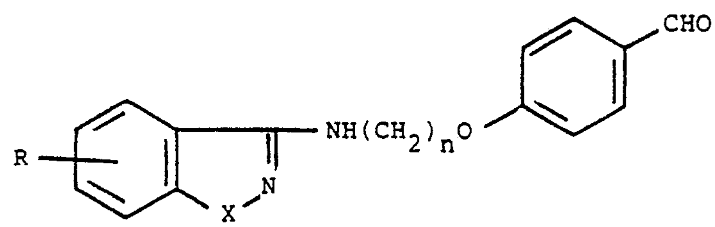

(VIIc)

dans laquelle n, R et X sont tels que définis dans la revendication 1, avec la pipéridine dans des conditions d'amination par voie de réduction pour former un composé de formule I dans laquelle $R^3$ est un groupe (1-pipéridinyl)méthyle; ou bien

f) à faire réagir un aldéhyde de formule

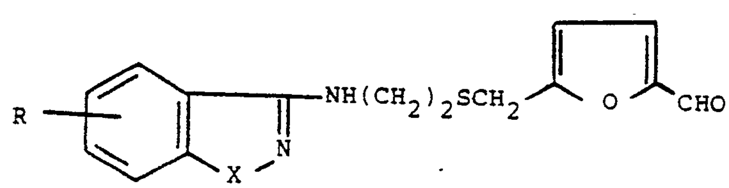

(VIId)

dans laquelle X et R sont tels que définis ci-dessus, avec la diméthylamine dans des conditions

d'amination par voie de réduction pour former un composé de formule I dans laquelle A répond à la formule Ia; ou bien

g) à faire réagir un composé de formule

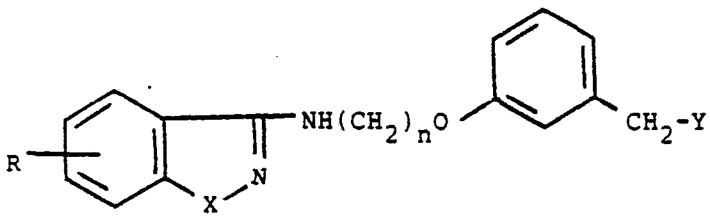

(VIII)

dans laquelle n, X et R sont tels que définis ci-dessus et Y représente OH ou L, le groupe L étant un groupe partant tel que défini ci-dessus, avec une amine de formule $HR^2$ tells que définie ci-dessus, la réaction étant conduite en présence d'un catalyseur au nickel lorsque Y représente OH, pour former un composé de formule I dans laquelle A répond à la formule Ic et $R^1$ est un groupe $R^2CH_2$—; ou bien

h) à faire réagir un composé de formule

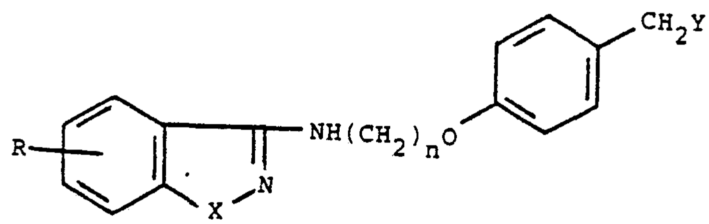

(VIIIa)

dans laquelle Y, n, X et R sont tels que définis ci-dessus, avec la pipéridine, la réaction étant conduite en présence d'un catalyseur au nickel lorsque Y représente OH, pour former un composé de formule I dans laquelle A répond à la formule Ic et $R^3$ est le groupe (1-pipéridinyl)méthyle; ou bien

i) à faire réagir un composé de formule

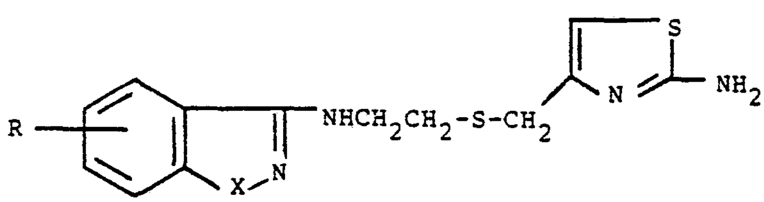

(IX)

dans laquelle X et R sont tels que définis ci-dessus, avec un composé capable de convertir —$NH_2$ en

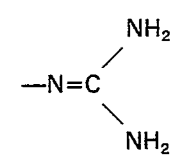

pour former un composé de formule I dans laquelle A répond à la formule Ic; ou bien

j) à faire réagir un composé de formule

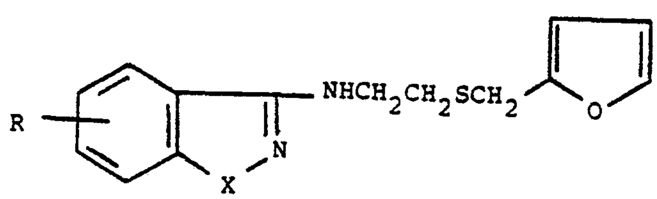

(X)

dans laquelle X et R sont tels que définis ci-dessus, avec la diméthylamine en présence de formaldéhyde ou de paraformaldéhyde pour former un composé de formule I dans laquelle A répond à la formule Ia; ou bien

k) à réagir un composé de formule

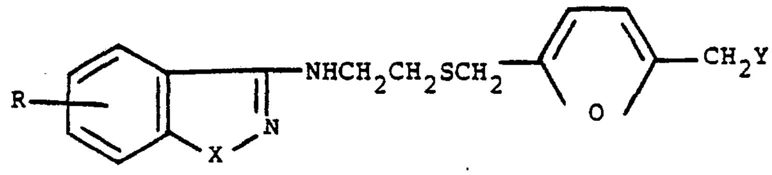

(XI)

dans laquelle Y représente OH ou un groupe partant L tel que défini ci-dessus, avec la diméthylamine, la réaction étant conduite en présence d'un catalyseur au nickel lorsque Y représent OH; ou bien

l) à convertir un composé basique de formule I en un sel pharmacologiquement acceptable ou à convertir un sel pharmacologiquement acceptable en la forme base libre.

2. Procédé suivant la revendication 1, dans lequel A répond à la formule Ic et $R^3$ est l'hydrogène dans le composé préparé.

3. Procédé suivant la revendication 1 ou la revendication 2, dans lequel A répond à la formule Ic et n est égal à 3 dans le composé préparé.

4. Procédé suivant l'une quelconque des revendications 1 à 3, dans lequel, dans le composé préparé, A répond à la formule Ic et $R^1$ est un groupe de formule $R^2CH_2$ dans laquelle $R^2$ est un radical di(alkyle inférieur)amino, 1-pyrrolidinyle, 2-pipéridinyle ou 1-hexahydro-azépinyle.

5. Procédé suivant l'une quelconque des revendications 1 à 4, dans lequel X représente $SO_2$ dans le composé préparé.

6. Procédé suivant la revendication 1, dans lequel le composé préparé est choisi entre les composés suivants:

1,1-dioxyde de *N*-[2-[[[5-[(diméthylamino)méthyl]-2-furannyl]méthyl]thio]éthyl]-1,2-benzisothiazole-3-amine;

1,1-dioxyde de *N*-[3-[3-[(1-pipéridinyle)méthyl)]phénoxyl]propyl]-6-nitro-1,2-benzisothiazole-3-amine;

1,1-dioxyde de *N*-[3-[3-[(1-pipéridinyl)méthyl]phénoxy]propyl]-6-amino-1,2-benzisothiazole-3-amine;

1,1-dioxyde de *N*-[3-[3-[(1-pipéridinyl)méthyl]phénoxy]propyl]-6-chloro-1,2-benzisothiazole-3-amine;

1,1-dioxyde de *N*-[3-[3-[(1-pipéridinyl)méthyl]phénoxy]propyl]-6-fluoro-1,2-benzisothiazole-3-amine;

1,1-dioxyde de *N*-[3-[3-[(1-pipéridinyl)méthyl]phénoxy]propyl]-5,6-dichloro-1,2-benzisothiazole-3-amine;

1,1-dioxyde de *N*-[3-[3-[(1-pipéridinyl)méthyl]phénoxy]propyl]-5-méthyl-1,2-benzisothiazole-3-amine;

1,1-dioxyde de *N*-[3-[3-[(1-pipéridinyl)méthyl]phénoxy]propyl]-5-chloro-1,2-benzisothiazole-3-amine;

1,1-dioxyde de *N*-[3-[3-[(1-pipéridinyl)méthyl]phénoxy]propyl]-5-méthoxy-1,2-benzisothiazole-3-amine;

1,1-dioxyde de *N*-[3-[3-[(1-pipéridinyl)méthyl]phénoxy]propyl]-6-sulfamoyl-1,2-benzisothiazole-3-amine;

1,1-dioxyde de *N*-[3-[3-[(1-pipéridinyl)méthyl]phénoxy]propyl]-6-bromo-1,2-benzisothiazole-3-amine;

1,1-dioxyde de *N*-[3-[3-[(1-pipéridinyl)méthyl]phénoxy]propyl]-5-fluoro-1,2-benzisothiazole-3-amine;

1,1-dioxyde de *N*-[3-[3-(4-morpholinylméthyl]phénoxy]propyl]-1,2-benzisothiazole-3-amine;

1,1-dioxyde de *N*-[3-[3-[(diéthylamino)méthyl]phénoxy]propyl]-1,2-benzisothiazole-3-amine;

1,1-dioxyde de *N*-[3-[3-[(diméthylamino)méthyl]phénoxy]propyl]-1,2-benzisothiazole-3-amine;

1,1-dioxyde de *N*-[3-[3-[(dibutylaminométhyl]phénoxy]propyl]-1,2-benzisothiazole-3-amine;

1,1-dioxyde de *N*-[3-[3-[(4-thiomorpholinyl)méthyl]phénoxy]propyl]-1,2-benzisothiazole-3-amine;

1,1-dioxyde de *N*-[3-[3-[(3-thiazolidinylméthyl]phénoxy]propyl]-1,2-benzisothiazole-3-amine;

1,1-dioxyde de *N*-[3-[3-[(octahydro-1(2*H*)-azocinyl)méthyl]phénoxy]propyl]-1,2-benzisothiazole-3-amine;

1,1-dioxyde de *N*-[3-[3-[(cyclohexylamino)méthyl]phénoxy]propyl]-1,2-benzisothiazole-3-amine;

1,1-dioxyde de *N*-[3-[3-[(cyclopentylamino)méthyl]phénoxy]propyl]-1,2-benzisothiazole-3-amine;

1,1-dioxyde de *N*-[3-[3-[[[(2-furannyl)méthyl]amino]méthyl]phénoxy]propyl]-1,2-benzisothiazole-3-amine;

1,1-dioxyde de *N*-[3-[3-[(benzylamino)méthyl]phénoxy]propyl]-1,2-benzisothiazole-3-amine;

1,1-dioxyde de *N*-[3-[3-[[bis(2-méthylpropyl)amino]méthyl]phénoxy]propyl]-1,2-benzisothiazole-3-amine;

1,1-dioxyde de *N*-[3-[3-[(butylamino)méthyl]phénoxy]propyl]-1,2-benzisothiazole-3-amine;

1,1-dioxyde de *N*-[3-[3-[(méthylpentylamino)méthyl]phénoxy]propyl]-1,2-benzisothiazole-3-amine;

1,1-dioxyde de *N*-[3-[3-[(butylpropylamino)méthyl]phénoxy]propyl]-1,2-benzisothiazole-3-amine;

1,1-dioxyde de *N*-[3-[3-[(butylméthylamino)méthyl]phénoxy]propyl]-1,2-benzisothiazole-3-amine;

1,1-dioxyde de *N*-[3-[3-[(dipentylamino)méthyl]phénoxy]propyl]-1,2-benzisothiazole-3-amine;

1,1-dioxyde de *N*-[3-[4-[(1-pipéridinyl)méthyl]phénoxy]propyl]-1,2-benzisothiazole-3-amine;

ou un sel pharmacologiquement acceptable de ce composé.

7. Procédé suivant la revendication 1, dans lequel le composé préparé est le S,S'-dioxyde de 4-[[[2-[(1,2-benzisothiazole-3-yl)amino]éthyl]thio]méthyl]-2-thiazolyl]guanidine;

la 3-[[3-[3-[(1-pipéridinyl)méthyl]phénoxy]propyl]-amino]]-1*H*-iso-indole-1-one;
ou un sel pharmacologiquement acceptable de ce composé.

8. Procédé suivant la revendiction 1, dans lequel le composé prépare est choisi entre le 1,1-dioxyde de *N*-[3-[3-[(1-pipéridinyl)méthyl]phénoxy]propyl]-1,2-benzisothiazole-3-amine;
le 1,1-dioxyde de *N*-[3-[3-[(dipropylamino)méthyl]phénoxy]propyl]-1,2-benzisothiazole-3-amine;
le 1,1-dioxyde de *N*-[3-[3-[(1-pyrrolidinylméthyl]phénoxy]propyl]-1,2-benzisothiazole-3-amine;
le 1,1-dioxyde de *N*-[3-[3-[(hexahydro-1*H*-azépine-1-yl)méthyl]phénoxy]propyl]-1,2-benzisothiazole-3-amine;
ou un sel pharmacologiquement acceptable de ce composé.

9. Procédé de préparation d'un composition pharmaceutique, caractérisé en ce qu'un composé de formule I tel que défini dans l'une quelconque des revendications 1 à 8 ou un sel pharmaceutiquement acceptable de ce composé est mis sous une forme qui convient à l'administration thérapeutique.

10. Procédé suivant la revendication 9, dans lequel le composé de formule I est préparé par un procédé suivant l'une quelconque des revendications 1 à 8.